# EUROPEAN PATENT APPLICATION

(11) **EP 4 484 446 A1**
(43) Date of publication of application: **01.01.2025**
(21) Application number: 23759273.8
(22) Date of filing: 24.02.2023
(51) Int. Cl.: C07K 16/36, A61K 47/68, A61P 35/00

(54) **ANTIBODY, AND DRUG CONJUGATE AND USE THEREOF**

(30) Priority: 24.02.2022 CN 202210172397; 01.08.2022 CN 202210916833; 27.09.2022 CN 202211183393
(71) Applicant: Evopoint Biosciences Co., Ltd., Suzhou, Jiangsu 215000 (CN)
(72) Inventor: HU, Yonghan, Suzhou, Jiangsu 215000 (CN); XU, Linfeng, Suzhou, Jiangsu 215000 (CN); WU, Zhenwei, Suzhou, Jiangsu 215000 (CN); RUAN, Ka, Suzhou, Jiangsu 215000 (CN); WANG, Wengui, Suzhou, Jiangsu 215000 (CN)
(74) Representative: Nederlandsch Octrooibureau
(86) International application number: PCT/CN2023/078127
(87) International publication number: WO 2023/160651

(57) **Abstract**

The present invention relates to an antibody and an antibody-drug conjugate, and particularly to an antibody and an antibody-drug conjugate (ADC) targeting the tissue factor, and a composition comprising the antibody or ADC, and a therapeutic application thereof.

## Description

### TECHNICAL FIELD

The present invention relates to an antibody and an antibody-drug conjugate, and particularly relates to an antibody and an antibody-drug conjugate (ADC) targeting the tissue factor (TF), a composition comprising the antibody or ADC, and a therapeutic application thereof.

### BACKGROUND

Tissue factor (TF) is also known as thromboplastin, CD142, or coagulation factor III. As a transmembrane glycoprotein, TF consists of an extracellular domain, a transmembrane region, and an intracellular domain.

TF is an essential molecule for the initiation of an extrinsic coagulation event and is expressed on the cell surface in a functional form. As a high affinity receptor for coagulation factor VII (FVII, a serine protease) in plasma, TF, after forming a complex with VIIa (the activated form of VII), triggers a catalytic event that activates factor IX or X, via specific limited proteolytic cleavage, thereby initiating the coagulation protease cascade. Under normal physiological conditions, TF is located on the adventitial cells of the vessel wall, the fibroblasts surrounding the vessel, etc., but it is very rare in tunica media or tunica intima layers of the vessel. TF is exposed to the circulating blood only when the integrity of the vessel wall is compromised, and exerts hemostatic effects by activating the coagulation cascade. In this process, TF acts as an "anchor" by virtue of its tight binding to the cell membrane, so that physiological coagulation is limited to the site of injury and does not spread distantly from the site of initiation of blood coagulation.

In contrast to limited expression on normal tissues and cells, TF has been shown to be overexpressed on a variety of malignant tumors, including cervical cancer, pancreatic cancer, lung cancer, prostate cancer, bladder cancer, ovarian cancer, breast cancer, colorectal cancer, and the like. Therefore, TF can be used as a target for development of antibody drugs and ADC (antibody-drug conjugate) drugs. The TF-targeted ADC drug Tivdak (Tisotumab Vedotin) developed by Seagen Inc. (SGEN)/Genmab A/S (GMAB) has been approved for cervical cancer indications in September, 2021, and has also been in clinical stage II for use in other indications such as ovarian cancer.

However, many anti-TF antibodies or antibody drugs currently being developed, although exhibiting certain efficacy in cancer therapy, have also been detected for the interference or inhibition of coagulation and related side effects caused thereby (Chenard-Poirier M, Hong DS, Coleman R, de Bono J, Mau-Sorensen M, Collins D, et al., A phase I/II safety study of tisotumab vedotin (HuMax-TFADC) in patients with solid tumors, Ann Oncol 2017; 28:v403-v27; Zhang X, Li Q, Zhao H, Ma L, Meng T, Qian J, et al., Pathological expression of tissue factor confers promising antitumor response to a novel therapeutic antibody SC1 in triple negative breast cancer and pancreatic adenocarcinoma, Oncotarget 2017; 8:59086-102).

Therefore, there remains a need in the art to develop a novel TF antibody and a novel ADC drug comprising the same. The TF antibody should have the ability to specifically target TF on the surface of tumor cells, but at the same time have minimal effect on TF-mediated coagulation in normal tissues, so as to provide a broader and superior dosing option for cancer patients.

### SUMMARY

In order to meet the above need in the art, the inventor provides a novel TF antibody and an antibody-drug conjugate (ADC) through extensive studies. As shown in the examples, the anti-TF antibody of the present invention not only exhibits high binding affinity and high specificity for TF-positive tumor cells, thereby being able to be rapidly and efficiently endocytosed by the tumor cells, but also has little effect on TF-mediated coagulation. Further, as shown in the examples, the ADC composed of the TF antibody of the present invention not only has good physical properties and no significant aggregation, but also exhibits significant tumor growth inhibitory activity and good tolerance to drug administration in animal models.

Accordingly, in a first aspect, the present invention provides an antibody-drug conjugate (ADC) having the following formula (I), or a pharmaceutical acceptable salt or solvate thereof:

Ab-[L-D]_{q} (I)

wherein,
Ab represents an anti-TF antibody,
L represents a linker,
D represents a cytotoxic or cytostatic drug, e.g., a topoisomerase I inhibitor, and
q = 1-20, e.g., q = 1-10, 1-8, 3-8, 4-8, or 6-8,
wherein the Ab comprises:
   - three CDRs of a heavy chain variable region (VH) sequence set forth in SEQ ID NO: 1 and three CDRs of a light chain variable region (VL) sequence set forth in SEQ ID NO: 2,
   - three CDRs of a heavy chain variable region (VH) sequence set forth in SEQ ID NO: 3 and three CDRs of a light chain variable region (VL) sequence set forth in SEQ ID NO: 4,
   - three CDRs of a heavy chain variable region (VH) sequence set forth in SEQ ID NO: 5 and three CDRs of a light chain variable region (VL) sequence set forth in SEQ ID NO: 6, or
   - three CDRs of a heavy chain variable region (VH) sequence set forth in SEQ ID NO: 7 and three CDRs of a light chain variable region (VL) sequence set forth in SEQ ID NO: 8,
      wherein preferably, the CDRs are defined according to Chothia, AbM, Kabat, IMGT, or any combination thereof,
      more preferably, the Ab comprises three CDRs of a heavy chain variable region (VH) sequence of SEQ ID NO: 7 and three CDRs of a light chain variable region (VL) sequence of SEQ ID NO: 8.

In a second aspect, the present invention provides a composition comprising the ADC or the pharmaceutical acceptable salt or solvate thereof of the present invention.

In a third aspect, the present invention provides use of the ADC or the pharmaceutical acceptable salt or solvate thereof of the present invention and the composition thereof in the treatment or prevention of TF-positive tumors and in the manufacture of a medicament for use in the treatment or prevention.

In a fourth aspect, the present invention provides an anti-TF antibody, and a pharmaceutical composition and use thereof.

The present invention is further illustrated in the following drawings and specific embodiments. However, these drawings and specific embodiments should not be construed as limiting the scope of the present invention, and modifications easily conceived by those skilled in the art will be included in the spirit of the present invention and the protection scope of the appended claims.

### BRIEF DESCRIPTION OF THE DRAWINGS

FIGs. 1A-1D show the binding affinity of exemplary antibodies of the present invention for human TF or monkey TF as assayed by antigenic protein-based ELSIA. BM is the reference antibody.
FIGs. 2A-2D show the cell binding affinity of exemplary antibodies of the present invention for different TF-positive tumor cells (NCI-H358 and KYSE520) as assayed by flow cytometry. BM is the reference antibody.
FIGs. 3A-3C show the blocking activity of the humanized antibodies of the present invention against the TF/FVIIa complex-mediated downstream signaling pathway as assayed by an inhibition experiment on IL-8 release.
FIGs. 4A-4B show the effect of the humanized antibodies of the present invention on coagulation as compared to the reference antibody BM. FIG. 4A: 2 mM Ca²⁺, 50 µg/mL antibody concentration; and FIG. 4B: 5 mM Ca²⁺, 20 µg/mL antibody concentration.
FIGs. 5A-5B show the results of serum stability assays on the humanized antibodies of the present invention after incubation in human serum at 37 °C for a period of time.
FIGs. 6A-6B show the change in antibody affinity before and after conjugation of the anti-TF humanized antibody to toxin-Linker (B81, i.e., DL-01 molecule) as determined by ELISA.
FIGs. 7A-7B show the results of *in vitro* killing activity assays on the ADCs of the present invention.
FIG. 8 shows the tumor inhibition effect of the ADCs of the present invention, 22F11H5-hz1, and 30G11B7 in mice after conjugation to the drug-linker (B81).
FIG. 9 shows the effect of ADCs comprising the antibodies 22F11H5-hz1 and 30G11B7 of the present invention, respectively, on the body weight of mice after administration.
FIG. 10 shows the tumor inhibition effect of the ADCs of the present invention, 2B 12B 10-hz1, and 27H8H3-hz1 in mice after conjugation to B81.
FIG. 11 shows the effect of ADCs comprising the antibodies 2B12B10-hz1 and 27H8H3-hz1 of the present invention, respectively, on the body weight of mice after administration.

### DETAILED DESCRIPTION

### Definitions

Unless otherwise defined, all technical and scientific terms used herein have the same meaning as those commonly understood by those of ordinary skill in the art. For the purposes of the present invention, the following terms are defined below.

When a tradename is used herein, the tradename includes the product formula, generic drug, and active pharmaceutical ingredient of the product with the tradename, unless otherwise clearly defined in the context.

The term "about" used in combination with a numerical value is intended to encompass the numerical values in a range from a lower limit less than the specified numerical value by 5% to an upper limit greater than the specified numerical value by 5%.

The term "and/or" used to connect two or more options should be understood to mean any one of the options or a combination of any two or more of the options.

As used herein, the term "comprise" or "include" is intended to mean that the elements, integers or steps are included, but not to the exclusion of any other elements, integers or steps. The term "comprise" or "include" used herein, unless indicated otherwise, also encompasses the situation where the entirety consists of the described elements, integers or steps. For example, when referring to an antibody variable region "comprising" a specific sequence, it is also intended to encompass an antibody variable region consisting of the specific sequence.

Herein, the terms "tissue factor" and "TF" are used interchangeably and, unless otherwise stated, include any variants of the human tissue factor, including sequence variants, particularly naturally occurring variants, allelic variants, as well as post-translationally modified variants and conformational variants, and encompass species homologs thereof. In addition, it should be understood that the term encompasses not only TF naturally or recombinantly expressed by a cell or TF expressed on a natural or recombinant cell, but also recombinantly expressed fusion proteins comprising the extracellular domain of TF. An example of the tissue factor is a human TF protein comprising the amino acid sequence under UniProtKB - P 13726, or a recombinant protein comprising the extracellular domain of the protein. Another example of the tissue factor is a monkey TF protein comprising the amino acid sequence under UniProtKB - A0A2K5VXA0, or a recombinant protein comprising the extracellular domain of the protein. Herein, unless otherwise specified, the term "tissue factor" or "TF" refers to the tissue factor derived from human.

Herein, the term "TF-positive" cell refers to a cell that is positive for TF cell surface expression, such as a cancer cell, an engineered cancer cell, or an engineered non-tumor cell. The expression level of TF on the cell surface can be determined by any conventional method known in the art for determining the expression level of cell surface antigens, for example, an FACS detection method or an immunofluorescence staining method. TF has significantly higher expression levels on a variety of tumor cells, e.g., MDA-MB-231 (breast cancer; > about 350,000 TF molecules/cell) and BxPC-3 (pancreatic cancer; > about 350,000 TF molecules/cell), than on normal tissues/cells. Preferably, herein, the TF-positive cell is a TF-positive tumor cell.

Herein, the term "antibody" refers to a polypeptide comprising at least an immunoglobulin light chain variable region or heavy chain variable region that specifically recognizes and binds to an antigen. The term encompasses various antibody structures, including but not limited to, monoclonal antibodies, polyclonal antibodies, single-chain or multi-chain antibodies, monospecific or multispecific antibodies (e.g., bispecific antibodies), chimeric or humanized antibodies, full-length antibodies, and antibody fragments, as long as they exhibit the desired antigen-binding activity.

Herein, "whole antibody" (used interchangeably herein with "full length antibody", "complete antibody", and "intact antibody") refers to an immunoglobulin molecule comprising at least two heavy (H) chains and two light (L) chains. Each heavy chain consists of a heavy chain variable region (abbreviated herein as VH) and heavy chain constant regions. Each light chain consists of a light chain variable region (abbreviated herein as VL) and light chain constant regions. The variable regions are domains in the heavy chains or light chains of antibodies that participate in binding of the antibodies to the antigens thereof. The constant regions are not directly involved in binding of antibodies to antigens, but exhibit a variety of effector functions. The light chains of antibodies can be divided into two classes (known as kappa (κ) and lambda (λ)) based on the amino acid sequences of their constant regions. The heavy chains of antibodies can be divided into 5 major distinct classes based on the amino acid sequence of their constant regions: IgA, IgD, IgE, IgG, and IgM, and several of these classes can be further divided into subclasses, e.g., IgG1, IgG2, IgG3, and IgG4, IgA1 and IgA2.

Herein, the terms "antibody fragment" and "antigen-binding fragment" of an antibody are used interchangeably to refer to a molecule that is not an intact antibody, which comprises the portion of the intact antibody for binding to the antigen to which the intact antibody binds. As understood by those skilled in the art, for antigen binding purposes, antibody fragments generally comprise amino acid residues from "complementarity determining regions" or "CDRs". Antibody fragments may be prepared by the recombinant DNA technology, or by enzymatic or chemical cleavage of intact antibodies. Examples of antibody fragments include, but are not limited to, Fab, scFab, disulfide-linked scFab, Fab', F(ab')₂, Fab'-SH, Fv, scFv, disulfide-linked scFv, linear antibody, diabody, triabody, tetrabody, and minibody. In some embodiments according to the present invention, the antibody fragment comprises a cysteine residue portion for forming interchain disulfide bonds between the light chain and the heavy chain or between the heavy chain and the heavy chain, e.g., cysteine residues of the Fab region and/or the hinge region of the IgG1 antibody, to provide amino acid residue sites useful for sulfhydryl conjugation chemistry. In some other embodiments according to the present invention, the antibody fragment comprises cysteine residues introduced into the Fc region, to provide amino acid residue sites useful for sulfhydryl conjugation chemistry.

Herein, the term "immunoglobulin" refers to a protein having a structure of a naturally occurring antibody. For example, immunoglobulins of the IgG class are heterotetrameric proteins of about 150,000 daltons, consisting of two light chains and two heavy chains that are disulfide-bonded. From N-terminus to C-terminus, each immunoglobulin heavy chain has one heavy chain variable region (VH), also known as a heavy chain variable domain, followed by three heavy chain constant domains (CH1, CH2, and CH3). From N-terminus to C-terminus, each immunoglobulin light chain has one light chain variable region (VL), also known as a light chain variable domain, followed by one light chain constant domain (CL). Accordingly, reference herein to an antibody being an IgG antibody means that the antibody is a heterotetrameric protein with an IgG immunoglobulin structure. In the IgG antibody, the VH-CH1 of the heavy chain is generally paired with the VL-CL of the light chain to form a Fab fragment that specifically binds to the antigen. Thus, an IgG antibody essentially consists of two Fab molecules and two dimerized Fc regions connected by an immunoglobulin hinge region. IgG immunoglobulins can be divided into subclasses, e.g., γ1 (IgG1), γ2 (IgG2), γ3 (IgG3), and γ4 (IgG4), based on the sequence of the heavy chain constant region. The light chains of IgG immunoglobulins can also be divided into two classes, known as κ and λ, based on the amino acid sequences of their constant domains. In some embodiments, the antibody according to the present invention is an IgG antibody, e.g., an IgG1, IgG2, IgG3, or IgG4 antibody. In some other embodiments, the antibody according to the present invention is an IgGκ or IgGλ antibody, e.g., an IgG1κ or IgG1λ antibody.

Herein, the term "complementarity determining region" or "CDR region" or "CDR" or "hypervariable region" are used interchangeably to refer to a region in an antibody variable domain which is highly variable in sequence and forms a structurally defined loop ("hypervariable loops") and/or contains antigen-contacting residues ("antigen-contacting sites"). The CDRs are primarily responsible for binding to antigenic epitopes. Herein, the CDRs of heavy and light chains of an antibody are numbered sequentially from the N-terminus, and are generally referred to as CDR1, CDR2, and CDR3. The CDRs located in the heavy chain variable domain of the antibody are referred to as HCDR1, HCDR2 and HCDR3, whereas the CDRs located in the light chain variable domain of the antibody are referred to as LCDR1, LCDR2 and LCDR3. In a given amino acid sequence of a light chain variable region or a heavy chain variable region, the CDR sequences may be determined using a variety of schemes well known in the art. For example, annotations of CDRs in a given light chain variable region or heavy chain variable region may be obtained at http://www.abysis.org/abysis/, including CDR sequences defined based on Kabat, AbM, Chothia, Contact, and IMGT. In addition, the CDRs may also be determined based on having the same Kabat numbering positions as the reference CDR sequences. Unless otherwise stated, residue positions of an antibody variable region (including heavy chain variable region residues and light chain variable region residues) are numbered according to the Kabat numbering system (Kabat et al., Sequences of Proteins of Immunological Interest, 5th Ed. Public Health Service, National Institutes of Health, Bethesda, Md. (1991)).

Herein, "variable region" or "variable domain" is a domain in the heavy chain or light chain of an antibody that participates in binding of the antibody to the antigen thereof. The heavy chain variable region (VH) and light chain variable region (VL) can be further subdivided into hypervariable regions (HVRs, also known as complementarity determining regions (CDRs)) with more conserved regions (i.e., framework regions (FRs)) inserted therebetween. Each VH or VL consists of three CDRs and four FRs, arranged from the N-terminus to C-terminus in the following order: FR1, CDR1, FR2, CDR2, FR3, CDR3, FR4. In some aspects, one or more residues in one or both of the two variable regions (i.e., VH and/or VL) of an antibody may be modified, for example, one or more CDR regions and/or one or more framework regions are subjected to residue modifications, particularly conservative residue substitutions, to obtain antibody variants that still substantially retain at least one biological property (e.g., antigen-binding ability) of the parent antibody prior to the modifications. In some other aspects, the variable regions of the antibody may be modified by CDR grafting. Since CDR sequences are responsible for most of the antibody-antigen interactions, a recombinant antibody variant that simulates the properties of known antibodies can be constructed. In such antibody variants, CDR sequences from known antibodies are grafted onto the framework regions of different antibodies with different properties. Properties of the mutated and/or modified antibodies or ADCs comprising the same, such as target antigen binding properties or other desired functional properties, such as endocytic activity, coagulation effects, pharmacokinetics, and *in vivo* tumor killing activity, can be assessed in an *in vitro* or *in vivo* assay.

Herein, the term "chimeric antibody" refers to an antibody in which the variable region sequences are derived from one species and the constant region sequences are derived from another species, e.g., an antibody in which the variable region sequences are derived from a mouse antibody and the constant region sequences are derived from a human antibody.

The term "humanized antibody" refers to an antibody in which CDR sequences derived from a non-human mammalian species, e.g., a mouse, are grafted onto human framework sequences. Additional framework region modifications may be made within the human framework sequences and/or additional amino acid modifications may be made in the CDR sequences, e.g., to perform affinity maturation of the antibody. Herein, in some embodiments, the humanized antibodies of the present invention have framework region sequences "derived from" a specific human germline sequence. As used herein, "derived from" means that the amino acid sequence of the framework region of the antibody has at least 90%, more preferably at least 95%, even more preferably at least 96%, 97%, 98% or 99% identity to the amino acid sequence of the corresponding framework region encoded by the human germline immunoglobulin gene, and the antibody retains the antigen-binding activity.

Herein, the term "isolated" antibody refers to an antibody that has been separated from a component of its natural environment. In some embodiments, the antibody is purified to a purity greater than 90%, 95%, or 99%, which can be determined, for example, by electrophoresis (e.g., SDS-PAGE, isoelectric focusing (IEF), or capillary electrophoresis), or chromatography (e.g., ion exchange or reverse-phase HPLC).

Herein, the term "epitope" refers to the region of an antigen to which an antibody binds. An epitope can be formed from contiguous amino acids or from non-contiguous amino acids juxtaposed by tertiary folding of a protein. In the present invention, preferably, the antibody according to the present invention binds to a native epitope of human TF, more preferably, to a native epitope of the extracellular domain of human TF expressed on the cell surface.

Herein, the term "affinity" or "binding affinity" refers to the inherent binding affinity that reflects the interaction between members of a binding pair. The affinity can be measured by common methods known in the art. One specific method for determining the affinity is the antigenic protein-based ELISA assay or cell-based ELISA assay as described in the examples herein, another specific method is the flow cytometry assay as described in the examples herein. The bio-layer interferometry (BLI) technique may also be used for dynamic affinity evaluation of antibodies.

The term "binding" or "specific binding", in the context of the binding of an antibody to a related antigen (herein, a TF antigen), is used to refer to the binding having an affinity with a K_{D} value of about 10⁻⁶ M or less, e.g., a K_{D} value of about 10⁻⁷ M or less, or about 10⁻⁸ M or less. The binding K_{D} value of an antibody to its related antigen is preferably at least 100-fold or, e.g., at least 1000-fold lower than the K_{D} value of a non-specific antigen (e.g., an unrelated antigen such as BSA). The measurement of K_{D} values is known in the art, e.g., the bio-layer interferometry (BLI) technique. The measurement may be performed, for example, in a ForteBio Octet^{®} instrument, using the antibody as a ligand and the antigen (e.g., a fusion protein comprising the extracellular domain of TF, such as TF-His) as an analyte.

The term "K_{D}" (M) herein refers to the dissociation equilibrium constant for specific antibody-antigen interaction. The affinity is inversely correlated with the K_{D} value, that is, the higher the affinity, the smaller the K_{D} value; conversely, the lower the affinity, the higher the K_{D} value. In general, the K_{D} value depends on the dissociation rate constant (Kd or Kdis, sec⁻¹) and association rate constant (Ka, M⁻¹·sec⁻¹) between interacting antibody-antigen pairs.

As understood by those skilled in the art, an antibody that specifically binds to human TF may have cross-reactivity with TF proteins from other species. In this context, the term "cross-reactivity" refers to the ability of an antibody to bind to TFs from different species. For example, in some embodiments, the antibody according to the present invention specific for human TF may also bind to TFs from other species (e.g., cynomolgus monkey TF). Methods for determining cross-reactivity include the methods described in the examples and standard assays known in the art, e.g., flow cytometry or the cell ELISA technique. Species cross-reactivity of antibodies is advantageous in some cases. For example, when a target antibody has species cross-reactivity with preclinical laboratory animals, e.g., primate, it will facilitate preclinical safety and efficacy evaluations of the target antibody prior to therapeutic or diagnostic use in humans.

Herein, the term "isotype" refers to the class of antibodies determined according to the heavy chain constant regions of the antibodies. For example, the antibodies according to the present invention may be IgA (e.g., IgA1 or IgA2), IgG1, IgG2 (e.g., IgG2a or IgG2b), IgG3, IgG4, IgE, IgM, and IgD antibodies, and have heavy chain constant regions of the immunoglobulin class. The antibodies of the present invention may also be IgG1 antibodies having constant regions of human IgG1. Furthermore, the present invention contemplates not only antibodies having native sequence constant regions, but also antibodies comprising variant sequence constant regions.

Herein, the term "native sequence Fc region" encompasses naturally occurring Fc region sequences of various immunoglobulins, such as Fc region sequences of various Ig subclasses and allotypes thereof (Gestur Vidarsson et al., IgG subclasses and allotypes: from structure to effector functions, 20 October 2014, doi: 10.3389/fimmu.2014.00520.). In some embodiments, the heavy chain Fc region of human IgG has an amino acid sequence extending from Cys226 or from Pro230 to the carboxyl terminus of the heavy chain. However, the C-terminal lysine (Lys447) of the Fc region may or may not be present. In some other embodiments, the heavy chain Fc region of human IgG carries a hinge sequence or a part of the hinge sequence of a native immunoglobulin at the N-terminus, e.g., according to EU numbering, the sequence from E216 to T225 or the sequence from D221 to T225.

Herein, the term "variant sequence Fc region" refers to an Fc region polypeptide comprising modifications relative to the native sequence Fc region polypeptide. The modification may be the additions, deletions or substitutions of amino acid residues. The substitutions may include substitutions of naturally occurring amino acids and non-naturally occurring amino acids. The purpose of the modifications may be to alter the binding of the Fc region to its receptor and the resulting effector functions.

The term "effector function" refers to those biological activities attributable to the Fc-region of an antibody that varies with the class of the antibody. IgG Fc regions are known to mediate several important effector functions, e.g., cytokine induction, ADCC, phagocytosis, complement-dependent cytotoxicity (CDC), and half-life/clearance rates of antibodies and antigen-antibody complexes. In some cases, depending on the therapeutic purpose, these effector functions may be desired for therapeutic antibodies, but may not be necessary in other cases. Thus, in one embodiment, the present invention provides an antibody having an Fc region that elicits an effector function, e.g., ADCC or CDC, to induce apoptosis or cytolysis in tumor cells bearing the TF antigen, and/or to inhibit proliferation, propagation and/or metastasis of tumor cells bearing the antigen TF. In some other embodiments, the present invention provides an antibody having an Fc region with an altered effector function. The effector function can be altered by making sequence changes to the Fc region of the antibody. Alternatively, an antibody with an altered class of glycosylation in the Fc region, e.g., a low-fucosylated or afucosylated antibody with reduced content of fucosyl residues or an antibody with increased bisecting GlcNac structures, can be made. Such altered glycosylation patterns have been shown to increase the ADCC ability of the antibody. Antibody variants having at least one galactose residue in an oligosaccharide linked to the Fc region may also be contemplated, and such antibody variants may have enhanced CDC function. Alterations of the glycosylation patterns of the Fc region can be conveniently achieved by altering the amino acid sequence of the Fc region to create or remove one or more glycosylation sites.

Herein, the term "receptor-mediated endocytosis" refers to the process whereby a ligand/receptor complex is internalized and delivered into the cytosol or transferred to a suitable intracellular compartment, triggered by binding of the ligand to the corresponding receptor on the cell surface. In some embodiments, the antibodies of the present invention trigger TF receptor-mediated endocytosis upon binding to TF expressed on the cell surface. Herein, the receptor-mediated endocytic activity of an antibody can be characterized by measuring the endocytosis rate, for example, by the method described in the examples. In some embodiments, the antibodies of the present invention with receptor-mediated endocytic activity can be used as a tool for transporting an anti-tumor drug into cancer cells in the ADCs of the present invention.

Herein, "sequence identity" refers to the degree to which sequences are identical on a nucleotide-by-nucleotide or amino acid-by-amino acid basis over a comparison window. The "percent sequence identity" can be calculated by the following steps: comparing two optimally aligned sequences over a comparison window; determining a number of positions in which nucleic acid bases (e.g., A, T, C, G, and I) or amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are the same in the two sequences to obtain the number of matched positions; dividing the number of matched positions by the total number of positions over the comparison window (i.e., the window size); and multiplying the result by 100 to obtain a percent sequence identity. Optimal alignment for determining the percent sequence identity can be achieved in a variety of ways known in the art, for example, using publicly available computer software such as BLAST, BLAST-2, ALIGN, or Megalign (DNASTAR) software. Those skilled in the art can determine suitable parameters for alignment of the sequences, including any algorithm necessary to yield optimal alignment within a full-length sequence range or target sequence region being compared.

Herein, with respect to antibody sequences, the percent amino acid sequence identity is determined by optimally aligning candidate antibody sequences to a given antibody sequence, in a preferred embodiment, according to the Kabat numbering scheme. Herein, without specifying the comparison window (i.e., the target antibody region to be compared), it will be suitable for alignment over the full length of a given antibody sequence.

Herein, unless otherwise stated, a reference antibody (BM) refers to an anti-TF antibody constructed using the amino acid sequences (SEQ ID NOs: 107 and 108) of the heavy and light chain variable regions from the Tisotumab antibody moiety disclosed in the patent CN103119065B. In the context of reference to comparison with a reference antibody, the reference antibody will have the same antibody structure as the portion of the antibody to be compared except for the variable region, e.g., the same heavy and light chain constant region sequences when both have heavy and light chain constant region structures.

Herein, the term "halogen" generally refers to fluorine, chlorine, bromine or iodine, and may be, for example, fluorine or chlorine.

The term "alkyl" as used herein refers to a linear or branched saturated hydrocarbyl group consisting of carbon atoms and hydrogen atoms. Specifically, the alkyl group has 1-10 carbon atoms, e.g., 1-8, 1-6, 1-5, 1-4, 1-3, or 1-2 carbon atoms. For example, as used herein, the term "C₁-C₆ alkyl" refers to a linear or branched saturated hydrocarbyl group having 1-6 carbon atoms, examples of which include, e.g., methyl, ethyl, propyl (including *n*-propyl and isopropyl), butyl (including *n*-butyl, isobutyl, *sec-*butyl, or *tert*-butyl), pentyl (including *n*-pentyl, isopentyl, neopentyl), hexyl (including *n*-hexyl, 2-methylpentyl, 3-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl), and the like.

The term "alkenyl" as used herein refers to a linear or branched unsaturated hydrocarbyl group consisting of carbon atoms and hydrogen atoms with at least one double bond. Specifically, the alkenyl group has 2-8, e.g., 2-6, 2-5, 2-4, or 2-3 carbon atoms. For example, as used herein, the term "C₂-C₆ alkenyl" refers to a linear or branched alkenyl group having 2-6 carbon atoms, e.g., vinyl, propenyl, allyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, 1,4-hexadienyl, and the like.

The term "alkynyl" as used herein refers to a linear or branched unsaturated hydrocarbyl group consisting of carbon atoms and hydrogen atoms with at least one triple bond. Specifically, the alkynyl group has 2-8, e.g., 2-6, 2-5, 2-4, or 2-3 carbon atoms. For example, as used herein, the term "C₂-C₆ alkynyl" refers to a linear or branched alkynyl group having 2-6 carbon atoms, e.g., ethynyl, propynyl, propargyl, 1-butynyl, 2-butynyl, 1-pentynyl, 2-pentynyl, 3-pentynyl, 4-methyl-1-pentynyl, 1-hexynyl, 2-hexynyl, 3-hexynyl, 5-methyl-2-hexynyl, and the like.

The term "alkylene" as used herein refers to a divalent group derived from a linear or branched saturated alkane by the removal of two hydrogen atoms from the same carbon atom or two different carbon atoms. Specifically, the alkylene group has 1-10 carbon atoms, e.g., 1-6, 1-5, 1-4, 1-3, or 1-2 carbon atoms. For example, as used herein, the term "C₁-C₆ alkylene" refers to a linear or branched alkylene group having 1-6 carbon atoms, including but not limited to, methylene, ethylene, propylene, butylene, and the like.

The term "alkenylene" as used herein refers to a divalent group derived from a linear or branched unsaturated olefin containing at least one double bond by the removal of two hydrogen atoms from the same carbon atom or two different carbon atoms. Specifically, the alkenylene group has 2-8, e.g., 2-6, 2-5, 2-4, or 2-3 carbon atoms. For example, as used herein, the term "C₂-C₆ alkenylene" refers to a linear or branched alkenylene group having 2-6 carbon atoms, e.g., ethenylene, propenylene, allylene, butenylene, pentenylene, and hexenylene.

The term "alkynylene" as used herein refers to a divalent group derived from a linear or branched unsaturated alkyne containing at least one triple bond by removal of two hydrogen atoms from the same carbon atom or two different carbon atoms. Specifically, the alkynylene group has 2-8, e.g., 2-6, 2-5, 2-4, or 2-3 carbon atoms. For example, as used herein, the term "C₂-C₆ alkynylene" refers to a linear or branched alkynylene group having 2-6 carbon atoms, e.g., ethynylene, propynylene, propargylene, butynylene, pentynylene, and hexynylene.

The term "cycloalkyl" as used herein refers to a monocyclic, fused polycyclic, bridged polycyclic, or spirocyclic non-aromatic monovalent hydrocarbon ring structure having a specified number of ring atoms, which may be saturated or unsaturated, e.g., contains 1 or more double bonds. The cycloalkyl group may contain 3 or more, e.g., 3-18, 3-10, or 3-8 carbon atoms in the ring, and may be, e.g., C₃₋₁₀ cycloalkyl, C₃₋₈ cycloalkyl, C₃₋₆ cycloalkyl, and C₅₋₆ cycloalkyl. Examples of the cycloalkyl group include, but are not limited to, cyclopropyl, cyclobutyl, cyclopentyl, cyclohexyl, cycloheptyl, and the like.

The term "heterocycle" or "heterocyclyl" as used herein refers to a 5- to 20-membered (e.g., 5-to 14-membered, 5- to 8-membered, 5- to 6-membered) aromatic or non-aromatic monocyclic, bicyclic, or polycyclic ring system having 1-4 heteroatom ring members independently selected from N, O, and S. One or more N, C, or S atoms in the heterocycle can be oxidized. Preferably, the heterocycle is a 5- to 10-membered ring system, either monocyclic or fused bicyclic. Representative examples include, but are not limited to, pyrrolidine, azetidine, piperidine, morpholine, tetrahydrofuran, tetrahydropyran, benzofuran, benzothiophene, indole, benzopyrazole, pyrrole, thiophene, furan, thiazole, imidazole, pyrazole, pyrimidine, pyridine, pyrazine, pyridazine, isothiazole, and isoxazole. It should be understood that this term encompasses heteroaryl as defined herein.

The term "aryl" refers to a monocyclic or polycyclic aromatic hydrocarbyl group having 6-20, e.g., 6-12, carbon atoms in the ring moiety. Preferably, the aryl group is (C₆-C₁₀) aryl. Non-limiting examples include phenyl, biphenyl, naphthyl, or tetrahydronaphthyl, each of which may be optionally substituted with 1-4 substituents, e.g., alkyl, trifluoromethyl, cycloalkyl, halogen, hydroxy, alkoxy, acyl, alkyl-C(O)-O-, aryl-O-, heteroaryl-O-, amino, sulfhydryl, alkyl-S-, aryl-S-, nitro, cyano, carboxyl, alkyl-O-C(O)-, carbamoyl, alkyl-S(O)-, sulfonyl, sulfonamido, heterocyclyl, and the like.

The term "heteroaryl" refers to a 5- to 20-membered (e.g., 5- to 14-membered, 5- to 8-membered, 5- to 6-membered) aromatic monocyclic or polycyclic ring system containing 1-4 heteroatoms selected from N, O, and S, which may be substituted or unsubstituted. Preferably, the heteroaryl group is a 5- to 10-membered ring system, either monocyclic or fused bicyclic. Representative heteroaryl groups include 2- or 3-thienyl, 2- or 3-furyl, 2- or 3-pyrrolyl, 2-, 4- or 5-imidazolyl, 3-, 4- or 5-pyrazolyl, 2-, 4- or 5-thiazolyl, 3-, 4- or 5-isothiazolyl, 2-, 4- or 5-oxazolyl, 3-, 4- or 5-isoxazolyl, 3- or 5-1,2,4-triazolyl, 4- or 5-1,2,3-triazolyl, tetrazolyl, 2-, 3- or 4-pyridyl, 3- or 4-pyridazinyl, 3-, 4- or 5-pyrazinyl, 2-pyrazinyl, 2-, 4- or 5-pyrimidinyl.

The term "heteroalkyl" refers to a stable linear or branched hydrocarbon consisting of a specified number of carbon atoms and one to ten, preferably one to three, heteroatoms selected from O, N, Si, and S, either fully saturated or containing 1-3 units of unsaturation, wherein the nitrogen and sulfur atoms may optionally be oxidized and the nitrogen heteroatom may optionally be quaternized. The heteroatoms O, N, Si, and S can be located at any internal position of the heteroalkyl group or at the position where the heteroalkyl group is linked to the rest of the molecule. Representative examples of the heteroalkyl group include -CH2-CH2-O-CH3, -CH2-CH2-NH-CH3, -CH2-CH2-N(CH3)-CH3, -CH2-S-CH2-CH3, -CH2-CH2-S(O)-CH3, -NH-CH2-CH2-NH-C(O)-CH2-CH3, -CH2-CH2-S(O)2-CH3, -CH=CH-O-CH3, -Si(CH3)3, -CH2-CH=N-O-CH3, and -CH=CH-N(CH3)-CH3. Up to two heteroatoms may be consecutive, e.g., -CH2-NH-OCH3 and -CH2-O-Si(CH3)3. In general, the C1-C4 heteroalkyl or heteroalkylene group has 1-4 carbon atoms and 1 or 2 heteroatoms and the C1-C3 heteroalkyl or heteroalkylene group has 1-3 carbon atoms and 1 or 2 heteroatoms. In some aspects, the heteroalkyl and heteroalkylene groups are saturated.

Unless otherwise indicated, the term "substituted" as used herein in the definition of each group means that the corresponding group may be substituted with, for example, but not limited to, the following groups: alkyl, alkenyl, alkynyl, cycloalkyl, aryl, heteroaryl, heterocyclyl, halogen, cyano, nitro, azido, carboxyl, hydroxy, sulfhydryl, amino, mono- or dialkylamino, mono- or dicycloalkylamino, mono- or diarylamino, mono- or diheteroylamino, mono- or diheteroarylamino, alkyl- or cycloalkyl- or heterocyclyl- or heteroaryl- or aryl-oxy, alkyl- or cycloalkyl- or heterocyclyl- or heteroaryl- or aryl-thio, alkyl- or cycloalkyl- or heterocyclyl- or heteroaryl- or aryl-acyl, alkyl- or cycloalkyl- or heterocyclyl- or heteroaryl- or aryl-acylamino, alkyl- or cycloalkyl- or heterocyclyl- or heteroaryl- or aryl-acyloxy, alkyl- or cycloalkyl- or heterocyclyl- or heteroaryl- or aryl-sulfonyl, alkyl- or cycloalkyl- or heterocyclyl- or heteroaryl- or aryl-sulfonyloxy, alkyl- or cycloalkyl- or heterocyclyl- or heteroaryl- or aryl-sulfonylamino, or the amino-formyl groups optionally substituted as described above, and respective groups thereof further substituted by the remaining optional substituents, wherein the respective groups are as defined herein. Examples of substituents include, but are not limited to, one or more groups independently selected from the following: halogen, OH, SH, CN, NH₂, NHCH₃, N(CH₃)₂, NO₂, N₃, C(O)CH₃, COOH, C(O)-amino, OCOCH₃, methyl, ethyl, propyl, isopropyl, butyl, isobutyl, sec-butyl, tert-butyl, cyclopropyl, methoxy, ethoxy, propoxy, oxo, trifluoromethyl, difluoromethyl, sulfonylamino, methylsulfonylamino, SO, SO₂, phenyl, piperidinyl, piperazinyl, and pyrimidinyl.

The term "substitution" or "substituted" as used herein means that one or more (e.g., 1, 2, 3, or 4) hydrogens on a specified atom is replaced with a designated group, provided that the normal valency of the specified atom under the present circumstances is not exceeded and a stable compound is formed, and the combination of a substituent and a variable is permitted only when such a combination forms a stable compound.

As used herein, the term "linker" refers to a bifunctional moiety that links a drug to an antibody in a drug-antibody conjugate. The linker of the present invention may have multiple components (e.g., in some embodiments, a linking group responsible for coupling to an antibody; a degradable peptide unit; and optionally a spacer group).

As used herein, the term "PEG unit" refers to an organic moiety comprising repeating ethylene-oxy subunits (PEG or PEG subunits), which may be polydisperse, monodisperse, or discrete (i.e., having a discrete number of ethylene-oxy subunits). Polydisperse PEG is a heterogeneous mixture of various sizes and molecular weights, whereas monodisperse PEG is generally purified from a heterogeneous mixture and thus has unique chain length and molecular weight. The PEG unit comprises discrete PEG, which is a compound synthesized in a stepwise manner rather than through a polymerization process. The discrete PEG provides a single molecule with a defined and specified chain length.

The term "pharmaceutical acceptable salt" refers to a salt that retains the biological effects and properties of the ADCs of the present invention, and is not biologically or otherwise undesired. The ADCs of the present invention may exist in their pharmaceutical acceptable salt forms, including acid addition salts and base addition salts. In the present invention, pharmaceutically acceptable non-toxic acid addition salts refer to salts formed by reaction of the ADCs of the present invention with organic or inorganic acids including, but not limited to, hydrochloric acid, sulfuric acid, hydrobromic acid, hydroiodic acid, phosphoric acid, nitric acid, perchloric acid, acetic acid, oxalic acid, maleic acid, fumaric acid, tartaric acid, benzenesulfonic acid, methanesulfonic acid, salicylic acid, succinic acid, citric acid, lactic acid, propionic acid, benzoic acid, *p*-toluenesulfonic acid, malic acid, etc. Pharmaceutically acceptable non-toxic base addition salts refer to salts formed by the ADCs of the present invention with organic or inorganic bases including, but not limited to, alkali metal salts, e.g., lithium, sodium or potassium salts; alkaline earth metal salts, e.g., calcium or magnesium salts; organic base salts, e.g., ammonium salts, formed with N group-containing organic bases.

The term "solvate" refers to an association compound formed by the ADC of the present invention with one or more solvent molecules. Solvents for forming solvates include, but are not limited to, water, methanol, ethanol, isopropanol, ethyl acetate, tetrahydrofuran, *N,N-*dimethylformamide, dimethylsulfoxide, etc.

"Pharmaceutically acceptable" and "pharmaceutical" are used interchangeably herein, provided that there is no contradiction according to the context.

The term "drug-to-antibody ratio" or "DAR" refers to the ratio of the drug moiety (D) conjugated to the Ab moiety described herein to the Ab moiety in an ADC. In some embodiments described herein, DAR may be determined by q in formula I, for example, the DAR may be 1-20, e.g., 2-18, 4-16, 5-12, 6-10, 2-8, 3-8, 2-6, 4-6, 6-10, e.g., 2, 3, 4, 5, 6, 7, 8, 9, 10, 11, 12, 13, 14, or 15. DAR may also be calculated as the average DAR of the molecular population in the product, i.e., the overall ratio of the small molecule drug moiety (D) conjugated to the Ab moiety described herein to the Ab moiety in the product as measured by detection methods (e.g., by conventional methods such as mass spectrometry, ELISA assay, electrophoresis, and/or HPLC), and such DAR is referred to herein as average DAR. In some embodiments, the average DAR value of the conjugate of the present invention is 1-20, e.g., 2-18, 4-16, 5-12, 6-10, 2-8, 3-8, 2-6, or 4-6, 6-10, e.g., 1.0-8.0 or 2.0-6.0, e.g., 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8.0, 7.9, 8, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, or 10.0, and ranges with any two of these values as endpoints.

The term "drug" as used herein encompasses any substance that is effective in preventing or treating tumors, e.g., cancer, including chemotherapeutic agents, cytokines, angiogenesis inhibitors, cytotoxic agents, other antibodies, small molecule drugs, or immunomodulatory agents (e.g., immunosuppressive agents).

The term "cytotoxic agent" as used herein refers to a substance that inhibits or prevents cell functions and/or causes cell death or destruction. Examples of cytotoxic agents include, but are not limited to, camptothecin drugs, auristatin, aureomycin, maytansinoids, ricin, ricin A chain, combrestatin, duocarmycin, dolastatin, aplysiatoxin, doxorubicin, daunomycin, taxol, cisplatin, cc1065, ethidium bromide, mitomycin, etoposide, tenoposide, vincristine, vinblastine, colchicine, dihydroxy anthracin dione, actinomycin, diphtherin, pseudomonas exotoxin (PE) A, PE40, abrin, abrin A chain, modeccin A chain, α-sarcin, gelonin, mitogellin, retstrictocin, phenomycin, enomycin, curicin, crotin, calicheamicin, *Sapaonaria officinalis* inhibitors and glucocorticoids and other therapeutic agents, and radioisotopes, e.g., At211, I131, I125, Y90, Re186, Re188, Sm153, Bi212 or 213, P32, and radioisotopes of Lu, including Lu177.

The term "small molecule drug" refers to a low molecular weight organic compound capable of regulating biological processes. "Small molecule" is defined as a molecule with a molecular weight of less than 10 kD, generally less than 2 kD and preferably less than 1 kD. The small molecule includes, but is not limited to, inorganic molecules, organic molecules, organic molecules containing inorganic components, molecules containing radioactive atoms, synthetic molecules, peptide mimetics, and antibody mimetics. As therapeutic agents, small molecules penetrate cells better, are less susceptible to degradation and are less likely to induce an immune response compared with large molecules.

The term "pharmaceutical composition" refers to such a composition that exists in a form allowing effective biological activity of the active ingredient contained therein, and does not contain additional ingredients having unacceptable toxicity to a subject to which the composition is administered.

The term "pharmaceutical auxiliary material" refers to diluents, adjuvants (e.g., Freund's adjuvants (complete and incomplete)), excipients, carriers, stabilizers, or the like, which are administered with the active substance.

The term "pharmaceutical combination" refers to a non-fixed combination product or a fixed combination product, including but not limited to a kit and a pharmaceutical composition. The term "non-fixed combination" means that two or more active ingredients (e.g., (i) the ADC molecule of the present invention or the antibody of the present invention, and (ii) other therapeutic agents) are administered to a patient as separate entities, either simultaneously or sequentially, without specific time limitation or at the same or different time intervals, wherein such administration provides prophylactically or therapeutically effective levels of the two or more active ingredients in the patient. The term "fixed combination" means that two or more active ingredients are administered to a patient as a single entity simultaneously. In some cases involving pharmaceutical combinations, it is preferred to select the dosage and/or time interval at which two or more active ingredients are administered so that the combined use of the ingredients can result in a greater effect than that would be achieved by the use of either ingredient alone in the treatment of a disease or condition. The ingredients contained in the pharmaceutical combination may each be in a separate formulation form, and the formulation form may be the same or different. In some embodiments, the ADC molecule of the present invention or the antibody of the present invention and other therapeutic agents used in the pharmaceutical combination are administered at a level not exceeding that when they are used alone.

The term "combination therapy" or "therapeutic combination" refers to the administration of two or more therapeutic agents or modalities (e.g., radiation therapy or surgery) to treat the diseases described herein. Such administration includes co-administration of the therapeutic agents in a substantially simultaneous manner, e.g., administration of a single capsule having a fixed proportion of two or more active ingredients. Alternatively, such administration includes co-administration of two or more active ingredients in multiple formulations (e.g., a tablet, a capsule, a powder, and a liquid) or in separate containers. The powder and/or liquid can be reconstituted or diluted to a desired dose before administration. In addition, such administration also includes using each type of the therapeutic agents at approximately the same time or in a sequential manner at different times. In any case, the treatment regimen will provide the beneficial effects of the therapeutic combinations in treating the disorders or conditions described herein.

Herein, the terms "individual", "subject", and "patient" are used interchangeably to refer to a mammal. Mammals include, but are not limited to, domesticated animals (e.g., cows, sheep, cats, dogs, and horses), primates (e.g., human and non-human primates such as monkeys), rabbits, and rodents (e.g., mice and rats). Preferably, the subject is a human.

The terms "tumor" and "cancer" are used interchangeably herein to refer to a physiological disease in mammals that is generally characterized by unregulated cell growth. This term encompasses both primary and metastatic forms of a tumor. This term also encompasses all neoplastic (neoplastic) cell growth and proliferation, whether malignant or benign, and all pre-cancerous and cancerous cells and tissues.

The term "anti-tumor effect" refers to an anti-tumor biological effect that can be exhibited by a variety of means, including but not limited to, for example, decrease in tumor volume, decrease in tumor cell number, decrease in tumor cell proliferation, or decrease in tumor cell survival.

As used herein, "treatment" (or "treat" or "treating") refers to slowing, interrupting, arresting, alleviating, stopping, lowering, or reversing the progression or severity of an existing symptom, disorder, condition, or disease.

As used herein, "prevention" (or "prevent" or "preventing") includes the inhibition of the development or progression of symptoms of a disease or disorder, or a specific disease or disorder. In general, in the context of cancer, the term "prevention" (or "prevent" or "preventing") refers to the administration of a drug prior to the onset of signs or symptoms of cancer, particularly in a subject at risk for cancer. In some embodiments, subjects with family history of cancer are candidates for preventive regimens.

As used herein, the term "effective amount" refers to an amount of a drug (e.g., the antibody-drug conjugate or the pharmaceutical acceptable salt or solvate of the present invention, or the antibody or the antigen-binding fragment thereof of the present invention, or a composition or pharmaceutical combination thereof) that produces the desired effect in a patient in need of treatment or prevention following administration of the patient in a single or multiple doses.

As used herein, the term "therapeutically effective amount" refers to an amount effective to achieve the desired therapeutic result at the required dosage and for the required period of time. The therapeutically effective amount is also an amount wherein, upon administration of the amount to a subject, any toxic or deleterious effects brought about by the administration will be less than the therapeutically beneficial effects it brings about. "Therapeutically effective amount" preferably achieves an improvement in a measurable parameter (e.g., tumor volume) of at least about 30%, even more preferably at least about 40%, 45%, 50%, 55%, 60%, 65%, 70%, 75%, 80%, 85%, 90%, or even 100% relative to an untreated individual.

As used herein, the term "prophylactically effective amount" refers to an amount effective to achieve the desired prophylactic result at the required dosage and for the required period of time. In general, since a prophylactic dose is used in a subject prior to or at an earlier stage of disease, the prophylactically effective amount will be less than the therapeutically effective amount.

Aspects of the present invention will be described in further detail in the following subsections.

### Antibody-drug conjugate of the present invention

### I. Antibody-drug conjugate

In one aspect, the present invention provides an antibody-drug conjugate (ADC) having the following formula (I), or a pharmaceutical acceptable salt or solvate thereof:

Ab-[L-D]_{q} (I)

wherein,
Ab represents an anti-TF antibody,
L represents a linker,
D represents a cytotoxic or cytostatic drug, e.g., a topoisomerase I inhibitor, and
q represents an integer or decimal of 1-20, e.g., q = 1-10, 1-8, 3-8, 4-8, or 6-8.

Components of the ADC of the present invention and the ADC of the present invention consisting of the components are described in detail below. It will be understood by those skilled in the art that any combination of any of the technical features of these components is within the contemplation of the present invention, unless otherwise expressly stated to the contrary. Furthermore, it will be understood by those skilled in the art that the ADC of the present invention may comprise any such combination of features, unless otherwise expressly stated to the contrary.

### Ab unit of antibody

In some aspects, the present invention provides an antibody-drug conjugate (ADC) comprising the antibody of the present invention that specifically binds to TF as an Ab unit of the conjugate of formula I of the present invention. The anti-TF antibody of the present invention used as the Ab unit may be a full-length antibody or an antibody fragment. In some embodiments, the Ab in formula (I) of the present invention is a full-length antibody comprising a heavy chain constant region and a light chain constant region. In some embodiments, the Ab in formula (I) of the present invention has a tetrameric structure formed by two light chains and two heavy chains. In still other embodiments, the Ab in formula (I) of the present invention is an IgG antibody, particularly an IgG1 antibody. In a further embodiment, the Ab in formula (I) of the present invention is a humanized antibody.

### Properties of antibody

The anti-TF antibody of the present invention used in the ADC of the present invention has one or more of the following properties:
(i) binding to the extracellular domain of human TF with high affinity;
(ii) binding to TF-positive tumor cells with high affinity;
(iii) having cross-reactivity with cynomolgus monkey TF;
(iv) having TF receptor-mediated endocytic activity;
(v) blocking the TF/FVIIa complex-mediated downstream signaling pathway in TF-positive cells; and
(vi) not substantially affecting coagulation initiated by TF on the cell surface, or having a reduced coagulation effect.

Preferably, the anti-TF antibody and the antibody-drug conjugate can bind to TF-positive tumor cells with high affinity, and have substantially no effect on coagulation initiated by TF on the cell surface or have a reduced coagulation effect.

For the properties of the anti-TF antibody of the present invention used in the ADC of the present invention, see also the detailed description provided below in the section "Anti-TF antibody of the present invention".

### CDRs of antibody

CDRs are regions in the antibody that are primarily responsible for binding to antigenic epitopes. In some embodiments, the anti-TF antibody used in the ADC of the present invention comprises the following CDR sequences:
(i) HCDR1, 2 and 3 sequences of a heavy chain variable region set forth in SEQ ID NO: 1, and LCDR1, 2 and 3 sequences of a light chain variable region set forth in SEQ ID NO: 2; or
(ii) HCDR1, 2 and 3 sequences of a heavy chain variable region set forth in SEQ ID NO: 3, and LCDR1, 2 and 3 sequences of a light chain variable region set forth in SEQ ID NO: 4; or
(iii) HCDR1, 2 and 3 sequences of a heavy chain variable region set forth in SEQ ID NO: 5, and LCDR1, 2 and 3 sequences of a light chain variable region set forth in SEQ ID NO: 6; or
(iv) HCDR1, 2 and 3 sequences of a heavy chain variable region set forth in SEQ ID NO: 7, and LCDR1, 2 and 3 sequences of a light chain variable region set forth in SEQ ID NO: 8.

Preferably, the CDRs are defined according to Chothia, AbM, Kabat, IMGT, or any combination thereof. More preferably, the CDRs are defined according to Kabat or IMGT, or a combination thereof, and still more preferably, the CDRs are defined according to IMGT. It should be understood that the CDRs may also be defined in any other methods known in the art.

In addition, it is known in the art that although CDRs vary from antibody to antibody, only a limited number of amino acid positions within the CDRs are directly involved in antigen binding. The smallest overlapping region can be determined using at least two of the Kabat, Chothia, AbM, and Contact methods, thereby providing a "minimal binding unit" for antigen binding. Such minimal binding unit may be a sub-portion of the CDR. As will be understood by those skilled in the art, residues in the rest of the CDR sequences can be determined by the antibody structure and protein folding. Thus, any variants of the CDRs presented herein are also contemplated by the present invention. For example, in a variant of the CDR, the amino acid residue of the minimal binding unit may remain unchanged, while the remaining CDR residues may be substituted.

In some embodiments, preferably, the anti-TF antibody used in the ADC of the present invention comprises 3 complementarity determining regions of a heavy chain variable region (HCDRs), and 3 complementarity determining regions of a light chain variable region (LCDRs), wherein:
(i) according to the Chothia scheme, the HCDR1 comprises or consists of an amino acid sequence set forth in SEQ ID NO: 9, the HCDR2 comprises or consists of an amino acid sequence set forth in SEQ ID NO: 10, the HCDR3 comprises or consists of an amino acid sequence set forth in SEQ ID NO: 11, the LCDR1 comprises or consists of an amino acid sequence set forth in SEQ ID NO: 21, the LCDR2 comprises or consists of an amino acid sequence set forth in SEQ ID NO: 22, and the LCDR3 comprises or consists of an amino acid sequence set forth in SEQ ID NO: 23; or
(ii) according to the AbM scheme, the HCDR1 comprises or consists of an amino acid sequence set forth in SEQ ID NO: 12, the HCDR2 comprises or consists of an amino acid sequence set forth in SEQ ID NO: 13, the HCDR3 comprises or consists of an amino acid sequence set forth in SEQ ID NO: 14, the LCDR1 comprises or consists of an amino acid sequence set forth in SEQ ID NO: 24, the LCDR2 comprises or consists of an amino acid sequence set forth in SEQ ID NO: 25, and the LCDR3 comprises or consists of an amino acid sequence set forth in SEQ ID NO: 26; or
(iii) according to the Kabat scheme, the HCDR1 comprises or consists of an amino acid sequence set forth in SEQ ID NO: 15, the HCDR2 comprises or consists of an amino acid sequence set forth in SEQ ID NO: 16, the HCDR3 comprises or consists of an amino acid sequence set forth in SEQ ID NO: 17, the LCDR1 comprises or consists of an amino acid sequence set forth in SEQ ID NO: 27, the LCDR2 comprises or consists of an amino acid sequence set forth in SEQ ID NO: 28, and the LCDR3 comprises or consists of an amino acid sequence set forth in SEQ ID NO: 29; or
(iv) according to the IMGT scheme, the HCDR1 comprises or consists of an amino acid sequence set forth in SEQ ID NO: 18, the HCDR2 comprises or consists of an amino acid sequence set forth in SEQ ID NO: 19, the HCDR3 comprises or consists of an amino acid sequence set forth in SEQ ID NO: 20, the LCDR1 comprises or consists of an amino acid sequence set forth in SEQ ID NO: 30, the LCDR2 comprises or consists of an amino acid sequence set forth in SEQ ID NO: 31, and the LCDR3 comprises or consists of an amino acid sequence set forth in SEQ ID NO: 32.

In some embodiments, preferably, the anti-TF antibody used in the ADC of the present invention comprises 3 complementarity determining regions of a heavy chain variable region (HCDRs), and 3 complementarity determining regions of a light chain variable region (LCDRs), wherein:
(i) according to the Chothia scheme, the HCDR1 comprises or consists of an amino acid sequence set forth in SEQ ID NO: 33, the HCDR2 comprises or consists of an amino acid sequence set forth in SEQ ID NO: 34, the HCDR3 comprises or consists of an amino acid sequence set forth in SEQ ID NO: 35, the LCDR1 comprises or consists of an amino acid sequence set forth in SEQ ID NO: 45, the LCDR2 comprises or consists of an amino acid sequence set forth in SEQ ID NO: 46, and the LCDR3 comprises or consists of an amino acid sequence set forth in SEQ ID NO: 47; or
(ii) according to the AbM scheme, the HCDR1 comprises or consists of an amino acid sequence set forth in SEQ ID NO: 36, the HCDR2 comprises or consists of an amino acid sequence set forth in SEQ ID NO: 37, the HCDR3 comprises or consists of an amino acid sequence set forth in SEQ ID NO: 38, the LCDR1 comprises or consists of an amino acid sequence set forth in SEQ ID NO: 48, the LCDR2 comprises or consists of an amino acid sequence set forth in SEQ ID NO: 49, and the LCDR3 comprises or consists of an amino acid sequence set forth in SEQ ID NO: 50; or
(iii) according to the Kabat scheme, the HCDR1 comprises or consists of an amino acid sequence set forth in SEQ ID NO: 39, the HCDR2 comprises or consists of an amino acid sequence set forth in SEQ ID NO: 40, the HCDR3 comprises or consists of an amino acid sequence set forth in SEQ ID NO: 41, the LCDR1 comprises or consists of an amino acid sequence set forth in SEQ ID NO: 51, the LCDR2 comprises or consists of an amino acid sequence set forth in SEQ ID NO: 52, and the LCDR3 comprises or consists of an amino acid sequence set forth in SEQ ID NO: 53; or
(iv) according to the IMGT scheme, the HCDR1 comprises or consists of an amino acid sequence set forth in SEQ ID NO: 42, the HCDR2 comprises or consists of an amino acid sequence set forth in SEQ ID NO: 43, the HCDR3 comprises or consists of an amino acid sequence set forth in SEQ ID NO: 44, the LCDR1 comprises or consists of an amino acid sequence set forth in SEQ ID NO: 54, the LCDR2 comprises or consists of an amino acid sequence set forth in SEQ ID NO: 55, and the LCDR3 comprises or consists of an amino acid sequence set forth in SEQ ID NO: 56.

In some embodiments, preferably, the anti-TF antibody used in the ADC of the present invention comprises 3 complementarity determining regions of a heavy chain variable region (HCDRs), and 3 complementarity determining regions of a light chain variable region (LCDRs), wherein:
(i) according to the Chothia scheme, the HCDR1 comprises or consists of an amino acid sequence set forth in SEQ ID NO: 57, the HCDR2 comprises or consists of an amino acid sequence set forth in SEQ ID NO: 58, the HCDR3 comprises or consists of an amino acid sequence set forth in SEQ ID NO: 59, the LCDR1 comprises or consists of an amino acid sequence set forth in SEQ ID NO: 69, the LCDR2 comprises or consists of an amino acid sequence set forth in SEQ ID NO: 70, and the LCDR3 comprises or consists of an amino acid sequence set forth in SEQ ID NO: 71; or
(ii) according to the AbM scheme, the HCDR1 comprises or consists of an amino acid sequence set forth in SEQ ID NO: 60, the HCDR2 comprises or consists of an amino acid sequence set forth in SEQ ID NO: 61, the HCDR3 comprises or consists of an amino acid sequence set forth in SEQ ID NO: 62, the LCDR1 comprises or consists of an amino acid sequence set forth in SEQ ID NO: 72, the LCDR2 comprises or consists of an amino acid sequence set forth in SEQ ID NO: 73, and the LCDR3 comprises or consists of an amino acid sequence set forth in SEQ ID NO: 74; or
(iii) according to the Kabat scheme, the HCDR1 comprises or consists of an amino acid sequence set forth in SEQ ID NO: 63, the HCDR2 comprises or consists of an amino acid sequence set forth in SEQ ID NO: 64, the HCDR3 comprises or consists of an amino acid sequence set forth in SEQ ID NO: 65, the LCDR1 comprises or consists of an amino acid sequence set forth in SEQ ID NO: 75, the LCDR2 comprises or consists of an amino acid sequence set forth in SEQ ID NO: 76, and the LCDR3 comprises or consists of an amino acid sequence set forth in SEQ ID NO: 77; or
(iv) according to the IMGT scheme, the HCDR1 comprises or consists of an amino acid sequence set forth in SEQ ID NO: 66, the HCDR2 comprises or consists of an amino acid sequence set forth in SEQ ID NO: 67, the HCDR3 comprises or consists of an amino acid sequence set forth in SEQ ID NO: 68, the LCDR1 comprises or consists of an amino acid sequence set forth in SEQ ID NO: 78, the LCDR2 comprises or consists of an amino acid sequence set forth in SEQ ID NO: 79, and the LCDR3 comprises or consists of an amino acid sequence set forth in SEQ ID NO: 80.

In some embodiments, preferably, the anti-TF antibody used in the ADC of the present invention comprises 3 complementarity determining regions of a heavy chain variable region (HCDRs), and 3 complementarity determining regions of a light chain variable region (LCDRs), wherein:
(i) according to the Chothia scheme, the HCDR1 comprises or consists of an amino acid sequence set forth in SEQ ID NO: 81, the HCDR2 comprises or consists of an amino acid sequence set forth in SEQ ID NO: 82, the HCDR3 comprises or consists of an amino acid sequence set forth in SEQ ID NO: 83, the LCDR1 comprises or consists of an amino acid sequence set forth in SEQ ID NO: 93, the LCDR2 comprises or consists of an amino acid sequence set forth in SEQ ID NO: 94, and the LCDR3 comprises or consists of an amino acid sequence set forth in SEQ ID NO: 95; or
(ii) according to the AbM scheme, the HCDR1 comprises or consists of an amino acid sequence set forth in SEQ ID NO: 84, the HCDR2 comprises or consists of an amino acid sequence set forth in SEQ ID NO: 85, the HCDR3 comprises or consists of an amino acid sequence set forth in SEQ ID NO: 86, the LCDR1 comprises or consists of an amino acid sequence set forth in SEQ ID NO: 96, the LCDR2 comprises or consists of an amino acid sequence set forth in SEQ ID NO: 97, and the LCDR3 comprises or consists of an amino acid sequence set forth in SEQ ID NO: 98; or
(iii) according to the Kabat scheme, the HCDR1 comprises or consists of an amino acid sequence set forth in SEQ ID NO: 87, the HCDR2 comprises or consists of an amino acid sequence set forth in SEQ ID NO: 88, the HCDR3 comprises or consists of an amino acid sequence set forth in SEQ ID NO: 89, the LCDR1 comprises or consists of an amino acid sequence set forth in SEQ ID NO: 99, the LCDR2 comprises or consists of an amino acid sequence set forth in SEQ ID NO: 100, and the LCDR3 comprises or consists of an amino acid sequence set forth in SEQ ID NO: 101; or
(iv) according to the IMGT scheme, the HCDR1 comprises or consists of an amino acid sequence set forth in SEQ ID NO: 90, the HCDR2 comprises or consists of an amino acid sequence set forth in SEQ ID NO: 91, the HCDR3 comprises or consists of an amino acid sequence set forth in SEQ ID NO: 92, the LCDR1 comprises or consists of an amino acid sequence set forth in SEQ ID NO: 102, the LCDR2 comprises or consists of an amino acid sequence set forth in SEQ ID NO: 103, and the LCDR3 comprises or consists of an amino acid sequence set forth in SEQ ID NO: 104.

### Variable regions of antibody

"Variable region" or "variable domain" is a domain in the heavy chain or light chain of an antibody that participates in binding of the antibody to the antigen thereof. In some embodiments, the anti-TF antibody of the present invention used in the ADC of the present invention comprises: heavy and light chain variable region sequences or variants thereof of any one of the exemplary antibodies 2B12B10-hz1, 30G11B7-hz1, 22F11H5-hz1, and 27H8H3-hz1 of the present invention, for example, an antibody or a fragment thereof having the same CDR sequences as one of the exemplary antibodies, and having the same or different framework region sequences, e.g., a humanized antibody. For substitutions in framework regions, protein sequences of the parent antibody can be compared to protein sequences in the database using a sequence similarity search tool, such as Gapped BLAST. Framework sequences in the database having a high degree of structural similarity to the framework sequences of the parent antibody to be altered, e.g., having at least 80%, 85%, 90%, or 95%, 96%, 97%, 98%, 99% or more sequence identity, are selected for the substitutions in the framework regions. In some cases, after the substitutions in the framework regions, the substituted framework sequences may be subjected to one to several residue mutations, e.g., back mutations, as desired, and the mutated antibody may be evaluated for retention or improvement of antigen-binding properties or other functional properties in an *in vitro* or *in vivo* assay.

In one embodiment, the anti-TF antibody of the present invention used in the ADC of the present invention comprises a heavy chain variable region and a light chain variable region, wherein: the heavy chain variable region comprises:
(i) an amino acid sequence set forth in SEQ ID NO: 1 or an amino acid sequence having at least 80%, 85%, 90%, 95%, 96%, 97%, 98% or 99% sequence identity thereto; or
(ii) an amino acid sequence set forth in SEQ ID NO: 3 or an amino acid sequence having at least 80%, 85%, 90%, 95%, 96%, 97%, 98% or 99% sequence identity thereto; or
(iii) an amino acid sequence set forth in SEQ ID NO: 5 or an amino acid sequence having at least 80%, 85%, 90%, 95%, 96%, 97%, 98% or 99% sequence identity thereto; or
(iv) an amino acid sequence set forth in SEQ ID NO: 7 or an amino acid sequence having at least 80%, 85%, 90%, 95%, 96%, 97%, 98% or 99% sequence identity thereto.

In another embodiment, the anti-TF antibody used in the ADC of the present invention comprises a heavy chain variable region and a light chain variable region, wherein: the light chain variable region comprises:
(i) an amino acid sequence set forth in SEQ ID NO: 2 or an amino acid sequence having at least 80%, 85%, 90%, 95%, 96%, 97%, 98% or 99% sequence identity thereto; or
(ii) an amino acid sequence set forth in SEQ ID NO: 4 or an amino acid sequence having at least 80%, 85%, 90%, 95%, 96%, 97%, 98% or 99% sequence identity thereto; or
(iii) an amino acid sequence set forth in SEQ ID NO: 6 or an amino acid sequence having at least 80%, 85%, 90%, 95%, 96%, 97%, 98% or 99% sequence identity thereto; or
(iv) an amino acid sequence set forth in SEQ ID NO: 8 or an amino acid sequence having at least 80%, 85%, 90%, 95%, 96%, 97%, 98% or 99% sequence identity thereto.

In some preferred embodiments, the anti-TF antibody comprises a heavy chain variable region and a light chain variable region, wherein the heavy chain variable region comprises an amino acid sequence set forth in SEQ ID NO: 1 or an amino acid sequence having at least 95%, 96%, 97%, 98% or 99% sequence identity thereto, and the light chain variable region comprises an amino acid sequence set forth in SEQ ID NO: 2 or an amino acid sequence having at least 95%, 96%, 97%, 98% or 99% sequence identity thereto. Preferably, the antibody or the antigen-binding fragment comprises or consists of a heavy chain variable region of SEQ ID NO: 1 and a light chain variable region of SEQ ID NO: 2.

In some preferred embodiments, the anti-TF antibody comprises a heavy chain variable region and a light chain variable region, wherein the heavy chain variable region comprises an amino acid sequence set forth in SEQ ID NO: 3 or an amino acid sequence having at least 95%, 96%, 97%, 98% or 99% sequence identity thereto, and the light chain variable region comprises an amino acid sequence set forth in SEQ ID NO: 4 or an amino acid sequence having at least 95%, 96%, 97%, 98% or 99% sequence identity thereto. Preferably, the antibody or the antigen-binding fragment comprises or consists of a heavy chain variable region of SEQ ID NO: 3 and a light chain variable region of SEQ ID NO: 4.

In some preferred embodiments, the anti-TF antibody comprises a heavy chain variable region and a light chain variable region, wherein the heavy chain variable region comprises an amino acid sequence set forth in SEQ ID NO: 5 or an amino acid sequence having at least 95%, 96%, 97%, 98% or 99% sequence identity thereto, and the light chain variable region comprises an amino acid sequence set forth in SEQ ID NO: 6 or an amino acid sequence having at least 95%, 96%, 97%, 98% or 99% sequence identity thereto. Preferably, the antibody or the antigen-binding fragment comprises or consists of a heavy chain variable region of SEQ ID NO: 5 and a light chain variable region of SEQ ID NO: 6.

In some preferred embodiments, the anti-TF antibody comprises a heavy chain variable region and a light chain variable region, wherein the heavy chain variable region comprises an amino acid sequence set forth in SEQ ID NO: 7 or an amino acid sequence having at least 95%, 96%, 97%, 98% or 99% sequence identity thereto, and the light chain variable region comprises an amino acid sequence set forth in SEQ ID NO: 8 or an amino acid sequence having at least 95%, 96%, 97%, 98% or 99% sequence identity thereto. Preferably, the antibody or the antigen-binding fragment comprises or consists of a heavy chain variable region of SEQ ID NO: 7 and a light chain variable region of SEQ ID NO: 8.

### Heavy and light chains of antibody

The anti-TF antibody of the present invention used as the Ab unit in the ADC of the present invention, in some cases, may comprise a heavy chain constant region and/or a light chain constant region. Preferably, the heavy chain constant region is a heavy chain constant region derived from a human immunoglobulin. Preferably, the light chain constant region is a light chain constant region derived from a human immunoglobulin. In some embodiments, the heavy chain constant region contained in the anti-TF antibody may be of any isotype or subtype, such as a heavy chain constant region of IgG1, IgG2, IgG3 or IgG4 isotype. In some embodiments, the anti-TF antibody preferably comprises an IgG1 heavy chain constant region, particularly a human IgG1 heavy chain constant region, e.g., an amino acid sequence set forth in SEQ ID NO: 106 or an amino acid sequence having at least 90%, 95%, 96%, 97%, 98% or 99% sequence identity thereto. In other embodiments, the light chain constant region contained in the anti-TF antibody may be a κ light chain constant region or a λ light chain constant region. In some embodiments, the anti-TF antibody comprises a human-derived κ light chain constant region or λ light chain constant region, preferably a human κ light chain constant region, e.g., an amino acid sequence set forth in SEQ ID NO: 105 or an amino acid sequence having at least 90%, 95%, 96%, 97%, 98% or 99% sequence identity thereto.

Variants of any of the above antibody embodiments are also contemplated by the present invention. An antibody variant that can be used as the Ab unit according to the present invention preferably retains at least 60%, 70%, 80%, 90%, or 100% of the biological activity (e.g., antigen-binding ability) of the parent antibody prior to the introduction of an alteration. More preferably, the alteration does not result in loss of binding ability of the antibody variant to an antigen, but optionally confers properties such as increased affinity for the antigen. It will be understood that the heavy chain variable regions or the light chain variable regions, or the CDRs or the framework regions of the antibodies may be altered independently or in combination. In addition, the Fc regions of the antibodies may also be altered. The alterations in the Fc regions may be made alone or in combination with the alterations in the framework regions and/or CDRs. The Fc regions may be altered, for example, to alter one or more effector functions of the antibody, such as serum half-life, complement fixation, Fc receptor binding, and/or antigen-dependent cytotoxicity. In addition, the antibody of the present invention may be chemically modified (e.g., linked to PEG), or its glycosylation pattern may be altered.

To form an ADC, the antibody according to the present invention may be conjugated to a toxin-linker using some natural conjugation sites on the antibody. Such natural conjugation sites include a sulfhydryl group of cysteine and an amino group of lysine. Typically, a more defined drug-to-antibody ratio (DAR) can be achieved by using interchain disulfide bridges of the antibody. Thus, in one embodiment, after reduction of the interchain disulfide bonds of the antibody, toxins are conjugated to the antibody of the present invention by sulfhydryl chemistry to form an ADC of formula (I). In addition, it is also contemplated to introduce artificial conjugation sites into the antibody to achieve more site-directed conjugation.

### Drug D unit

A drug D unit of the antibody-drug conjugate is also referred to herein as a payload of an ADC drug. The drug D that can be used in the ADC of the present invention is not particularly limited, and may be any drug that is toxic or inhibitory to cells or a prodrug thereof. Those skilled in the art can select a suitable drug molecule as the payload of the ADC based on the desired ADC drug action mechanism and cell killing effect.

In some embodiments, the drug D is a cytotoxic agent. A variety of cytotoxic agents suitable for use as payloads with different mechanisms have been reported in the art, including, but not limited to:
(1) microtubule inhibitors/disruptors: for example, but not limited to, auristatins (e.g., MMAE or MMAF), maytansine derivatives (e.g., DM2, DM4), tubulysins, cryptomycins, and anti-mitotic EG5 inhibitors (e.g., kinesin spindle protein (KSP) inhibitors);
(2) DNA damaging agents: for example, but not limited to, pyrrolobenzodiazepines (e.g., pyrrolo[2,1-c][1,4]benzodiazepine (PBD)), duocarmycins, indolinobenzodiazepine, duocarmycins, and calicheamicins;
(3) topoisomerase inhibitors: for example, but not limited to, camptothecins (e.g., exatecan and its derivative Dxd); and
(4) other cytotoxic agents: apoptosis inducers (Bcl-xL inhibitor), thailanstatin and analogs thereof, amatoxin, nicotinamide phosphoribosyltransferase (NAMPT) inhibitors, and carmaphycin.

The drug D in the formula (I) of the present invention may be any compound selected from the above. In some embodiments, the drug D is an anti-tumor growth inhibitor selected from maytansine derivatives, calicheamicin derivatives, and auristatin derivatives. In one embodiment, the drug D is a tubulin inhibitor/stability disruptor, such as vinca alkaloids, vincristine, paclitaxel, and docetaxel. In one embodiment, the drug D is a DNA synthesis inhibitor, such as methotrexate, 5-fluorouracil, cytarabine, gemcitabine, mercaptopurine, pentostatin, fludarabine, and cladribine. In one embodiment, the drug D is a DNA topoisomerase inhibitor, such as a topoisomerase I inhibitor (e.g., camptothecins) and a topoisomerase II inhibitor (e.g., actinomycin D, adriamycin, mitoxantrone).

In some preferred embodiments, the drug D of the ADC according to the present invention is a topoisomerase I inhibitor. Atypical example of the topoisomerase I inhibitor is a camptothecin drug, for example, but not limited to, camptothecin (CPT), hydroxycamptothecin, 9-aminocamptothecin, exatecan, topotecan, belotecan, irinotecan, SN-38, and FL118, and derivatives thereof. See, e.g., Vesela Kostova et al, The Chemistry Behind ADCs, Pharmaceuticals 2021, 14, 442. https://doi.org/10.3390/ph14050442; and WO2019/195665, which are incorporated herein by reference.

In some embodiments of the present invention, the drug D of the ADC of the present invention is a camptothecin drug selected from:
- camptothecin and derivatives thereof, e.g., 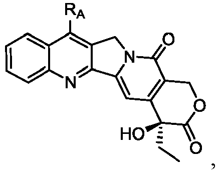 wherein R_{A} is selected from, for example, hydrogen and alkyl optionally substituted with a substituent, wherein the substituent includes, but is not limited to, hydroxy and amino, wherein the amino moiety or the hydroxy moiety may be unsubstituted or substituted with, for example, alkyl, alkylacyl, or alkylsulfonyl; in one embodiment, the drug D is 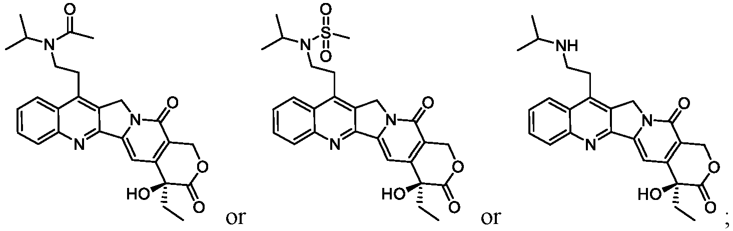
- 10,11-methylenedioxy CPT (also abbreviated as MDCPT or FL118) and derivatives thereof, e.g., 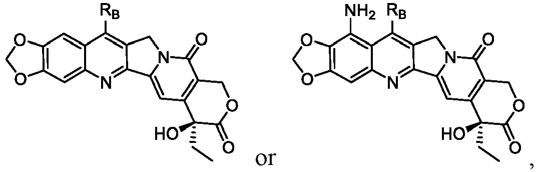 wherein R_{B} is selected from, for example, hydrogen, cycloalkyl, phenyl, and alkyl optionally substituted with a substituent, wherein the substituent includes, but is not limited to, halogen, hydroxy, optionally substituted alkoxy, cycloalkyl, heterocycloalkyl, phenyl, and amino, wherein the amino moiety may be optionally substituted; in one embodiment, the drug D is 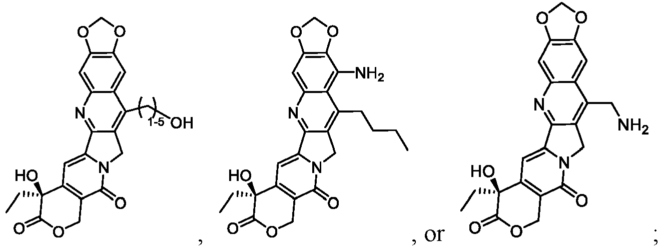
- 10-hydroxy CPT (also abbreviated as HCPT) and derivatives thereof, e.g., 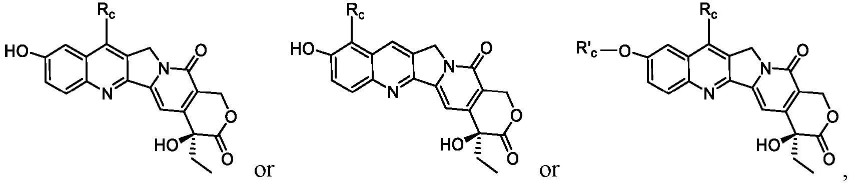 wherein Rc is selected from, for example, optionally substituted alkyl and cycloalkyl; R'c is selected from, for example, H, alkylacyl, and optionally substituted heterocycloalkylacyl; in one embodiment, the drug D is 7-ethyl-10-hydroxy CPT (SN-38) or its prodrug irinotecan (CPT-11), or the drug D is topotecan, 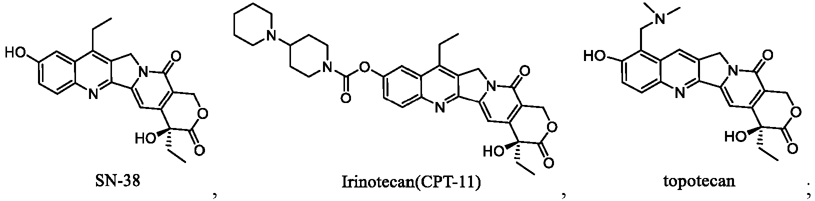 and
- exatecan and derivatives thereof, e.g., 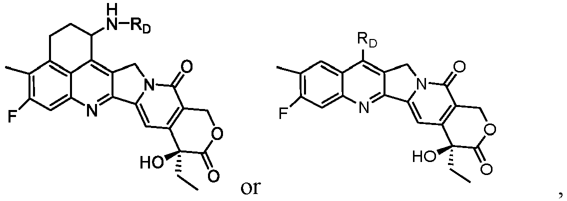 wherein R_{D} is selected from, for example, H, alkyl optionally substituted with a substituent, and alkyl-C(=O)- optionally substituted with a substituent, the substituent being, for example, -OH or substituted or unsubstituted amino; in one embodiment, the drug D is: 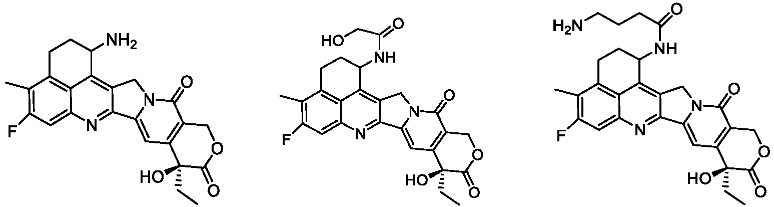 exatecan , Dxd(1) , or Dxd (2); and in another embodiment, the drug D is:

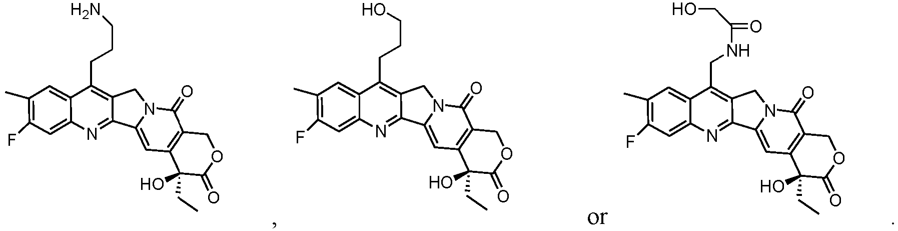

F118 ((20S)-10,11-methylenedioxy camptothecin) was obtained by high-throughput screening of compound libraries. FL118 has been demonstrated to have significantly higher *in vivo* and *in vitro* anti-cancer activity in many different cancer types as compared to other camptothecin derivatives. In addition to inhibiting topoisomerase I, FL118 can also selectively inhibit the gene promoter activity and endogenous expression of anti-apoptotic proteins such as survivin, XIAP, cIAP2, and Mcl-1. See Xiang Ling et al, A Novel Small Molecule FLU 8 That Selectively Inhibits Survivin, Mcl-1, XIAP and cIAP2 in a p53-Independent Manner, Shows Superior Antitumor Activity, PLoS ONE 7(9): e45571. doi:10.1371/journal.pone.0045571. Although the use of FL118 alone is severely hampered by its extremely poor water solubility and toxic side effects, as shown in the examples, the conjugate formed by combining the compound with the antibody and linker of the present invention not only effectively exerts the tumor killing effect of the compound, but also overcomes the drawbacks of the compound, and the formed conjugate has a low aggregation degree and good tolerance in animals.

Thus, in some preferred embodiments of the present invention, the drug D of the ADC of the present invention is an FL118 derivative, particularly position 7-substituted FL118.

In some embodiments, the drug D in formula (I) of the present invention is a camptothecin drug comprising the structure of formula D below: 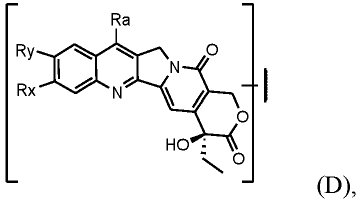 wherein,
Rₓ and R_{y} are each independently selected from H, halogen, -OH, and C₁-C₆ alkyl; or Rₓ and R_{y}, together with the carbon atom to which they are connected, form a 5- to 6-membered heterocyclic ring having 1 or 2 atoms selected from N, S, and O;
Rₐ is selected from H, halogen, -OH, optionally substituted C₁-C₈ alkyl or C₃-C₈ alkynyl or C₃-C₈ alkenyl, optionally substituted C₁-C₈ alkoxy, optionally substituted C₃-C₈ cycloalkyl, optionally substituted phenyl, optionally substituted heterocycloalkyl, and optionally substituted heteroaryl,
wherein preferably, Rₐ is selected from:
   hydrogen;
   C₃-C₈ cycloalkyl;
   phenyl; and
   C₁-C₈ alkyl or C₃-C₈ alkynyl or C₃-C₈ alkenyl optionally substituted with a substituent selected from: halogen, hydroxy, C₁-C₄ alkoxy optionally substituted with NH₂, NH(C₁-C₄ alkyl) and N(C₁-C₄ alkyl)₂, C₃-C₈ cycloalkyl, heterocycloalkyl, phenyl, and NR¹R²,
      wherein R¹ and R² are each independently selected from:
         hydrogen;
         C₁-C₈ alkyl optionally substituted with a substituent selected from: hydroxy, amino, amino substituted with one or two C₁-C₄ alkyl, amino substituted with one or two C₁-C₄ hydroxyalkyl, and amino substituted with (C₁-C₄ hydroxyalkyl) and (C₁-C₄ alkyl);
         C₁-C₄ alkyl substituted with 1 or 2 C₃-C₁₀ cycloalkyl, C₃-C₁₀ heterocycloalkyl, phenyl or heteroaryl;
         C₃-C₁₀ cycloalkyl;
         C₃-C₁₀ heterocycloalkyl;
         C₂-C₆ heteroalkyl;
         heteroaryl;
         optionally halogenated phenyl; and
         C₁-C₈ alkyl-C(=O)- optionally substituted with hydroxy or amino;
      or, R¹ and R², in combination with the nitrogen atom to which they are connected, form a 5-, 6-, or 7-membered heterocyclic ring having 0-3 substituents selected from halogen, C₁-C₄ alkyl, OH, C₁-C₄ alkoxy, NH₂, NH(C₁-C₄ alkyl), and N(C₁-C₄ alkyl)₂,
wherein cycloalkyl, heterocycloalkyl, phenyl, heteroaryl are each independently optionally substituted with 0-3 substituents selected from OH, C₁-C₄ alkyl, C₁-C₄ alkoxy, NH₂, NH(C₁-C₄ alkyl), and N(C₁-C₄ alkyl)₂ at each occurrence.

In some preferred embodiments, Rₓ and R_{y} are each independently H. In other preferred embodiments, Rₓ is F, and R_{y} is methyl. In still other preferred embodiments, Rₓ and R_{y}, together with the carbon atom to which they are connected, form a 5-membered heterocyclic ring containing two O atoms.

In some embodiments, the drug D in formula (I) of the present invention is a camptothecin drug comprising the structure of formula Dₐ or formula D_{b} below: 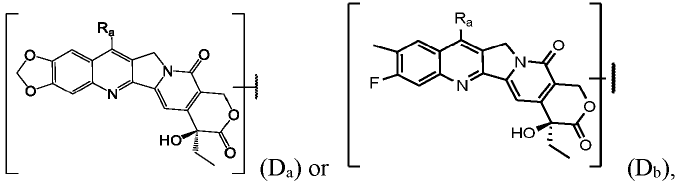 particularly a camptothecin drug of (Dₐ),
wherein Rₐ is selected from:
hydrogen;
C₃-C₈ cycloalkyl;
phenyl; and
C₁-C₈ alkyl, C₃-C₈ alkynyl or C₃-C₈ alkenyl optionally substituted with a substituent selected from: halogen, hydroxy, C₁-C₄ alkoxy optionally substituted with NH₂, NH(C₁-C₄ alkyl) and N(C₁-C₄ alkyl)₂, C₃-C₈ cycloalkyl, heterocycloalkyl, phenyl, and NR¹R²,
   wherein R¹ and R² are each independently selected from:
      hydrogen;
      C₁-C₈ alkyl optionally substituted with a substituent selected from: hydroxy, amino, amino substituted with one or two C₁-C₄ alkyl, amino substituted with one or two C₁-C₄ hydroxyalkyl, and amino substituted with (C₁-C₄ hydroxyalkyl) and (C₁-C₄ alkyl);
      C₁-C₄ alkyl substituted with 1 or 2 C₃-C₁₀ cycloalkyl, C₃-C₁₀ heterocycloalkyl, phenyl or heteroaryl;
      C₃-C₁₀ cycloalkyl;
      C₃-C₁₀ heterocycloalkyl;
      C₂-C₆ heteroalkyl;
      heteroaryl;
      optionally halogenated phenyl; and
      C₁-C₈ alkyl-C(=O)- optionally substituted with hydroxy or amino;
   or, R¹ and R², in combination with the nitrogen atom to which they are connected, form a 5-, 6-, or 7-membered heterocyclic ring having 0-3 substituents selected from halogen, C₁-C₄ alkyl, OH, C₁-C₄ alkoxy, NH₂, NH(C₁-C₄ alkyl), and N(C₁-C₄ alkyl)₂,
   wherein cycloalkyl, heterocycloalkyl, phenyl, heteroaryl are each independently optionally substituted with 0-3 substituents selected from OH, C₁-C₄ alkyl, C₁-C₄ alkoxy, NH₂, NH(C₁-C₄ alkyl), and N(C₁-C₄ alkyl)₂ at each occurrence.

In some embodiments, Rₐ is hydrogen.

In some embodiments, Rₐ is C₃-C₈ cycloalkyl (e.g., cyclopropyl, cycloheptyl, and cyclopentyl).

In some embodiments, Rₐ is phenyl. In other embodiments, Rₐ is phenyl substituted with NH₂ or NH(C₁-C₄ alkyl).

In some embodiments, Rₐ is C₁-C₆ alkyl, e.g., methyl, ethyl, propyl, 2-methylpropyl, butyl, isobutyl, 2,2-dimethyl-propyl, 2,2-dimethyl-butyl, n-hexyl, n-pentyl, and 3-ethyl-pentyl.

In some embodiments, Rₐ is C₁-C₄ alkyl optionally substituted with hydroxy or C₁-C₄ alkoxy, wherein the alkoxy is optionally further substituted with -NH₂, -NH(C₁-C₄ alkyl), and -N(C₁-C₄ alkyl)₂.

In some embodiments, Rₐ is C₂-C₄ alkenyl or C₂-C₄ alkynyl optionally substituted with hydroxy or amino.

In some embodiments, Rₐ is C₁-C₄ alkyl substituted with 5- to 6-membered heterocycloalkyl having 1 or 2 atoms selected from N, S, and O, wherein the heterocycloalkyl moiety is optionally further substituted with C₁-C₄ alkyl, and preferably, the heterocycloalkyl is piperazinyl or morpholinyl.

In some embodiments, Rₐ is C₁-C₄ alkyl substituted with -NR¹R², particularly -methylene-NR¹R², wherein R¹ and R² are each independently selected from:
hydrogen;
-C₁-C₄ alkyl;
-C₁-C₄ alkyl substituted with a substituent selected from:
   -OH, -NH₂, -NH(C₁-C₄ alkyl), -N(C₁-C₄ alkyl)₂, -phenyl, -phenylene-NH₂,-phenylene-C₁-C₄ alkoxy, -diphenyl, -C₃-C₁₀ cycloalkyl, -C₃-C₁₀ cycloalkylene-C₁-C₄ alkyl, and -C₃-C₁₀ heterocycloalkyl, wherein the heterocycloalkyl has 1 or 2 heteroatoms selected from N, S, and O, and preferably, the heterocycloalkyl is piperidinyl;
phenyl; halogenated phenyl;
C₃-C₁₀ cycloalkyl;
-C(=O)-C₁-C₄ alkyl-OH; and -C(=O)-C₁-C₄ alkyl-NH₂.

In some embodiments, Rₐ is C₁-C₄ alkyl substituted with -NR¹R², particularly -methylene-NR¹R², wherein R¹ and R², in combination with the nitrogen atom to which they are connected, form a 5-, 6-, or 7-membered heterocycloalkyl ring. In some aspects, R¹ and R², in combination with the nitrogen atom to which they are connected, form a 6-membered ring. In some aspects, the 6-membered ring is morpholinyl or piperazinyl.

In some preferred embodiments, Rₐ is selected from: -C₁-C₄ alkylene-OH, -C₁-C₄ alkylene-O-C₁-C₄ alkylene-NH₂, -C₁-C₄ alkylene-NH₂, -C₁-C₄ alkylene-NH(C₁-C₄ hydroxyalkyl), -C₁-C₄ alkylene-N(C₁-C₄ hydroxyalkyl)₂, -C₁-C₄ alkylene-N(C₁-C₄ aminoalkyl)(C₁-C₄ hydroxyalkyl), -C₁-C₄ alkylene-NH(C₁-C₄ aminoalkyl), -C₁-C₄ alkylene-NH-C(=O)-C₁-C₄ alkylene-OH; -C₁-C₄ alkylene-NH-C(=O)-C₁-C₄ alkylene-NH₂, -C₁-C₄ alkylene-N(C₁-C₄ alkyl)(C(=O)-C₁-C₄ alkylene-OH); and -C₁-C₄ alkylene-N(C₁-C₄ alkyl)(C(=O)-C₁-C₄ alkylene-NH₂).

In some more preferred embodiments, Rₐ is -C₁-C₄ alkylene-NH-C(=O)-C₁-C₄ alkylene-OH; or -C₁-C₄ alkylene-NH-C(=O)-C₁-C₄ alkylene-NH₂.

In some more preferred embodiments, Rₐ is -C₂-C₄ alkenylene-NH₂ or -C₂-C₄ alkenylene-OH, e.g., -CH=CH-CH₂-NH₂ or -CH=CH-CH₂-OH.

In some more preferred embodiments, Rₐ is -C₁-C₄ alkylene-OH, -C₁-C₄ alkylene-O-C₁-C₄ alkylene-NH₂, or -C₁-C₄ alkylene-NH₂.

In some more preferred embodiments, Rₐ is -C₁-C₄ alkylene-OH. In some aspects, Rₐ is hydroxymethyl. In some aspects, Rₐ is hydroxyethyl. In some aspects, Rₐ is hydroxypropyl.

In other more preferred embodiments, Rₐ is -C₁-C₄ alkylene-NH₂. In some aspects, Rₐ is aminomethyl. In some aspects, Rₐ is aminoethyl. In some aspects, Rₐ is aminopropyl.

Preferably, the drug D is linked to the linker L via a free hydroxy or amino group thereon for conjugation to the antibody. More preferably, the drug D of formula D or formula Dₐ or D_{b} is linked to the linker L via a hydroxy or amino group on Rₐ for conjugation to the antibody. Preferably, after being linked to the linker, Rₐ has a structure selected from: 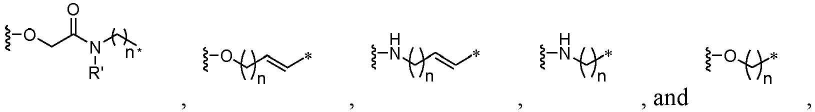 wherein n is an integer of 1-4, preferably n = 2 or 3; R' is H or C₁-C₄ alkyl, preferably H; the left wavy line indicates the position of linkage to the linker L, and the right asterisk indicates the position of linkage to the camptothecin nucleus;
more preferably, after being linked to the linker, Rₐ has a structure selected from: 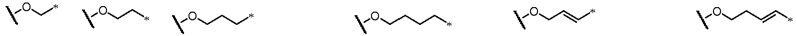 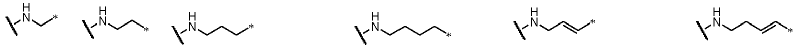 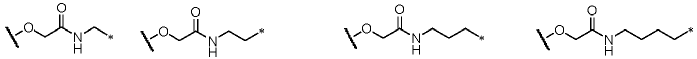 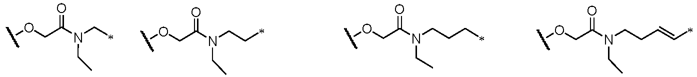 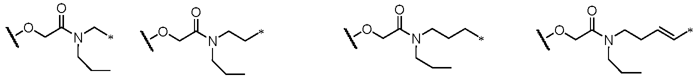 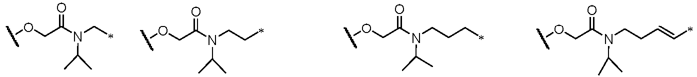
and most preferably, after being linked to the linker, Rₐ has a structure selected from: 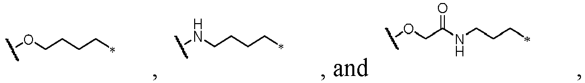
wherein the left wavy line indicates the position of linkage to the linker L, and the right asterisk indicates the position of linkage to the camptothecin nucleus.

In some preferred embodiments, the L-D unit in formula (I) of the present invention comprises the following structure: 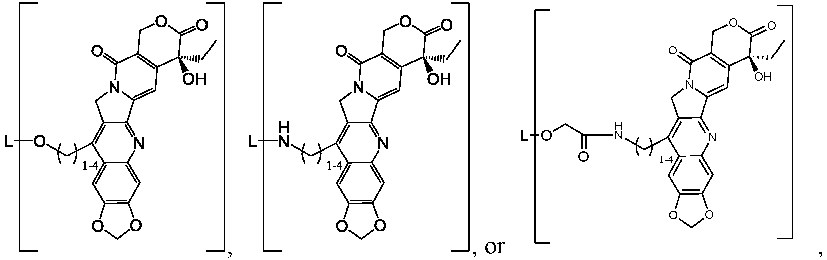 and more preferably, the L-D unit in formula (I) comprises the following structure:

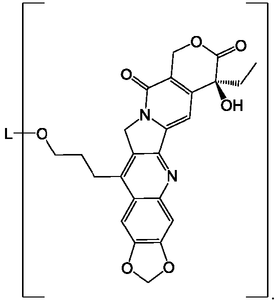

### Linker L unit

A linker L applicable to the present invention may be any linker capable of achieving conjugation of the antibody of the present invention to a drug. Suitably, the addition of the linker should ensure sufficient stability of the ADC of the present invention in the circulatory system, while providing a rapid and effective release of active forms of the toxic drug at target sites (e.g., tumor cells or tumor environment).

In some embodiments, the linker in formula (I) of the present invention is a non-degradable linker. Examples of non-degradable linkers include, but are not limited to, *N*-succinimidyl-4-(*N-*maleimidomethyl)cyclohexane-1-carboxylate (SMCC). Typically, ADCs comprising such linkers must be internalized by the cells, and antibody moieties of the ADCs are degraded by intracellular lysosomal proteases, releasing active molecules of the drug.

In still other embodiments, the linker in formula (I) of the present invention is a degradable linker. Drug release from ADCs comprising such linkers is triggered by the property of cleavage sites in the linkers. Thus, the cleavage sites of such linkers can be designed based on the characteristics of the target treatment sites (e.g., tumor cell lysosomes and/or tumor environment). In most cases, the degradable linker may consist of a conjugation moiety, a degradable moiety, and optionally a spacer group moiety. The conjugation moiety is responsible for linking of the antibody to the linker-drug, and may be selected based on the desired antibody conjugation chemistry to be employed. Under an enzyme-based release mechanism, the degradable moiety will comprise peptides or peptide analogs that can be recognized by enzymes, e.g., oligopeptides or oligopeptide analogs, such as Val-Ala, Val-Cit, Phe-Lys, Gly-Phe-Leu-Gly, Ala-Leu-Ala-Leu, Gly-Gly-Phe-Gly, cyclobutyl-Ala, and cyclobutyl-Cit, which can be degraded by proteinases. In some cases, the properties of the ADC, e.g., stability of the ADC in blood circulation and/or efficacy of the ADC at a target site, may be improved by introducing modifications at peptide residue positions adjacent to the enzyme cleavage site in the linker. In some cases, if desired, a spacer group may also be introduced between the degradable moiety of the linker and the drug D so as to facilitate the release (particularly traceless release) of the active molecules of the drug from the rest of the conjugate, for example, a para-aminobenzyl carbamate (PABA) or aminomethyl (-NHCH₂-) spacer group which can be spontaneously eliminated in acidic media may be introduced. In addition, when the drug is highly hydrophobic, in some cases, it may be contemplated (but not necessary) to add, for example, a PEG unit to improve properties of the ADC, for example, to reduce precipitation and aggregation. Linkers applicable to the present invention include, but are not limited to, those disclosed in WO2022/170971 (which is hereby incorporated by reference).

In some embodiments, the linker L in formula (I) of the present invention has a structure of formula (II) below:

-Z-Y-M- (II),

wherein,
Z is a linking group linked to Ab,
Y is a peptide of 2-5 amino acids, preferably dipeptide, tripeptide or tetrapeptide, and
M is absent or is a spacer group for linking to the drug D.

### Z linking group

Typically, a sulfonyl group of cysteine of an antibody is present in the form of a disulfide bond. The disulfide bond of the antibody is opened, providing a free sulfhydryl group as a conjugation site. One approach to form an ADC by conjugation to the sulfhydryl group of the antibody is to subject the free sulfhydryl group on the antibody to a Michael addition reaction with a heterocyclic (e.g., maleimide) linker. Another approach is to subject a linker comprising a leaving group-substituted heteroaromatic ring to a nucleophilic substitution reaction with the free sulfhydryl group in the antibody molecule to obtain an antibody-drug conjugate. Both approaches are applicable to the ADC of the present invention. Thus, the linker according to the present invention may in some aspects comprise a heterocyclic (e.g., maleimide) or heteroaromatic (e.g., pyrimidine) Z linking group, and in some cases, preferably comprise a heteroaromatic (e.g., pyrimidine) linking group, so as to provide a conjugate with greater stability in blood circulation.

In some embodiments, Z in formula (II) has the following structure:

-Z₁-Z₂-Z₃-Z₄-,

wherein Z₁ is a sulfur atom in Ab;
Z₂ is a 5- to 10-membered heterocyclyl, preferably containing 1 or 2 heteroatoms selected from N, S, and O;
Z₃ is selected from: a bond, -C(=O)-, -C₁-C₁₀ alkylene-C(=O)-, -C₃-C₁₀ alkynylene-C(=O)-, -C₃-C₁₀ alkenylene-C(=O)-, -C₁-C₁₀ heteroalkylene-C(=O)-, -C₃-C₈ cycloalkylene-C(=O)-, -O-C₁-C₈ alkylene-C(=O)-, -arylene-C(=O)-, -C₁-C₁₀ alkylene-arylene-C(=O)-, -arylene-C₁-C₁₀ alkylene-C(=O)-, -C₁-C₁₀ alkylene-C₃-C₈ cycloalkylene-C(=O)-, -C₃-C₈ cycloalkylene-C₁-C₁₀ alkylene-C(=O)-, -C₃-C₈ heterocyclylene-C(=O)-, -C₁-C₁₀ alkylene-C₃-C₈ heterocyclylene-C(=O)-, -C₃-C₈ heterocyclylene-C₁-C₁₀ alkylene-C(=O)-, -heteroarylene-C(=O)-, -C₁-C₁₀ alkylene-heteroarylene-C(=O)-, and -heteroarylene-C₁-C₁₀ alkylene-C(=O)-; and
Z₄ is a bond or a PEG unit represented by the following formula: 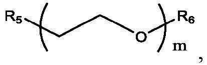 wherein R₅ is selected from C₁₋₄ alkylene, -NH-, and -NH-C₁₋₄ alkylene-heteroaryl-, wherein the heteroaryl is 5- or 6-membered nitrogen-containing heteroaryl, preferably triazolyl; and R₆ is -C(=O)-, -C₁₋₄ alkylene, -C₁₋₄ alkylene-C(=O)-, -NH-C(=O)-(CH₂OCH₂)-C(=O)-, and -C₁₋₄ alkylene-NH-C(=O)-(CH₂OCH₂)-C(=O)-, wherein m is an integer of 2-12, for example, m = 2, 4, 6, or 8.

In one embodiment, Z₂ is 5- to 10-membered heteroaryl. In one embodiment, Z₂ is selected from pyrimidine, thiazole, benzothiazole, oxazole, quinazoline, and pyrrolo [2,3d] pyrimidine optionally substituted with one or more substituents independently selected from hydrogen, halogen, nitro, C₁₋₆ alkyl, and halogenated C₁₋₆ alkyl. In some embodiments, Z₂ is a heteroaryl group selected from: 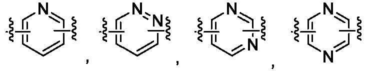 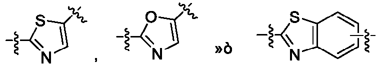 wherein Z₂ is linked to Z₁ via a carbon atom adjacent to the heteroatom. In a preferred embodiment, Z₂ is pyrimidinylene, preferably 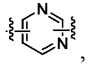 and more preferably 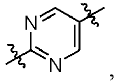 wherein the left wavy line indicates the position of linkage to Z₁, and the right wavy line indicates the position of linkage to Z₃.

In some embodiments, Z₂ is maleimido, 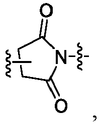 wherein the left wavy line indicates the position of linkage to Z₁, and the right wavy line indicates the position of linkage to Z₃.

In some embodiments, Z₃ is -C₃-C₈ heterocyclylene-C(=O)-, -C₁-C₁₀ alkylene-C₃-C₈ heterocyclylene-C(=O)-, and -C₃-C₈ heterocyclylene-C₁-C₁₀ alkylene-C(=O)-, preferably, wherein the heterocycle in the heterocyclylene is a heteroaromatic ring, e.g., a heteroaromatic ring of triazole, pyrazole, thiazole, oxazole, isoxazole, or pyridazine. In some embodiments, Z₃ is-heteroarylene-C(=O)-, -C₁-C₁₀ alkylene-heteroarylene-C(=O)-, or -heteroarylene-C₁-C₁₀ alkylene-C(=O)-.

In some embodiments, Z₃ is -heteroarylene-C₁-C₁₀ alkylene-C(=O)-, particularly, 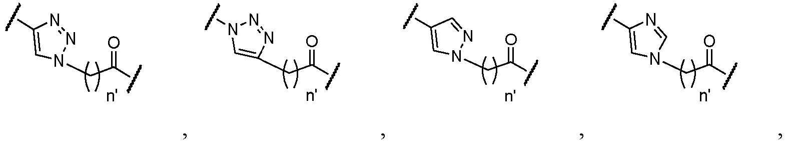 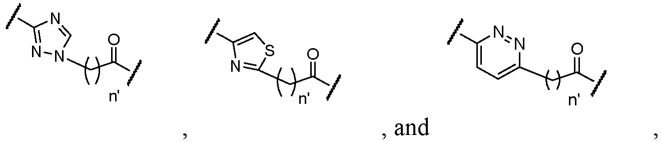 wherein n' is 1-6, preferably, Z₃ is 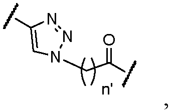 and more preferably 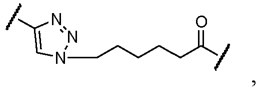 wherein the left wavy line indicates the position of linkage to Z₂, and the right wavy line indicates the position of linkage to Z₄, and preferably, Z₂ is pyrimidinylene.

In some embodiments, Z₃ is arylene- or heteroarylene-C(=O)-, particularly, 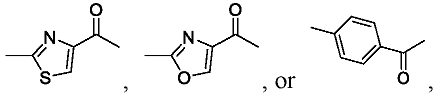 wherein Z₃ is linked to Z₄ via -C-(=O)-, and preferably, Z₂ is pyrimidinylene.

In other embodiments, Z₃ is -(C≡C)-C₁₋₅ alkylene-C(=O)-, -(CH=CH)-C₁₋₅ alkylene-C(=O)-,-C₁₋₆ alkylene-C(=O)-, or -C₃₋₈ cycloalkylene-C(=O)-, wherein Z₃ is linked to Z₄ via -C-(=O)-.

In some preferred embodiments, Z₂ is pyrimidinylene, and Z₃ is -C(=O)-.

In some preferred embodiments, Z₂ is pyrimidinylene, and Z₃ is -(C≡C)-C₁₋₅ alkylene-C(=O)- or -(CH=CH)-C₁₋₅ alkylene-C(=O)-, particularly -(C≡C)-C₁₋₅ alkylene-C(=O)-.

In some preferred embodiments, Z₂ is maleimido, and Z₃ is -C₁₋₆ alkylene-C(=O)- or -C₃₋₈ cycloalkylene-C(=O)-.

In some embodiments, Z₄ is a bond, and Z₃ is directly linked to Y in formula (II).

In some embodiments, Z₄ is a unit comprising 2-12 PEGs. In some embodiments, Z₄ is 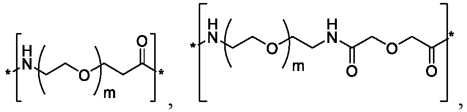 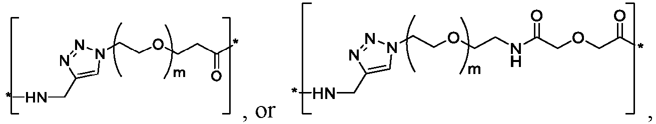 wherein m = 1-8, e.g., 2, 3, 4, 5, 6,7, or 8.

In some embodiments, Z₄ is selected from: 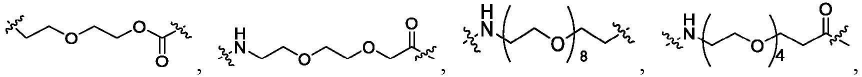 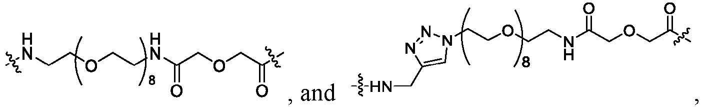 wherein the left wavy line indicates the position of linkage to Z₃, and the right wavy line indicates the position of linkage to Y in formula II.

In some embodiments, Z in formula (I) of the present invention has the following structure: 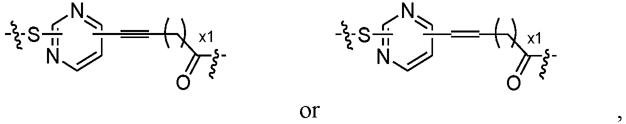 wherein x₁= 1-8, e.g., x₁ = 3.

In some embodiments, Z in formula (I) of the present invention has the following structure: 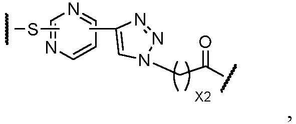 wherein x₂ = 1-6.

In some preferred embodiments, Z in formula (I) of the present invention has the following structure: 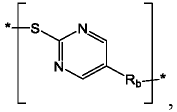
wherein S is a sulfur atom in Ab, and R_{b} is unsubstituted or substituted -C₃₋₁₀ alkynyl-C(=O)-or -C₃₋₁₀ alkenyl-C(=O)-, or -heteroarylene-C₁-C₁₀ alkylene-C(=O)-,
   preferably, R_{b} is: 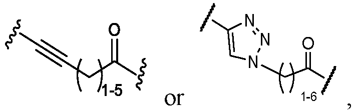
wherein the left wavy line indicates the position of linkage to pyrimidinyl, and the right wavy line indicates the position of linkage to Y;
and more preferably, Z has the following structure:

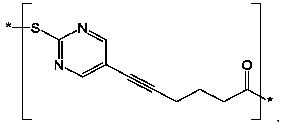

In some embodiments, Z in formula (I) of the present invention has the following structure: 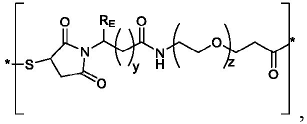 wherein R_{E} is hydrogen, C₁₋₆ alkyl, C₁₋₆ aminoalkyl, C₁₋₆ haloalkyl, C₁₋₆ hydroxyalkyl; y = 0-4, e.g., 0, 2, or 5; and the alkyl, amino and hydroxy moieties are optionally substituted; and in some embodiments, R_{E} is optionally substituted aminoalkyl, e.g., substituted with -C₁₋₄ alkylene-NH₂, -C₁₋₄ alkylene NHR_{F}, and -C₁₋₄ alkylene N(R_{F})₂, wherein each R_{F} is independently selected from C₁₋₆ alkyl and C₁₋₆ haloalkyl; or the two R_{F} groups, in combination with the nitrogen to which they are attached, form an azetidinyl, pyrrolidinyl, or piperidinyl group.

In some embodiments, Z in formula (I) of the present invention has the following structure:

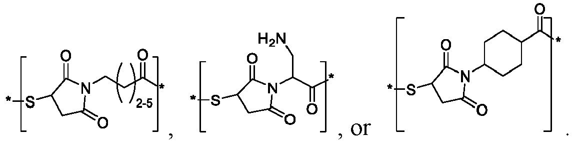

### Peptide-containing Y unit

In some aspects, for the purpose of limiting or minimizing the non-targeted toxicity of ADCs while ensuring the release of toxin molecules at the target tumor site, it is advantageous to design antibody-drug conjugates so that they can be degraded by proteinases in tumor cells or in tumor environment (especially enzymes with significantly higher activity in the tumor cells and/or in the tumor environment relative to blood) while targeting the tumor cells with high selectivity. For this purpose, oligopeptides or oligopeptide analogs that can be recognized and cleaved by such proteinases can be contained in linkers of such ADCs.

Thus, in one embodiment, Y unit that is part of a linker of the present invention is a peptide-containing degradable moiety such as a degradable peptide linker containing two or more (e.g., 2-12, such as 2, 3, 4, 5, or 6) contiguous or non-contiguous amino acids. Each amino acid in the peptide-containing unit may be independently selected from natural amino acids and non-natural amino acids, for example, selected from: alanine, arginine, aspartic acid, asparagine, histidine, glycine, glutamic acid, glutamine, phenylalanine, lysine, substituted lysine, leucine, serine, tyrosine, threonine, isoleucine, proline, tryptophan, valine, cysteine, methionine, selenocysteine, ornithine, β-alanine, citrulline and derivatives thereof. In some embodiments, each amino acid is independently selected from alanine, arginine, aspartic acid, asparagine, histidine, glycine, glutamic acid, glutamine, phenylalanine, lysine, leucine, serine, tyrosine, threonine, isoleucine, proline, tryptophan, valine, cysteine, methionine and derivatives thereof. In some embodiments, each amino acid is independently selected from alanine, arginine, aspartic acid, asparagine, histidine, glycine, glutamic acid, glutamine, phenylalanine, lysine, leucine, serine, tyrosine, threonine, isoleucine, proline, tryptophan, valine, N-methylglycine, β-alanine and derivatives thereof. In some aspects, upon the internalization of the ADCs into the tumor cells, amide bonds in the Y unit will be recognized and degraded by enzymes in the lysosomes of the tumor cells to release drug moiety D. In other aspects, the amide bonds in the Y unit can be recognized and degraded by enzymes in the tumor environment to release the drug moiety D. Linkers with such peptide-containing Y units will facilitate the release of the toxin molecules in the tumor cells and in the tumor environment by designing antibody-drug conjugates with a tumor-biased distribution.

In some embodiments, Y is a unit including the following selected from: Val-Cit, Phe-Lys, Val-Ala, Val-Lys-Gly, Ala-Ala-Ala, Val-Ala, Gly-Phe-Leu-Gly, Ala-Leu-Ala-Leu, Gly-Gly-Phe-Gly, cyclobutyl-Ala, and cyclobutyl-Cit. In some embodiments, Y is a unit including the following selected from: Val, Cit, Phe, Lys, D-Val, Leu, Gly, Ala, Asn, Cit-Val, Val-Ala, Lys-Val, Val-Lys(Ac), Phe-Lys, Phe-Lys(Ac), D-Val-Leu-Lys, Gly-Gly-Arg, and Ala-Ala-Asn.

In some preferred embodiments, Y is a dipeptide, tripeptide, tetrapeptide, or pentapeptide containing a substituted lysine. In one embodiment, the substituted lysine is: 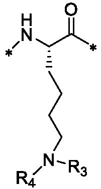 wherein R₃ and R₄ are each independently selected from: H, C₁₋₆ alkyl, -CO-NH₂,-CONH(C₁₋₆ alkyl) and -CONH(C₁₋₆ alkyl)₂, wherein the alkyl is optionally substituted with the following groups selected from: halogen, C₁₋₆ alkoxy, C₁₋₆ haloalkyl, C₃₋₆ cycloalkyl, 6-10 membered aryl and 5-14 membered heteroaryl.

In some preferred embodiments, Y is a peptide having an amino acid sequence of the following formula from N-terminus to C-terminus;

Xaa₁-Xaa₂-Xaa₃-Xaa₄-Xaa₅,

wherein Xaa₁ is absent or is an amino acid selected from valine, glycine, alanine, and glutamic acid;
   Xaa₂ is an amino acid selected from phenylalanine, leucine, alanine, and valine, preferably valine;
   Xaa₃ is unsubstituted or substituted lysine;
   Xaa₄ is an amino acid selected from leucine, glycine, and alanine;
   Xaa₅ is absent or is an amino acid selected from glycine and alanine;
wherein the N-terminus of the amino acid sequence is linked to Z unit of the linker and the C-terminus is linked to M unit or directly to the drug D,
preferably, Xaa₃ is lysine in which an ε-amino group is monosubstituted or disubstituted with C₁-C₃ alkyl,
and more preferably, Y is a peptide selected from: Phe-Lys-Gly, Leu-lys-Gly, Gly-Val-Lys-Gly, Val-Lys-Gly-Gly, Val-Lys-Gly, Val-Lys-Ala and Val-Lys-Leu, wherein the Lys residue is unsubstituted lysine or lysine monosubstituted or disubstituted with C₁-C₃ alkyl.

### M spacer group

In some embodiments, the ADCs of the present invention have a spacer group (M) between the releasable peptide-containing unit (Y) and the drug (D). The spacer group may be a functional group that facilitates the linkage of the peptide-containing unit (Y) to the drug D, or can provide an additional structural component to further facilitate the release of the drug D from the rest of the conjugate (e.g., a self-immolative group such as *p*-aminobenzyl (PAB) component).

In some embodiments, the M spacer group linking the Y unit to the drug D unit may be selected from: 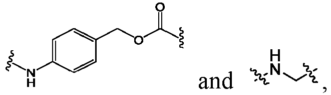 preferably 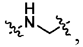 wherein the left wavy line indicates the linkage to Y and the right wavy line indicates the linkage to the drug D unit.

In other embodiments, M is a covalent bond, and Y is directly linked to the drug D in formula I, such as through an amide bond.

In a preferred embodiment, a Y-M unit in the linker L comprises the following structure: 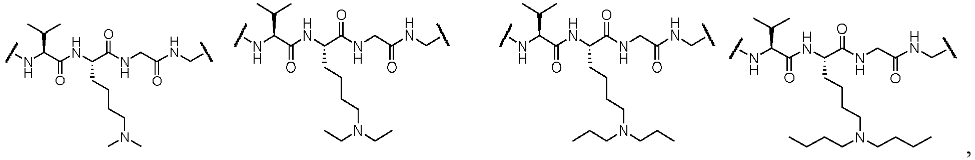 and preferably, the Y-M unit is linked to the following Z unit:

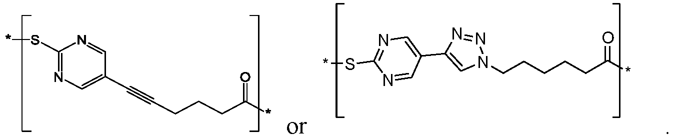

### Exemplary linker L

In some preferred embodiments, L in formula (I) of the present invention is a linker comprising the following structure: 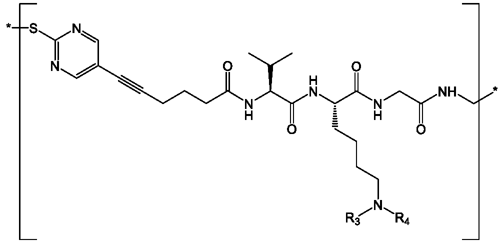 wherein R₃ and R₄ are each independently selected from methyl, ethyl, and propyl.

In some more preferred embodiments, R₃ and R₄ are both methyl; or R₃ and R₄ are both ethyl; or R₃ and R₄ are both propyl.

In some embodiments, the L-D unit in formula I of the present invention is linked to an antibody by forming a thioether bond with a sulfhydryl group of a free cysteine of a light chain and/or heavy chain of the Ab.

### Exemplary ADC

In some embodiments, the present invention provides an ADC having the following structure, or a pharmaceutical acceptable salt or solvate thereof: 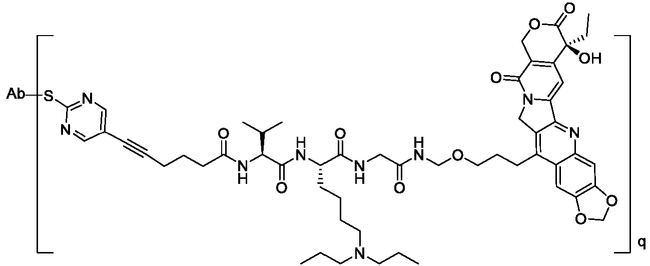 or 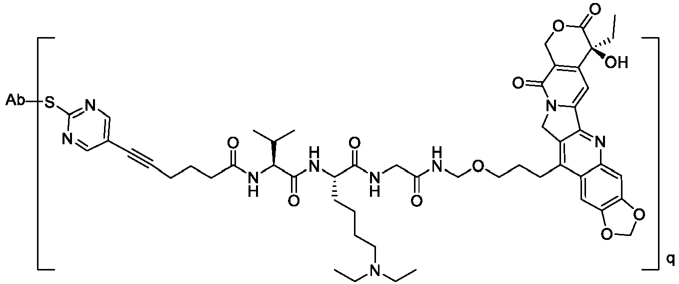 or 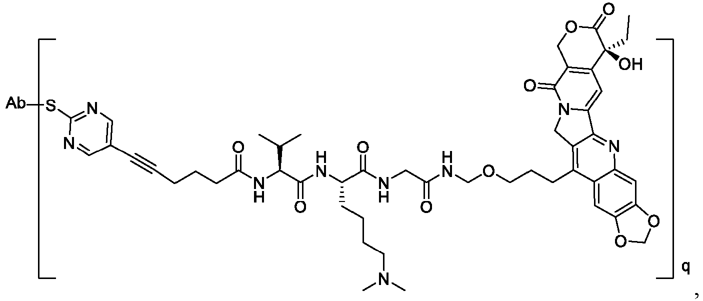 wherein q represents an average DAR value of 1-20, such as an average DAR value of about 2, 3, 4, 6, or 8.

In some embodiments, the ADC according to the present invention has at least one or more of the following advantages:
- significantly facilitating the distribution and enrichment of the ADC into the tumor environment relative to the distribution of the ADC in the blood circulation;
- effectively limiting the premature release of the payload in the plasma, and having a high blood circulation stability;
- providing the effective release of active forms of the toxic drug in the tumor environment;
- conferring an efficient *in vivo* tumor-killing effect in an animal; and
- good tolerance in the animal.

In still other embodiments, the ADC according to the present invention can further have at least one or more of the following advantages:
- the addition of toxin-linkers not inducing the aggregation, and being capable of achieving a high drug loading with an DAR value up to 8; and
- acceptable PK properties.

### II. Preparation of antibody-drug conjugates

The generation of antibody-drug conjugates can be accomplished by any technique known to those skilled in the art. In some aspects, drug-linkers are conjugated to antibodies by reacting with amino acid residues of the antibodies. In some embodiments, drug D is conjugated to a cysteine residue of an antibody using a heteroaryl linker L with a leaving group to prepare the conjugate of formula I of the present invention. In some embodiments, an interchain disulfide bond of the antibody can be disrupted to expose the free sulfhydryl group for the conjugation to a heteroaryl linker-drug by controlling the conditions under which the antibody is treated with a reducing agent, such as tris(2-carboxyethyl)phosphine (TCEP). For IgG1 antibodies, up to four linking disulfide bonds can be reduced, thereby generating up to 8 reactive sulfhydryl groups for conjugation. Conjugates prepared by this method may comprise zero, one, two, three, four, five, six, seven, or eight drugs in each antibody molecule.

When the prepared conjugates are conjugate compositions having different drug conjugation sites and/or numbers, the drug loading of the conjugates is represented by the average DAR, which is the average number of drug molecules per antibody. The average number of drugs per antibody for the produced antibody-drug conjugate compositions can be characterized by conventional means, such as mass spectrometry, ELISA assays and HPLC. In other embodiments, the quantitative distribution of the antibody-drug conjugates, denoted by q, can also be determined. The separation, purification and characterization of homogeneous antibody-drug conjugates, where q is a certain value, from antibody-drug conjugates with other drug loadings can be achieved by means such as reversed-phase HPLC or electrophoresis.

Thus, in one aspect of the present invention, q represents the number of drug-linker (L-D) moieties conjugated to one single antibody (Ab), and is preferably an integer from 1 to 16, 1 to 12, 1 to 10, or 1 to 8. In this case, individual ADCs can also be referred to as ADC compounds. In any embodiments of the present invention, there may be 1, 2, 3, 4, 5, 6, 7, 8, 9, 10, 11, 12, 13, 14, 15, or 16 drug-linker moieties conjugated to one single antibody on the ADC compounds according to the present invention.

In another aspect of the present invention, q represents the average DAR of the prepared conjugate compositions. In this case, q may be an integer or decimal ranging from 1 to about 16, from 1 to about 12, from 1 to about 10, or from 1 to about 8, from 2 to about 16, from 2 to about 12, from 2 to about 10, or from 2 to about 8. In some aspects, q represents an average DAR of about 3. In some aspects, q represents an average DAR of about 6. In some aspects, q represents an average DAR of about 8.

### III. Pharmaceutical composition

In some embodiments, the present invention provides a composition comprising any of the ADCs or the pharmaceutical acceptable salts or solvates thereof described herein, wherein, preferably, the composition is a pharmaceutical composition or a pharmaceutical formulation. In one embodiment, the composition further comprises a pharmaceutical auxiliary material. In one embodiment, the composition, e.g., the pharmaceutical composition, comprises the ADC or the pharmaceutical acceptable salt or solvate thereof of the present invention, and a combination of one or more additional therapeutic agents.

The present invention further comprises a composition (including a pharmaceutical composition) comprising the ADC or the pharmaceutical acceptable salt or solvate thereof of the present invention. Such compositions can further comprise a suitable pharmaceutical auxiliary material, such as a pharmaceutical carrier and a pharmaceutical excipient known in the art, including buffers.

As used herein, the "pharmaceutical carrier" includes any and all solvents, dispersion media, isotonic agents and absorption delaying agents, and the like that are physiologically compatible.

For use and application of the pharmaceutical auxiliary materials, see Handbook of Pharmaceutical Excipients, 8th Ed., R. C. Rowe, P. J. Seskey and S. C. Owen, Pharmaceutical Press, London, Chicago.

The compositions of the present invention may be in a variety of forms. Such forms include, for example, liquid, semi-solid and solid dosage forms, such as liquid solutions (e.g., injectable solutions and infusible solutions), pulvis or suspensions, liposomes, and suppositories. The preferred form depends on the intended mode of administration and therapeutic use.

The drug, preferably in the form of a lyophilized formulation or an aqueous solution, comprising the ADC described herein can be prepared by mixing the ADC or the pharmaceutical acceptable salt or solvate thereof of a desired purity of the present invention with one or more optional pharmaceutical auxiliary materials.

The pharmaceutical composition or formulation of the present invention can further comprise more than one active ingredient required by a specific indication treated, preferably those having complementarity activity without adversely affecting one another. For example, it may be desirable to also provide additional therapeutic agents, including chemotherapeutic agents, angiogenesis inhibitors, cytokines, cytotoxic agents, other antibodies, small molecule drugs, or immunomodulators (e.g., immune checkpoint inhibitors or agonists), and the like. The active ingredients are suitably combined in an amount effective for an intended purpose.

A sustained release formulation can be prepared. Suitable examples of the sustained release formulation include a semi-permeable matrix of a solid hydrophobic polymer containing an antibody. The matrix is in the form of a shaped article, such as a film or a microcapsule.

### IV. Pharmaceutical combination and kit

In some embodiments, the present invention further provides a pharmaceutical combination or a pharmaceutical combination product comprising the or the pharmaceutical acceptable salt or solvate thereof of the present invention, and one or more additional therapeutic agents.

Another object of the present invention is to provide a kit of parts comprising the pharmaceutical combination of the present invention; preferably, the kit is in the form of a pharmaceutical dose unit. Dose units may thus be provided according to the dosing regimen or the interval between drug administrations.

In one embodiment, the kit of parts of the present invention comprises the following in the same package:
- a first container containing a pharmaceutical composition comprising the ADC or the pharmaceutical acceptable salt or solvate thereof of the present invention;
- a second container containing a pharmaceutical composition comprising additional therapeutic agents.

The mode and order of administration of the components of the combination product can be determined by those skilled in the art based on factors such as the disease to be treated, the individual condition and the like. The combination product of the present invention can be used in a therapeutic method of the present invention. In some embodiments, the present invention provides a combination product, wherein the additional therapeutic agents refer to, for example, a therapeutic agent, such as an antibody, which is effective to stimulate an immune response and thus further enhance, stimulate or upregulate the immune response in a subject. In some embodiments, the combination product is used for preventing or treating TF-positive tumors.

### Anti-TF antibody of the present invention

By screening for antibody sequences with sequence uniqueness and high antigen-binding specificity; and further by evaluating the properties of the obtained antibodies, including affinity, endocytic activity, signaling pathway blocking, coagulation effects, stability, pharmacokinetics, etc., the present inventors identified the humanized anti-TF monoclonal antibodies with excellent properties according to the present invention. The antibody of the present invention not only exhibits high binding affinity and high specificity to TF-positive tumor cells, thereby being able to be rapidly and effectively endocytosed by the tumor cells, but also has little effect on TF-mediated coagulation. Thus, the antibody is not only suitable for being used alone or in combination with other anti-cancer drugs for cancer treatment, but also suitable for being used as a molecular component for forming a novel anti-cancer molecule targeting cancer tissues.

Thus, in some aspects, the present invention further provides a humanized anti-TF monoclonal antibody with various excellent properties, a nucleic acid encoding the antibody, a vector and a host cell comprising the nucleic acid, as well as an immunoconjugate comprising the antibody, a multispecific antibody, a pharmaceutical composition and use.

### I. Antibody of the present invention and properties thereof

The anti-TF antibody of the present invention and properties thereof are described in detail below. As will be understood by those skilled in the art, any of the antibody technical features (including structural and property features) and any combination thereof described in this section are also equally applicable to the antibody of the present invention as the Ab unit of the ADC of the present invention, unless otherwise expressly stated to the contrary.

### Anti-TF antibody of the present invention

The present invention provides an antibody or an antigen-binding fragment thereof, particularly a humanized antibody or an antigen-binding fragment thereof, that specifically binds to TF, preferably a human TF protein.

In some embodiments, the present invention provides an anti-TF antibody or an antigen-binding fragment thereof, which comprises:
(i) HCDR1, 2 and 3 sequences of a heavy chain variable region set forth in SEQ ID NO: 1, and LCDR1, 2 and 3 sequences of a light chain variable region set forth in SEQ ID NO: 2; or
(ii) HCDR1, 2 and 3 sequences of a heavy chain variable region set forth in SEQ ID NO: 3, and LCDR1, 2 and 3 sequences of a light chain variable region set forth in SEQ ID NO: 4; or
(iii) HCDR1, 2 and 3 sequences of a heavy chain variable region set forth in SEQ ID NO: 5, and LCDR1, 2 and 3 sequences of a light chain variable region set forth in SEQ ID NO: 6; or
(iv) HCDR1, 2 and 3 sequences of a heavy chain variable region set forth in SEQ ID NO: 7, and LCDR1, 2 and 3 sequences of a light chain variable region set forth in SEQ ID NO: 8; or
(v) a variant of the CDR combination of any one of (i)-(iv), preferably the variant comprising 1-10 amino acid alterations (including but not limited to, insertions, deletions and/or substitutions, preferably amino acid substitutions, and more preferably conservative substitutions) in total in 6 CDRs, and preferably the heavy chain CDR3 remaining unchanged.

Preferably, the above CDRs are defined according to Kabat or IMGT or a combination thereof, and more preferably according to IMGT. However, the CDRs may also be defined in any other methods known in the art.

In some embodiments, the anti-TF antibody or the antigen-binding fragment thereof according to the present invention comprises 3 complementarity determining regions of a heavy chain variable region (HCDRs), and 3 complementarity determining regions of a light chain variable region (LCDRs), wherein
(i) according to the Chothia scheme, the HCDR1 comprises or consists of an amino acid sequence set forth in SEQ ID NO: 9, the HCDR2 comprises or consists of an amino acid sequence set forth in SEQ ID NO: 10, the HCDR3 comprises or consists of an amino acid sequence set forth in SEQ ID NO: 11, the LCDR1 comprises or consists of an amino acid sequence set forth in SEQ ID NO: 21, the LCDR2 comprises or consists of an amino acid sequence set forth in SEQ ID NO: 22, and the LCDR3 comprises or consists of an amino acid sequence set forth in SEQ ID NO: 23; or
(ii) according to the AbM scheme, the HCDR1 comprises or consists of an amino acid sequence set forth in SEQ ID NO: 12, the HCDR2 comprises or consists of an amino acid sequence set forth in SEQ ID NO: 13, the HCDR3 comprises or consists of an amino acid sequence set forth in SEQ ID NO: 14, the LCDR1 comprises or consists of an amino acid sequence set forth in SEQ ID NO: 24, the LCDR2 comprises or consists of an amino acid sequence set forth in SEQ ID NO: 25, and the LCDR3 comprises or consists of an amino acid sequence set forth in SEQ ID NO: 26; or
(iii) according to the Kabat scheme, the HCDR1 comprises or consists of an amino acid sequence set forth in SEQ ID NO: 15, the HCDR2 comprises or consists of an amino acid sequence set forth in SEQ ID NO: 16, the HCDR3 comprises or consists of an amino acid sequence set forth in SEQ ID NO: 17, the LCDR1 comprises or consists of an amino acid sequence set forth in SEQ ID NO: 27, the LCDR2 comprises or consists of an amino acid sequence set forth in SEQ ID NO: 28, and the LCDR3 comprises or consists of an amino acid sequence set forth in SEQ ID NO: 29; or
(iv) according to the IMGT scheme, the HCDR1 comprises or consists of an amino acid sequence set forth in SEQ ID NO: 18, the HCDR2 comprises or consists of an amino acid sequence set forth in SEQ ID NO: 19, the HCDR3 comprises or consists of an amino acid sequence set forth in SEQ ID NO: 20, the LCDR1 comprises or consists of an amino acid sequence set forth in SEQ ID NO: 30, the LCDR2 comprises or consists of an amino acid sequence set forth in SEQ ID NO: 31, and the LCDR3 comprises or consists of an amino acid sequence set forth in SEQ ID NO: 32.

In some embodiments, the anti-TF antibody or the antigen-binding fragment thereof according to the present invention comprises 3 complementarity determining regions of a heavy chain variable region (HCDRs), and 3 complementarity determining regions of a light chain variable region (LCDRs), wherein
(i) according to the Chothia scheme, the HCDR1 comprises or consists of an amino acid sequence set forth in SEQ ID NO: 33, the HCDR2 comprises or consists of an amino acid sequence set forth in SEQ ID NO: 34, the HCDR3 comprises or consists of an amino acid sequence set forth in SEQ ID NO: 35, the LCDR1 comprises or consists of an amino acid sequence set forth in SEQ ID NO: 45, the LCDR2 comprises or consists of an amino acid sequence set forth in SEQ ID NO: 46, and the LCDR3 comprises or consists of an amino acid sequence set forth in SEQ ID NO: 47; or
(ii) according to the AbM scheme, the HCDR1 comprises or consists of an amino acid sequence set forth in SEQ ID NO: 36, the HCDR2 comprises or consists of an amino acid sequence set forth in SEQ ID NO: 37, the HCDR3 comprises or consists of an amino acid sequence set forth in SEQ ID NO: 38, the LCDR1 comprises or consists of an amino acid sequence set forth in SEQ ID NO: 48, the LCDR2 comprises or consists of an amino acid sequence set forth in SEQ ID NO: 49, and the LCDR3 comprises or consists of an amino acid sequence set forth in SEQ ID NO: 50; or
(iii) according to the Kabat scheme, the HCDR1 comprises or consists of an amino acid sequence set forth in SEQ ID NO: 39, the HCDR2 comprises or consists of an amino acid sequence set forth in SEQ ID NO: 40, the HCDR3 comprises or consists of an amino acid sequence set forth in SEQ ID NO: 41, the LCDR1 comprises or consists of an amino acid sequence set forth in SEQ ID NO: 51, the LCDR2 comprises or consists of an amino acid sequence set forth in SEQ ID NO: 52, and the LCDR3 comprises or consists of an amino acid sequence set forth in SEQ ID NO: 53; or
(iv) according to the IMGT scheme, the HCDR1 comprises or consists of an amino acid sequence set forth in SEQ ID NO: 42, the HCDR2 comprises or consists of an amino acid sequence set forth in SEQ ID NO: 43, the HCDR3 comprises or consists of an amino acid sequence set forth in SEQ ID NO: 44, the LCDR1 comprises or consists of an amino acid sequence set forth in SEQ ID NO: 54, the LCDR2 comprises or consists of an amino acid sequence set forth in SEQ ID NO: 55, and the LCDR3 comprises or consists of an amino acid sequence set forth in SEQ ID NO: 56.

In some embodiments, the anti-TF antibody or the antigen-binding fragment thereof according to the present invention comprises 3 complementarity determining regions of a heavy chain variable region (HCDRs), and 3 complementarity determining regions of a light chain variable region (LCDRs), wherein
(i) according to the Chothia scheme, the HCDR1 comprises or consists of an amino acid sequence set forth in SEQ ID NO: 57, the HCDR2 comprises or consists of an amino acid sequence set forth in SEQ ID NO: 58, the HCDR3 comprises or consists of an amino acid sequence set forth in SEQ ID NO: 59, the LCDR1 comprises or consists of an amino acid sequence set forth in SEQ ID NO: 69, the LCDR2 comprises or consists of an amino acid sequence set forth in SEQ ID NO: 70, and the LCDR3 comprises or consists of an amino acid sequence set forth in SEQ ID NO: 71; or
(ii) according to the AbM scheme, the HCDR1 comprises or consists of an amino acid sequence set forth in SEQ ID NO: 60, the HCDR2 comprises or consists of an amino acid sequence set forth in SEQ ID NO: 61, the HCDR3 comprises or consists of an amino acid sequence set forth in SEQ ID NO: 62, the LCDR1 comprises or consists of an amino acid sequence set forth in SEQ ID NO: 72, the LCDR2 comprises or consists of an amino acid sequence set forth in SEQ ID NO: 73, and the LCDR3 comprises or consists of an amino acid sequence set forth in SEQ ID NO: 74; or
(iii) according to the Kabat scheme, the HCDR1 comprises or consists of an amino acid sequence set forth in SEQ ID NO: 63, the HCDR2 comprises or consists of an amino acid sequence set forth in SEQ ID NO: 64, the HCDR3 comprises or consists of an amino acid sequence set forth in SEQ ID NO: 65, the LCDR1 comprises or consists of an amino acid sequence set forth in SEQ ID NO: 75, the LCDR2 comprises or consists of an amino acid sequence set forth in SEQ ID NO: 76, and the LCDR3 comprises or consists of an amino acid sequence set forth in SEQ ID NO: 77; or
(iv) according to the IMGT scheme, the HCDR1 comprises or consists of an amino acid sequence set forth in SEQ ID NO: 66, the HCDR2 comprises or consists of an amino acid sequence set forth in SEQ ID NO: 67, the HCDR3 comprises or consists of an amino acid sequence set forth in SEQ ID NO: 68, the LCDR1 comprises or consists of an amino acid sequence set forth in SEQ ID NO: 78, the LCDR2 comprises or consists of an amino acid sequence set forth in SEQ ID NO: 79, and the LCDR3 comprises or consists of an amino acid sequence set forth in SEQ ID NO: 80.

In some embodiments, the anti-TF antibody or the antigen-binding fragment thereof according to the present invention comprises 3 complementarity determining regions of a heavy chain variable region (HCDRs), and 3 complementarity determining regions of a light chain variable region (LCDRs), wherein
(i) according to the Chothia scheme, the HCDR1 comprises or consists of an amino acid sequence set forth in SEQ ID NO: 81, the HCDR2 comprises or consists of an amino acid sequence set forth in SEQ ID NO: 82, the HCDR3 comprises or consists of an amino acid sequence set forth in SEQ ID NO: 83, the LCDR1 comprises or consists of an amino acid sequence set forth in SEQ ID NO: 93, the LCDR2 comprises or consists of an amino acid sequence set forth in SEQ ID NO: 94, and the LCDR3 comprises or consists of an amino acid sequence set forth in SEQ ID NO: 95; or
(ii) according to the AbM scheme, the HCDR1 comprises or consists of an amino acid sequence set forth in SEQ ID NO: 84, the HCDR2 comprises or consists of an amino acid sequence set forth in SEQ ID NO: 85, the HCDR3 comprises or consists of an amino acid sequence set forth in SEQ ID NO: 86, the LCDR1 comprises or consists of an amino acid sequence set forth in SEQ ID NO: 96, the LCDR2 comprises or consists of an amino acid sequence set forth in SEQ ID NO: 97, and the LCDR3 comprises or consists of an amino acid sequence set forth in SEQ ID NO: 98; or
(iii) according to the Kabat scheme, the HCDR1 comprises or consists of an amino acid sequence set forth in SEQ ID NO: 87, the HCDR2 comprises or consists of an amino acid sequence set forth in SEQ ID NO: 88, the HCDR3 comprises or consists of an amino acid sequence set forth in SEQ ID NO: 89, the LCDR1 comprises or consists of an amino acid sequence set forth in SEQ ID NO: 99, the LCDR2 comprises or consists of an amino acid sequence set forth in SEQ ID NO: 100, and the LCDR3 comprises or consists of an amino acid sequence set forth in SEQ ID NO: 101; or
(iv) according to the IMGT scheme, the HCDR1 comprises or consists of an amino acid sequence set forth in SEQ ID NO: 90, the HCDR2 comprises or consists of an amino acid sequence set forth in SEQ ID NO: 91, the HCDR3 comprises or consists of an amino acid sequence set forth in SEQ ID NO: 92, the LCDR1 comprises or consists of an amino acid sequence set forth in SEQ ID NO: 102, the LCDR2 comprises or consists of an amino acid sequence set forth in SEQ ID NO: 103, and the LCDR3 comprises or consists of an amino acid sequence set forth in SEQ ID NO: 104.

In some embodiments, the anti-TF antibody or the antigen-binding fragment thereof according to the present invention comprises a heavy chain variable region and a light chain variable region, wherein the heavy chain variable region comprises:
(i) an amino acid sequence set forth in SEQ ID NO: 1 or an amino acid sequence having at least 80%, 85%, 90%, 95%, 96%, 97%, 98% or 99% sequence identity thereto; or
(ii) an amino acid sequence set forth in SEQ ID NO: 3 or an amino acid sequence having at least 80%, 85%, 90%, 95%, 96%, 97%, 98% or 99% sequence identity thereto; or
(iii) an amino acid sequence set forth in SEQ ID NO: 5 or an amino acid sequence having at least 80%, 85%, 90%, 95%, 96%, 97%, 98% or 99% sequence identity thereto; or
(iv) an amino acid sequence set forth in SEQ ID NO: 7 or an amino acid sequence having at least 80%, 85%, 90%, 95%, 96%, 97%, 98% or 99% sequence identity thereto.

In some embodiments, the anti-TF antibody or the antigen-binding fragment thereof according to the present invention comprises a heavy chain variable region and a light chain variable region, wherein the light chain variable region comprises:
(i) an amino acid sequence set forth in SEQ ID NO: 2 or an amino acid sequence having at least 80%, 85%, 90%, 95%, 96%, 97%, 98% or 99% sequence identity thereto; or
(ii) an amino acid sequence set forth in SEQ ID NO: 4 or an amino acid sequence having at least 80%, 85%, 90%, 95%, 96%, 97%, 98% or 99% sequence identity thereto; or
(iii) an amino acid sequence set forth in SEQ ID NO: 6 or an amino acid sequence having at least 80%, 85%, 90%, 95%, 96%, 97%, 98% or 99% sequence identity thereto; or
(iv) an amino acid sequence set forth in SEQ ID NO: 8 or an amino acid sequence having at least 80%, 85%, 90%, 95%, 96%, 97%, 98% or 99% sequence identity thereto.

In some embodiments, the anti-TF antibody or the antigen-binding fragment thereof according to the present invention comprises a heavy chain variable region and a light chain variable region, wherein the heavy chain variable region comprises:
(i) an amino acid sequence set forth in SEQ ID NO: 1 or an amino acid sequence having at least 80%, 85%, 90%, 95%, 96%, 97%, 98% or 99% sequence identity thereto; or
(ii) an amino acid sequence set forth in SEQ ID NO: 3 or an amino acid sequence having at least 80%, 85%, 90%, 95%, 96%, 97%, 98% or 99% sequence identity thereto,
and/or wherein the light chain variable region comprises:
(i) an amino acid sequence set forth in SEQ ID NO: 2 or an amino acid sequence having at least 80%, 85%, 90%, 95%, 96%, 97%, 98% or 99% sequence identity thereto; or
(ii) an amino acid sequence set forth in SEQ ID NO: 4 or an amino acid sequence having at least 80%, 85%, 90%, 95%, 96%, 97%, 98% or 99% sequence identity thereto.

In some embodiments, the anti-TF antibody or the antigen-binding fragment thereof according to the present invention comprises a heavy chain variable region and a light chain variable region, wherein the heavy chain variable region comprises:
(i) an amino acid sequence set forth in SEQ ID NO: 5 or an amino acid sequence having at least 80%, 85%, 90%, 95%, 96%, 97%, 98% or 99% sequence identity thereto; or
(ii) an amino acid sequence set forth in SEQ ID NO: 7 or an amino acid sequence having at least 80%, 85%, 90%, 95%, 96%, 97%, 98% or 99% sequence identity thereto,
and/or wherein the light chain variable region comprises:
(i) an amino acid sequence set forth in SEQ ID NO: 6 or an amino acid sequence having at least 80%, 85%, 90%, 95%, 96%, 97%, 98% or 99% sequence identity thereto; or
(ii) an amino acid sequence set forth in SEQ ID NO: 8 or an amino acid sequence having at least 80%, 85%, 90%, 95%, 96%, 97%, 98% or 99% sequence identity thereto.

In some preferred embodiments, the anti-TF antibody or the antigen-binding fragment thereof according to the present invention comprises a heavy chain variable region and a light chain variable region, wherein the heavy chain variable region comprises an amino acid sequence set forth in SEQ ID NO: 1 or an amino acid sequence having at least 95%, 96%, 97%, 98% or 99% sequence identity thereto, and wherein the light chain variable region comprises an amino acid sequence set forth in SEQ ID NO: 2 or an amino acid sequence having at least 95%, 96%, 97%, 98% or 99% sequence identity thereto. Preferably, the antibody or the antigen-binding fragment comprises a heavy chain variable region of SEQ ID NO: 1 and a light chain variable region of SEQ ID NO: 2.

In some preferred embodiments, the anti-TF antibody or the antigen-binding fragment thereof according to the present invention comprises a heavy chain variable region and a light chain variable region, wherein the heavy chain variable region comprises an amino acid sequence set forth in SEQ ID NO: 3 or an amino acid sequence having at least 95%, 96%, 97%, 98% or 99% sequence identity thereto, and wherein the light chain variable region comprises an amino acid sequence set forth in SEQ ID NO: 4 or an amino acid sequence having at least 95%, 96%, 97%, 98% or 99% sequence identity thereto. Preferably, the antibody or the antigen-binding fragment comprises a heavy chain variable region of SEQ ID NO: 3 and a light chain variable region of SEQ ID NO: 4.

In some preferred embodiments, the anti-TF antibody or the antigen-binding fragment thereof according to the present invention comprises a heavy chain variable region and a light chain variable region, wherein the heavy chain variable region comprises an amino acid sequence set forth in SEQ ID NO: 5 or an amino acid sequence having at least 95%, 96%, 97%, 98% or 99% sequence identity thereto, and wherein the light chain variable region comprises an amino acid sequence set forth in SEQ ID NO: 6 or an amino acid sequence having at least 95%, 96%, 97%, 98% or 99% sequence identity thereto. Preferably, the antibody or the antigen-binding fragment comprises a heavy chain variable region of SEQ ID NO: 5 and a light chain variable region of SEQ ID NO: 6.

In some preferred embodiments, the anti-TF antibody or the antigen-binding fragment thereof according to the present invention comprises a heavy chain variable region and a light chain variable region, wherein the heavy chain variable region comprises an amino acid sequence set forth in SEQ ID NO: 7 or an amino acid sequence having at least 95%, 96%, 97%, 98% or 99% sequence identity thereto, and wherein the light chain variable region comprises an amino acid sequence set forth in SEQ ID NO: 8 or an amino acid sequence having at least 95%, 96%, 97%, 98% or 99% sequence identity thereto. Preferably, the antibody or the antigen-binding fragment comprises a heavy chain variable region of SEQ ID NO: 7 and a light chain variable region of SEQ ID NO: 8.

In some embodiments, the anti-TF antibody or the antigen-binding fragment thereof according to the present invention comprises heavy and light chain variable region sequences or variants thereof of any one of the exemplary antibodies 2B12B10-hz1, 30G11B7-hz1, 22F11H5-hz1, and 27H8H3-hz1 of the present invention, e.g., an antibody having the same CDR sequences as one of the exemplary antibodies, and having the same or different framework region sequences. Preferably, the antibody is a humanized antibody. More preferably, the heavy chain variable region of the antibody has a framework region sequence derived from a human germline.

The antibody according to the present invention may comprise a heavy chain constant region and/or a light chain constant region. Preferably, the heavy chain constant region is a heavy chain constant region derived from a human immunoglobulin. Preferably, the light chain constant region is a light chain constant region derived from a human immunoglobulin. The heavy chain constant region contained in the antibody of the present invention may be of any isotype or subtype, such as a heavy chain constant region of IgG1, IgG2, IgG3, or IgG4 isotype. Thus, in some embodiments, the present invention provides an anti-TF antibody comprising an IgG1, IgG2, IgG3, or IgG4 heavy chain constant region, preferably an IgG1 heavy chain constant region, particularly a human IgG1 heavy chain constant region, e.g., an amino acid sequence set forth in SEQ ID NO: 106 or an amino acid sequence having at least 90%, 95%, 96%, 97%, 98% or 99% sequence identity thereto. The light chain constant region contained in the antibody of the present invention may be a κ light chain constant region or a λ light chain constant region. Thus, in some embodiments, the present invention provides an anti-TF antibody comprising a κ light chain constant region or a λ light chain constant region, preferably the antibody of the present invention comprises a human κ light chain constant region, e.g., an amino acid sequence set forth in SEQ ID NO: 105 or an amino acid sequence having at least 90%, 95%, 96%, 97%, 98% or 99% sequence identity thereto. In some embodiments, the heavy and/or light chain of the anti-TF antibody or the fragment thereof of the present invention further comprises a signal peptide sequence.

In some aspects, the present invention further provides variants of any of the antibodies according to the present invention described herein, particularly variants of any one of the exemplary antibodies 2B12B10-hz1, 30G11B7-hz1, 22F11H5-hz1, and 27H8H3-hz1 of the present invention.

Herein, the term "variant" related to an antibody refers to an antibody that comprises a target antibody region having amino acid alterations by virtue of at least 1, such as 1-30, 1-20 or 1-10, e.g. 1, 2, 3, 4 or 5 amino acid substitutions, deletions and/or insertions compared to a given parent antibody, wherein the variant substantially retains at least one biological property (e.g. antigen-binding capacity) of the parent antibody molecule. The target antibody region may be the full length of the antibody, the heavy chain variable region or the light chain variable region or a combination thereof, or one or more heavy chain CDRs or one or more light chain CDRs or a combination thereof. Such variants of particular exemplary antibodies of the present invention are contemplated by the present invention.

In some embodiments, the antibody variants of the present invention preferably retain at least 60%, 70%, 80%, 90% or 100% of the biological activity (e.g., substantially equivalent antigen-binding capacity and/or receptor endocytic activity, or substantially equivalent degree of effect on coagulation) of the parent antibody prior to the introduction of the alterations or modifications. It will be understood that the heavy chain variable regions or the light chain variable regions or the CDRs or the framework regions of the antibodies according to the present invention may be altered independently or in combination to produce the antibody variants of the present invention. In addition, the Fc regions of the antibodies according to the present invention may also be altered. The alterations in the Fc regions may be made alone or in combination with the alterations in the framework regions and/or CDRs and/or the heavy chain variable regions and/or the light chain variable regions. The Fc regions may be selectively altered depending on the particular application of the antibody molecule, for example, to confer one or more altered effector functions to the antibody, such as altered serum half-life, complement fixation, Fc receptor binding, and/or antigen-dependent cytotoxicity. In addition, the antibody of the present invention may be chemically modified (e.g., linked to PEG), or its glycosylation pattern may be altered.

In some embodiments, the antibody variants according to the present invention have amino acid alterations in the heavy chain sequence and/or the light chain sequence relative to any one of the exemplary antibodies of the present invention. Preferably, the variant comprises an amino acid sequence comprising at least one, two or three but no more than 20, 10 or 5 amino acid alterations, or an amino acid sequence having at least 80%, 85%, 90%, 92%, 95%, 97%, 98%, 99% or more identity to the corresponding antibody, in the heavy chain sequence or the light chain sequence or both. Preferably, the amino acid alterations do not occur in the CDRs, and more preferably, the amino acid alterations do not occur in the variable regions. In some embodiments, at least some or all of the amino acid alterations contained in the antibody variant are conservative amino acid substitutions. The "conservative substitution" refers to an amino acid alteration that results in the replacement of an amino acid with a chemically similar amino acid. Amino acid modifications such as substitutions can be introduced into the antibody of the present invention by standard methods known in the art, such as site-directed mutagenesis and PCR-mediated mutagenesis, to achieve conservative substitutions.

In some other aspects, the present invention further provides an anti-tissue factor (TF) antibody or an antigen-binding fragment thereof having one or more of the following properties, relative to any given antibody according to the present invention described above (particularly any one of the exemplary antibodies 2B12B10-hz1, 30G11B7-hz1, 22F11H5-hz1 and 27H8H3-hz1 of the present invention): (i) binding to the same or overlapping epitope as a given antibody according to the present invention; (ii) competing for binding to a human tissue factor (TF) with a given antibody according to the present invention; (iii) inhibiting (e.g., competitively inhibiting) the binding of a given antibody according to the present invention to a cell expressing a human tissue factor on the cell surface; and preferably exhibiting the same or similar binding affinity and/or specificity as a given antibody according to the present invention.

Herein, an antibody that "competes for binding to a TF antigen" with a given antibody refers to an antibody that blocks 50% or more of the binding of the given antibody to the TF antigen in a competitive assay; and conversely, the given antibody also blocks 50% or more of the binding of the antibody to the TF antigen in a competitive assay. Exemplary competitive assays are described in: Antibodies, Harbor and Lane (Cold Spring Harbor Press, Cold Spring Harbor, NY). The antibody that competes for binding can bind to the same epitope region, e.g., the same epitope, adjacent epitopes, or overlapping epitopes, as the given antibody.

Herein, an antibody that inhibits (e.g., competitively inhibits) the binding of a given antibody to its antigen refers to an antibody that inhibits 50%, 60%, 70%, 80%, 90%, or 95% or more of the binding of the given antibody to its antigen; and conversely, the given antibody also inhibits 50%, 60%, 70%, 80%, 90%, or 95% or more of the binding of the antibody to its antigen. The binding of an antibody to its antigen can be measured by binding affinity. Methods for determining affinity are known in the art.

Herein, an antibody that exhibits the same or similar binding affinity and/or specificity as a given antibody refers to an antibody that is capable of having at least 50%, 60%, 70%, 80%, 90% or 95% or more of the binding affinity and/or specificity of the given antibody. This can be determined by any method known in the art for determining the binding affinity and/or specificity.

In some embodiments, the antibody according to the present invention is an IgG1 antibody, particularly an IgG1κ or IgG1λ isotype. Preferably, the IgG1 antibody according to the present invention has a human IgG1 constant region, and more preferably is a human IgG1κ isotype.

### Properties of anti-TF antibody of the present invention

The anti-TF antibody of the present invention has one or more of the following properties:
(i) binding to the extracellular domain of human TF with high affinity;
(ii) binding to TF-positive tumor cells with high affinity;
(iii) having cross-reactivity with cynomolgus monkey TF;
(iv) having TF receptor-mediated endocytic activity;
(v) blocking the TF/FVIIa complex-mediated downstream signaling pathway in TF-positive cells; and
(vi) not substantially affecting coagulation initiated by TF on the cell surface, or having a reduced coagulation effect.

In some aspects, the antibody of the present invention has a high binding affinity and high binding specificity for TF. The binding EC50 value of the antibody of the present invention to the extracellular domain of TF and/or TF-positive tumor cells can be determined by FACS or ELSA assays (e.g., the assays described in the examples). Alternatively, the binding kinetic parameters of the antibody can be determined by bio-layer interferometry to characterize the binding affinity and specificity of the antibody. In some embodiments, the anti-TF antibody of the present invention has a K_{Ð} value of less than 10⁻⁷ M, such as a K_{D} value of about 10⁻⁸ M or less, preferably less than 5 × 10⁻⁸ M, as determined by bio-layer interferometry (BIL). In other embodiments, the antibody of the present invention has a K_{d} value of about 10⁻¹ to 10⁻³ s⁻¹, such as a K_{d} value of about 10⁻² or about 10⁻³ s⁻¹, as determined by bio-layer interferometry (BIL). Preferably, the binding kinetic parameters of the antibody, such as K_{D} and K_{d} values, are determined using the ForteBio assay method described in the examples; and optionally, such values are compared to those of a reference antibody in the same assay. In some embodiments, the antibody has the same or similar, or smaller binding kinetic K_{D} value as compared to the reference antibody. In other embodiments, the antibody has a relatively fast dissociation rate (K_{d} value) as compared to the reference antibody. Previous studies have shown that in some cases, antibodies with faster dissociation rates can infiltrate tumors more effectively, thereby facilitating the distribution of the antibody in solid tumors (see, Influence of Affinity and Antigen Internalization on the Uptake and Penetration of Anti-HER2 Antibodies in Solid Tumors; DOI: 10.1158/0008-5472.CAN-10-2277).

In some embodiments, the anti-TF antibody of the present invention binds strongly to TF-positive tumor cells. The cell binding EC50 value (i.e., the half maximal effective concentration for cell binding) and/or the maximum binding amount of the antibody can be determined by cell ELISA or flow cytometry and compared to the reference antibody to reflect the cell binding affinity of the antibody. In some embodiments, the EC₅₀ value of the antibody of the present invention is about 50% to about 160%, such as about 80%, about 90%, about 100%, about 110%, about 120%, or about 130%, of the EC₅₀ value of the reference antibody in at least one TF-positive tumor cell, e.g., a NIC-H358 cell or a KYSE50 cell.

In some embodiments, the anti-TF antibody of the present invention exhibits cross-reactivity with human and monkey TFs. In some embodiments, the antibody of the present invention binds to human TF with a K_{D} value that is approximately comparable to a K_{D} value of the antibody binding to monkey TF, for example, the ratio of the two K_{D} values is between 1 and 10, e.g., between 1 and 5, and preferably between about 1 and 3. In still other embodiments, the antibody binds to human TF with a K_{d} value that is approximately comparable to a K_{d} value of the antibody binding to monkey TF, for example, the ratio of the two K_{d} values is between 1 and 10, e.g., between 1 and 5, and preferably between about 1 and 3.

In some embodiments, the antibody of the present invention has TF receptor-mediated endocytic activity. The evaluation of the endocytic activity of antibodies can be performed in a cell-based assay, such as the assay described in the examples. In some embodiments, in a TF-positive cell-based assay, the antibody to be tested is incubated with TF-positive cells (especially TF-positive tumor cells) at 37 °C for a period of time (e.g., 2 h or 4 h) relative to a negative control maintained at 4 °C for the same period of time, and the change in the amount of the TF antibody binding to the cell surface is determined using fluorescence detection by flow cytometry, to determine the endocytosis rate of the antibody. In some embodiments, the antibody of the present invention has a substantially comparable or superior endocytosis rate compared to the reference antibody. In other embodiments, the endocytosis rate of the antibody of the present invention is about 50%-200% or more, e.g., about 70% or more, such as about 80% or more, preferably about 100% or more, or about 110% or more, and more preferably about 120% or more, such as about 130% or more, about 150% or more, or about 200% or more, of the endocytosis rate of the reference antibody under equivalent assay conditions. In still other embodiments, the antibody of the present invention exhibits more rapid endocytosis, for example, reaching an endocytosis rate of about 30%, 40%, or 50% in a shorter time, compared to the reference antibody. In still other embodiments, the antibody of the present invention has an endocytosis rate of at least 10%, such as 15-20%, preferably at least 25%, such as 20-35%, and more preferably 35% or more, such as about 40% or 45% or more, e.g., after incubation at 37 °C for 2 h, in an evaluation assay of the endocytic activity as described in the examples.

In some embodiments, the antibody of the present invention does not substantially affect coagulation, or has a reduced coagulation effect. The effect of the antibody on coagulation can be determined by detecting the interference of the antibody on coagulation initiated by TF-positive cells in the presence of Ca²⁺ ions and human plasma in a cell-based *in vitro* coagulation assay. In some embodiments, the antibody of the present invention does not substantially affect coagulation initiated by TF on the cell surface compared to the negative control without the antibody, or has a reduced coagulation effect compared to the reference antibody. Changes in coagulation time caused by the antibody can be detected relative to the negative control without the antibody and/or the reference antibody, thereby reflecting the effect of the antibody on coagulation. Preferably, the effect of the antibody on the TF-mediated coagulation process is characterized by detecting the change in absorbance at 405 nm over time following the addition of human plasma to a solution containing TF-positive cells and high or low concentrations of free Ca²⁺ ions and the antibody to be tested, and estimating the coagulation time (V50) required to reach the half maximal OD405 absorbance value, according to a coagulation assay described in the examples. The concentration of Ca²⁺ ions used for the detection may be one or more concentrations selected from 1-5 mM, such as 2 mM and 5 mM. The concentration of the antibody used for the detection may be one or more concentrations selected from 10-50 µg/mL, such as 20 µg/mL and 50 µg/mL. In some embodiments, the effect of the antibody on coagulation is determined in the presence of 5 mM Ca²⁺ and 20 µg/mL antibody. In other embodiments, the effect of the antibody on coagulation is determined in the presence of 2 mM Ca²⁺ and 50 µg/mL antibody. In some embodiments, it can be considered that the antibody does not substantially affect coagulation, or has a reduced coagulation effect, if the coagulation time (V50) caused by the antibody is delayed by no more than 50 seconds, preferably no more than 40 seconds, no more than 30 seconds, no more than 20 seconds, 10 seconds, or 5 seconds, relative to the negative control without the antibody under at least one assay condition, e.g., in the presence of 5 mM Ca²⁺ and 20 µg/mL antibody. In other embodiments, it can be considered that the antibody does not substantially affect coagulation, or has a reduced coagulation effect, if the antibody does not cause a delay in the coagulation time (V50), or preferably, the coagulation time (V50) is reduced by about 10% or more, or more preferably about 20% or 30% or 40% or more, and still more preferably about 50% or 60% or more, relative to the reference antibody under at least one assay condition, e.g., in the presence of 5 mM Ca²⁺ and 20 µg/mL antibody. In still other embodiments, it can be considered that the antibody does not substantially affect coagulation, or has a reduced coagulation effect, if the antibody does not cause a delay in the coagulation time (V50), or preferably, the coagulation time (V50) is reduced by about 10%, 20%, or 30% or more, relative to the reference antibody under at least one assay condition, e.g., in the presence of 2 mM Ca²⁺ and 50 µg/mL antibody.

In still other embodiments, the antibody of the present invention has the ability to block the TF/FVIIa complex-mediated downstream signaling pathway in TF-positive tumor cells. The blocking activity of the anti-TF antibody against the TF downstream signaling pathway can be determined by detecting the level of IL-8 release resulting from the activation of this pathway. In some embodiments, the antibody of the present invention exhibits an inhibitory effect on the TF signaling pathway according to a blocking activity evaluation assay on the signaling pathway described in the examples. In some embodiments, the antibody of the present invention has blocking activity on the TF signaling pathway with an IC₅₀ value of less than 500 ng/mL or 150 ng/mL and a maximum inhibition rate of 90% or more, preferably 95% or more. In some embodiments, the antibody of the present invention has blocking activity on the TF signaling pathway with an IC₅₀ value comparable to (±10%) or less than that of the reference antibody.

In still other embodiments, the anti-TF antibody or the antigen-binding fragments thereof of the present invention further has one or more of the following properties: (vii) a good production performance; (viii) a good stability; and (ix) favorable pharmacokinetic properties.

In some embodiments, after one-step purification of the antibody product produced from recombinant mammalian cells using protein A affinity chromatography, the purified antibody of the present invention can have a purity of 90% or more as determined by SEC-HPLC, and exhibits good hydrophilicity in HIC assay.

In still other embodiments, the antibody of the present invention has good stability, including thermal stability and repeated freezing-thawing stability. In some embodiments, the SEC-HPLC purity (i.e., the percentage of antibody monomer as determined by SEC-HPCL) of the antibodies of the present invention is maintained above 90% or preferably above 95%, or decreases by no more than 5% or preferably no more than 3%, after being left to stand at 37 °C for 3-14 days according to an accelerated stability assay in the examples. In other embodiments, the non-reduced CE-SDS purity (i.e., the percentage of main peak) is maintained above 85% or preferably above 90%, the reduced CE-SDS purity (i.e., the percentage of LC + HC) is maintained above 90% or preferably above 95%, or either non-reduced purity or the reduced purity is decreased by no more than 5% or 4%. In still other embodiments, the SEC purity of the antibodies of the present invention is maintained above 95% or decreases by no more than 5% or preferably no more than 3% after repeated freezing-thawing for 4-8 times according to a repeated freezing-thawing assay in the examples.

In still other embodiments, the antibody of the present invention has serum stability. The serum stability of the antibody can be determined by detecting the change in affinity for the TF antigen after incubation of the antibody in serum at 37 °C for a period of time according to a serum stability assay in the examples. It can be considered that the antibody has serum stability if the change in affinity is not significant. In some embodiments, the antibody of the present invention can be retained in human serum for at least 14 days with 90% or more retention of its antigen-binding affinity.

### II. Polynucleotide, vector, and host

In yet another aspect, the present invention provides a nucleic acid encoding any of the above anti-TF antibodies or fragments thereof. Further provided are a vector comprising the nucleic acid, and a host cell comprising the nucleic acid or the vector.

In some embodiments, the present invention provides a nucleic acid molecule comprising a polynucleotide encoding at least one CDR and typically all three CDRs from a heavy chain (VH) sequence or a light chain (VL) sequence of the antibody binding to TF described above. In some further embodiments, the present invention provides a nucleic acid molecule comprising a polynucleotide encoding a complete or substantially complete variable region sequence of the heavy chain and/or the light chain of the anti-TF antibody described above. As will be understood by those skilled in the art, each antibody or polypeptide amino acid sequence can be encoded by a variety of nucleic acid sequences because of codon degeneracy.

In one embodiment, the present invention provides one or more vectors comprising the nucleic acid of the present invention, including cloning vectors and expression vectors. In one embodiment, the vector is an expression vector, such as a eukaryotic expression vector. The vector that can be used in the present invention includes, but is not limited to, a virus, a plasmid, a cosmid, a λ phage or a yeast artificial chromosome (YAC).

In one embodiment, the present invention provides a host cell comprising the vector of the present invention. The suitable host cell for cloning or expressing the vector encoding the antibody includes a prokaryotic or eukaryotic cell. For example, the antibody can be produced in bacteria, particularly when glycosylation and Fc effector functions are not required. After expression in bacteria, such as *E.coli*, the antibody can be isolated from the bacterial cell paste in the soluble fraction and can be further purified. In yet another embodiment, the host cell is a eukaryotic cell. In another embodiment, the host cell is selected from a yeast cell, a mammalian cell, and other cells suitable for preparing an antibody or an antigen-binding fragment thereof. Examples of useful mammalian host cell lines include monkey kidney CV1 line (COS-7) transformed with SV40; human embryonic kidney line (293HEK or 293 cells); Chinese hamster ovary (CHO) cells, including DHFR-CHO cells; and myeloma cell lines such as Y0, NS0 and Sp2/0.

### III. Preparation of antibodies

In yet another aspect, the present invention provides a method for preparing the anti-TF antibody of the present invention.

In one embodiment, the method of the present invention comprises culturing a host cell comprising a nucleic acid encoding the antibody of the present invention under conditions suitable for expressing antibodies, and optionally recovering the antibody from the host cell (or the host cell culture medium). For recombinant production of the anti-TF antibody, an isolated or artificially synthesized or recombinantly synthesized nucleic acid encoding the antibody (e.g., the antibody described above) can be inserted into one or more vectors for further cloning and/or expression in the host cell.

In yet another embodiment, the present invention further provides an anti-TF antibody or an antigen-binding fragment thereof having one or more of the following properties, and a method for preparing the same:
(i) binding to the same or overlapping epitope as any one of the antibodies according to the present invention;
(ii) competing for binding to a human TF with any one of the antibodies according to the present invention; and
(iii) inhibiting (e.g., competitively inhibiting) the binding of any one of the antibodies according to the present invention to a cell expressing the human TF on the cell surface,

wherein the antibody according to the present invention is particularly antibody 2B12B10-hz1, 30G11B7-hz1, 22F11H5-hz1 or 27H8H3-hz1,
preferably, the preparation method comprises: using the antibody according to the present invention as a reference, and screening an antibody having the above properties from the antibody mixture in a binding affinity assay. The antibody mixture that can be used herein includes, but is not limited to, antiserum from TF-immunized animals, or a yeast or mammalian display antibody library.

### IV. Assay

The anti-TF antibody provided herein can be identified, screened, or characterized for its physical/chemical properties and/or biological activity through a variety of assays known in the art.

Suitable methods that can be used to determine the binding of the antibody to the TF antigen are known in the art. For example, the affinity of the antibody for a protein comprising the extracellular domain of TF (e.g., a fusion protein) or TF-positive tumor cells can be readily determined using conventional techniques known to those skilled in the art. The methods include, but are not limited to, an assay using ELISA, an assay using a BIAcore 2000 instrument or ForteBio Octet^{®} instrument, a radioimmunoassay using radiolabeled target antigens, or cell ELISA or FACS technique or bio-layer interferometry described in the examples of the present application. Herein, preferably, the measurement of the affinity parameters is performed in accordance with or substantially in accordance with the method described in the examples.

Methods suitable for determining the endocytic activity of the antibody are known in the art. For example, the endocytosis rate of the antibody after binding to the cell surface can be determined in a cell-based assay, particularly in a TF-positive tumor cell-based assay. Herein, preferably, the endocytic activity of the antibody is performed in accordance with or substantially in accordance with the method described in the examples.

Methods suitable for determining the effect of the antibody on coagulation are known in the art. For example, the change in a coagulation parameter, such as coagulation time, in the presence of the antibody can be determined relative to a control (e.g., a negative control or a positive control) to reflect the coagulation effect of the antibody. Herein, preferably, the effect of the antibody on coagulation is performed in accordance with or substantially in accordance with the method described in the examples.

### V. Immunoconjugate and immunofusion

In yet another aspect, the present invention provides an immunofusion or an immunoconjugate produced by fusing or conjugating the antibody of the present invention to a heterologous molecule.

In one embodiment, in the immunofusion, the antibody (or the antigen-binding fragment thereof) of the present invention is linked to a heterologous peptide or a polypeptide molecule directly or through an amino acid linker. The heterologous peptides or polypeptides that can be mentioned include, but are not limited to, proteins or polypeptides conferring additional functional activity to the fusion, or tag peptides facilitating the purification or detection of the immunofusion.

In one embodiment, in the immunoconjugate, the antibody (or the antigen-binding fragment thereof) of the present invention is conjugated to a therapeutic agent or a diagnostic agent or a detectable agent. In some embodiments, the antibody of the present invention can be conjugated to the heterologous molecule in the form of a full-length antibody or an antibody fragment. In the conjugates, linkers can be used to covalently link different entities of the conjugates. Suitable linkers include chemical linkers or peptide linkers. Advantageously, the linkers are "cleavable linkers" that facilitate the release of the polypeptides following delivery to a target site. For example, acid-labile linkers, peptidase-sensitive linkers, photolabile linkers, dimethyl linkers or disulfide-containing linkers can be used.

In embodiments conjugated to a therapeutic agent, the therapeutic agent suitable for the conjugate includes, but is not limited to, a cytotoxin (e.g., a cytostatic agent or a cytotoxic agent), a drug, or a radioisotope.

In embodiments conjugated to a diagnostic agent or a detectable agent, such conjugates can be used as part of a clinical testing method (e.g., to determine the efficacy of a specific therapy) for monitoring or predicting the onset, development, progression, and/or severity of a disease or disorder. Such diagnosis and detection can be achieved by conjugating the antibody to the detectable agent, which includes, but is not limited to, a variety of enzymes, such as horseradish peroxidase; prosthetic groups, such as streptavidin/biotin and avidin/biotin; fluorescent substances; luminescent substances; radioactive substances; and positron-emitting metals and non-radioactive paramagnetic metal ions used in various positron emission tomography techniques.

### VI. Multispecific antibody

In yet another aspect, the present invention provides a multispecific (including bispecific) antibody molecule that specifically binds to TF. In one embodiment, in the multispecific antibody, the antibody (or the antigen-binding fragment thereof) of the present invention has a first binding specificity for TF. In yet another embodiment, the multispecific antibody further comprises a second binding specificity, or further comprises a second binding specificity and a third binding specificity for two different molecules. The second binding specificity and the third binding specificity may for example be directed against another antigen expressed on the surface of a tumor cell, or against an antigen expressed on the surface of a T cell.

In such multispecific molecules, the binding specificity is preferably dependent on a "binding site" or an "antigen-binding site" (a region of an antibody molecule that actually binds to an antigen) of the antibody. More preferably, the antigen-binding site is comprised of a VH/VL pair consisting of a light chain variable domain (VL) and a heavy chain variable domain (VH) of the antibody.

### VII. Pharmaceutical composition and pharmaceutical formulation

The present invention further provides a composition (including a pharmaceutical composition or a pharmaceutical formulation) comprising an anti-TF antibody or an immunoconjugate/fusion or a multispecific antibody thereof, and a composition comprising a polynucleotide encoding the anti-TF antibody or the immunoconjugate/fusion or the multispecific antibody thereof. Such compositions can further optionally comprise a suitable pharmaceutical auxiliary material, such as a pharmaceutical carrier and a pharmaceutical excipient known in the art, including buffers. For use and application of the excipients, see Handbook of Pharmaceutical Excipients, 5th Ed., R. C. Rowe, P. J. Seskey and S. C. Owen, Pharmaceutical Press, London, Chicago. In some embodiments, the pharmaceutical formulation comprising the present invention can be prepared by mixing the anti-TF antibody, the immunoconjugate or the multispecific antibody of the present invention of a desired purity with one or more optional pharmaceutical auxiliary materials (Remington's Pharmaceutical Sciences, 16th Ed., Osol, A. eds. (1980)).

In the pharmaceutical composition and the pharmaceutical formulation of the present invention, the antibody of the present invention can be the sole active agent, or can be combined with additional therapeutic agents. The therapeutic agents that can be combined with the antibody of the present invention include, but are not limited to, therapeutic agents having beneficial therapeutic efficacy for the disease and/or disorder to be treated. For example, the active ingredients can be those required for a specific indication being treated, preferably those having complementary activities that do not adversely affect one another. For example, additional pharmaceutical ingredients that provide anti-cancer activity can be provided. The antibody of the present invention is suitably present in combination with the active ingredients in an amount effective for an intended purpose in the pharmaceutical composition and the pharmaceutical formulation.

### VIII. Combination product

In yet another aspect, the present invention further provides a combination product comprising the antibody or the antigen-binding fragment thereof, the multispecific antibody or the immunoconjugate of the present invention, and one or more additional therapeutic agents (e.g., chemotherapeutic agents, other antibodies, cytotoxic agents, antineoplastic agents, etc.). The components that make up the combination product, such as the antibody of the present invention and the additional therapeutic agents, can be formulated separately in different formulations and preferably contained in different containers. The mode and order of administration of the components of the combination product can be determined by those skilled in the art based on factors such as the disease to be treated, the individual condition and the like. The combination product of the present invention can be used in a therapeutic method of the present invention. In some embodiments, the present invention provides a combination product, wherein the additional therapeutic agents refer to, for example, a therapeutic agent, such as an antibody, which is effective to stimulate an immune response and thus further enhance, stimulate or upregulate the immune response in a subject. In some embodiments, the combination product is used for preventing or treating TF-positive tumors.

### IX. Method and use

In one aspect, the present invention provides a method and use of the TF antibody and the antigen-binding fragment thereof of the present invention, for example, *in vivo* and *in vitro* for:
(1) targeting and binding to TF-positive tumor cells; and/or
(2) blocking the TF/VIIa-mediated downstream signaling pathway in the TF-positive tumor cells; and
(3) inhibiting and/or killing the TF-positive tumor cells.

In some embodiments, the method and use of the present invention relate to the treatment of a disease in an individual or a subject. In other embodiments, the method and use of the present invention relate to detecting the presence of TF in a sample from, for example, a subject. In still other embodiments, the present invention further provides use of the anti-TF antibody or the antigen-binding fragment thereof of the present invention in the preparation of a product (e.g., a pharmaceutical composition or a pharmaceutical product or a combination product or a test product) for the above-mentioned use.

In one aspect, the present invention provides a method and use of the antibody or the antigen-binding fragment of the present invention for preventing and/or treating a TF-positive tumor in a subject, comprising administering the antibody or the antigen-binding fragment of the present invention in a prophylactically and/or therapeutically effective amount. In another aspect, the present invention further provides a method and use of the antibody or the antigen-binding fragment of the present invention for treating or preventing other diseases associated with elevated expression of cell membrane TF in a subject, comprising administering to a subject in need thereof the anti-TF antibody or the antigen-binding fragment thereof of the present invention.

In yet another aspect, the present invention provides a method and a kit for detecting TF in a sample, wherein the method comprises: (a) contacting the sample with the antibody or the antigen-binding fragment thereof or the immunoconjugate/fusion of the present invention; and (b) detecting the formation of a complex of the antibody or the antigen-binding fragment thereof or the immunoconjugate with a TF protein. In some embodiments, the sample is from a cancer patient. The detection may be *in vitro* or *in vivo.*

The term "detection" or "detect" used herein includes quantitative and qualitative detections, and exemplary detections may involve immunohistochemistry, immunocytochemistry, flow cytometry (e.g., FACS), magnetic beads complexed with antibody molecules, ELISA, and PCR (e.g., RT-PCR). In certain embodiments, the biological sample is blood, serum, or other liquid samples of biological origin. In certain embodiments, the biological sample includes cells or tissues. In some embodiments, the biological sample is from a hyperproliferative or cancerous lesion. In certain embodiments, the TF to be detected is human TF.

In one embodiment, the anti-TF antibody is used to select a subject suitable for treatment with the anti-TF antibody. In yet another embodiment, the antibody of the present invention can be used to diagnose a cancer or tumor, e.g., to assess (e.g., monitor) the treatment or progression, diagnosis, and/or staging of a disease (e.g., the hyperproliferative or cancerous disease) described herein in a subject.

In certain embodiments, provided is a labeled anti-TF antibody. The label includes, but is not limited to, a label or moiety that is detected directly, e.g., a fluorescent label, a chromophoric label, an electron-dense label, a chemiluminescent label, and a radioactive label, and a moiety that is detected indirectly, such as an enzyme or a ligand, for example, by enzymatic reaction or molecular interaction. Exemplary labels include, but are not limited to, radioisotopes 32P, 14C, 125I, 3H and 1311, fluorophores (such as rare earth chelates or fluorescein) and derivatives thereof, rhodamine and derivatives thereof, dansyl, umbelliferone, luceriferase (such as firefly luciferase and bacterial luciferase (U.S. Patent No. 4,737,456)), fluorescein, 2,3-dihydrophthalazinedione, horseradish peroxidase (HR), alkaline phosphatase, β-galactosidase, glucoamylase, lysozyme, carbohydrate oxidase (such as glucose oxidase, galactose oxidase and glucose-6-phosphate dehydrogenase), heterocyclic oxidase (such as uricase and xanthine oxidase), enzymes oxidizing dye precursors with hydrogen peroxide (such as HR, lactoperoxidase, or microperoxidase), biotin/avidin, spin labels, phage labels, stable free radicals, etc.

### Treatment method and use of the present invention

Based on the good targeting of the anti-TF antibody of the present invention or the ADC of the present invention to TF and the above-mentioned excellent properties, the present invention further provides use and a method of the ADC of the present invention and the antibody of the present invention in the treatment and prevention of TF-related diseases.

The TFs are overexpressed on the surface of cancer tissue cells from a variety of origins, thereby being suitable targets for the development of cancer immunotherapies. Thus, in one aspect, the present invention provides use of the ADC or the pharmaceutical acceptable salt or solvate thereof of the present invention, and the antibody or the antigen-binding fragment thereof of the present invention for preventing and/or treating a TF-positive tumor in a subject. In the use, the antibody-drug conjugate or the pharmaceutical acceptable salt or solvate thereof of the present invention, or the antibody or the antigen-binding fragment thereof of the present invention may be administered to the subject as the sole active agent, or may be administered to the subject in combination with additional therapies or therapeutic agents. The additional therapies and therapeutic agents include, for example, drugs that target antigens on the surface of tumor cells to destroy tumors by binding and/or blocking these molecules; and drugs that activate the immune system of the subject to spontaneously destroy the tumors.

In yet another aspect, the present invention further provides a method for preventing or treating a TF-positive tumor in a subject, comprising administering to a subject in need thereof the ADC or the pharmaceutical acceptable salt or solvate thereof of the present invention, or administering to a subject in need thereof the anti-TF antibody or the antigen-binding fragment thereof of the present invention.

The TF-positive tumors suitable for the method and use of the present invention may be selected from: myeloma (e.g., multiple myeloma), acute lymphoblastic leukemia, chronic lymphoblastic leukemia, acute myelogenous leukemia (AML), non-Hodgkin's lymphoma, a central nervous system tumor, glioma, brain cancer, head and neck cancer (e.g., head and neck squamous cell carcinoma), gastrointestinal cancer, genitourinary system cancer, lung cancer (e.g., NSCLC), esophageal cancer, gastric cancer, hepatobiliary cancer, pancreatic cancer, colorectal cancer, rectal cancer, bladder cancer, kidney cancer, breast cancer (especially triple negative breast cancer), prostate cancer, endometrial cancer, ovarian cancer, cervical cancer, melanoma, sarcoma, and skin cancer.

The TF-positive tumors suitable for the method and use of the present invention may be a cancer at an early, intermediate or advanced stage, or may be a metastatic cancer. In addition, the TF-positive tumors suitable for the method and use of the present invention may be tumors that have previously undergone treatment to develop immune escape.

In some embodiments, the TF-positive tumor treated according to the method of the present invention is selected from: cervical cancer, pancreatic cancer, lung cancer, prostate cancer, bladder cancer, ovarian cancer, breast cancer, colorectal cancer, esophageal cancer, head and neck cancer, and gastric cancer, including primary or advanced or metastatic cancer. In yet another embodiment, the TF-positive tumor is cervical cancer. In another embodiment, the TF-positive tumor is non-small cell lung cancer. In yet another embodiment, the TF-positive tumor is esophageal squamous cell carcinoma. In yet another embodiment, the TF-positive tumor is breast cancer.

In some embodiments, the TF-positive tumor treated according to the method of the present invention has at least 10%, 20%, 30%, 40%, 50%, 60%, 70%, 75%, 80%, 85%, 90%, 95%, or 100% TF-positive cells. The expression level of tissue factors on tumor biopsies can be assessed by immunohistochemistry. High percentages of TF-positive cells have been detected on various cancer biopsies, for example, cervical cancer (100%), non-small cell lung cancer (34-88%), endometrial cancer (14-100%), prostate cancer (47-75%), ovarian cancer (75-100%), esophageal cancer (43-91%), and bladder cancer (78%).

Preferably, in some embodiments, the method according to the present invention is used for treating tumors with a high percentage of TF-positive cells, e.g., tumors with at least 25%, 50%, 75% or 100% TF-positive cells, e.g., tumors with TF-positive cells ranging from 25-50% to 75-100%, such as bladder cancer, lung cancer, pancreatic cancer, prostate cancer, ovarian cancer, and cervical cancer. In some embodiments, the method of the present invention is used for inducing tumor regression in the cancer.

In still other embodiments, the method according to the present invention may also be used for treating cancers with a percentage of TF-positive cells lower than 25% or 20%. In some embodiments, the method of the present invention is used for resulting in tumor growth inhibition in the cancer.

In yet another aspect, the present invention further provides a method for preventing or treating other diseases associated with elevated expression of cell membrane TF, comprising administering to a subject in need thereof the anti-TF antibody or the antigen-binding fragment thereof of the present invention, or administering to a subject in need thereof the ADC or the pharmaceutical acceptable salt or solvate thereof of the present invention. The diseases include, but are not limited to, for example, benign tumors, neurofibromas, hemangiomas; atherosclerosis; vascular diseases, such as retinopathy and macular degeneration; inflammatory diseases, such as rheumatoid arthritis, osteoarthritis and ankylosing spondylitis; and autoimmune inflammation, such as multiple sclerosis.

In any of the above embodiments of the method of the present invention, administration of the antibody or the binding fragment thereof according to the present invention and the ADC or the pharmaceutical acceptable salt or solvate thereof according to the present invention may comprise 1) a therapeutic measure, which cures, slows, alleviates symptoms of the diagnosed pathological condition or disease, and/or stops progression of the diagnosed pathological condition or disease; or 2) a prophylactic or preventative measure, which prevents and/or slows the progression of the pathological condition or disease. Thus, in the method of the present invention, the subject may be an individual already suffering from the disease, an individual susceptible to the disease, or an individual for whom the disease is to be prevented. The individual will benefit from the therapeutic measure or the prophylactic measure and exhibits an alleviation or amelioration in the occurrence, recurrence or progression of the disease, disorder, condition, and/or symptom as compared to an individual not receiving the treatment. In some embodiments, the present invention relates to the treatment of the disease or disorder. In other embodiments, the present invention relates to the prevention of the disease or disorder.

The antibody or the binding fragment thereof according to the present invention and the ADC or the pharmaceutical acceptable salt or solvate thereof according to the present invention, and optionally additional therapeutic agents in combination therewith can be administered by any suitable method, including parenteral, intratumoral and intranasal administration. Parenteral infusion includes intramuscular, intravenous, intra-arterial, intraperitoneal or subcutaneous administration. Various administration schedules are encompassed herein, including, but not limited to, single administration or multiple administrations at multiple time points, bolus injection, and pulse infusion.

In order to prevent or treat diseases, the appropriate dosage of the antibody or the binding fragment thereof according to the present invention and the ADC or the pharmaceutical acceptable salt or solvate thereof according to the present invention (when used alone or in combination with one or more additional therapeutic agents) will depend on types of diseases to be treated, particular types of drugs used, severity and progression of the disease, purpose of administration (prophylactic or therapeutic) of the drug, previous treatments, clinical histories of patients, responses to the antibody, and the discretion of an attending physician. The antibody or the binding fragment thereof according to the present invention and the ADC or the pharmaceutical acceptable salt or solvate thereof according to the present invention can be administered to a patient in a single treatment or over a series of treatments.

In some embodiments, the subject is receiving or has received additional therapies, such as chemotherapy and/or radiotherapy, or other immunotherapies, prior to receiving therapies according to the method of the present invention.

In any of the above embodiments of the method and use of the present invention, the composition, the multispecific antibody or the immunoconjugate/fusion or the combination product of the present invention can be administered in place of the antibody or the antigen-binding moiety thereof of the present invention or in place of the ADC or the pharmaceutical acceptable salt or solvate thereof of the present invention. Alternatively, in the methods, in addition to administering the antibody or the antigen-binding moiety thereof of the present invention or the ADC or the pharmaceutical acceptable salt or solvate thereof of the present invention, the composition, the multispecific antibody or the immunoconjugate/fusion or the combination product of the present invention can be further administered.

In some embodiments, the present invention further provides use of the ADC or the pharmaceutical acceptable salt or solvate thereof of the present invention in the preparation of a drug for use in the above therapeutic and prophylactic methods. In other embodiments, the present invention further provides use of the antibody or the antigen-binding fragment thereof, the composition, the immunoconjugate/fusion, the multispecific antibody of the present invention in the preparation of a drug for use in the above therapeutic and prophylactic methods.

Any or all of the features described above and throughout the present application may be combined in various embodiments of the present invention. The present invention will be further illustrated with reference to the following examples. However, it should be understood that the examples are for illustrative purposes, and should not be construed as constituting any limitation.

### Examples

### Example I. Preparation and Characterization of Humanized Anti-TF Antibodies

### General methods

### Recombinant expression of cytokine antigens

The amino acid sequence of the human tissue factor is referred to P13726 in the Uniprot protein database, with the extracellular segment of aa 33-aa 251; and the amino acid sequence of the cynomolgus monkey tissue factor is referred to A0A2K5VXA0 in the Uniprot protein database, with the extracellular segment of aa 33-aa 252.

The amino acid sequences of the above extracellular segments were codon-optimized and synthesized in an expression vector pTT5-His by General Biosystems (Anhui) Co., Ltd. HEK293-EBNA cells were transiently transfected by PEImax (Polysciences, 24765-1), and after 7 days of expression, human TF-His (h.TF-His) and cynomolgus monkey TF-His (c.TF-His) antigens were obtained by purification on a nickel column. The recombinantly expressed and purified TF antigens were used in the subsequent examples as an immunogen or in ELISA assays.

### Antigenic protein-based ELISA

The ELISA plate was coated overnight with TF antigen (h.TF-His or c.TF-His antigen) in 50 mM carbonate coating buffer (CBS coating solution) at a coating concentration of 1 µg/mL in a volume of 100 µL at 4 °C. The next day, the coating solution was removed, each well was washed once with 300 µL of PBS, and 100 µL of 2% BSA (Biotopped, A6020) in PBS was added to each well for blocking at 37 °C for 2 h. The test sample was subjected to a gradient dilution with 2% BSA in PBS to establish 11 concentration points. 100 µL of each of the samples obtained by the gradient dilution was added to the plate, and the plate was incubated at 37 °C for 2 h. The supernatant was removed, and each well was washed three times with 300 µL of PBST. 100 µL of HRP-conjugated secondary antibody in 2% BSA was added to each well, and the plate was incubated at 37 °C for 1 h. For the murine antibody from hybridomas, an HRP-labeled anti-mouse secondary antibody (Jackson, 115-035-003) was used; and for the humanized antibody, an HRP-labeled anti-human secondary antibody (Jackson, 109-035-088) was used. At the end of the incubation, the secondary antibody was removed, each well was washed five times with 300 µL of PBST, and 100 µL of TMB substrate (Huzhou InnoReagents Co., Ltd., TMB-S-004) was added to each well for color development for 5-20 min. 50 µL of 2 N HCl was added to each well to stop the reaction, and OD450nm values were read using a microplate reader (manufacturer: MD, model: SpectraMax) and imported into GraphPad software for fitting and plotting to determine the binding of the samples to TF antigenic proteins.

### Cell-based ELISA

TF antigen-positive cells (MDA-MB-231 or NCI-H358 cells) were seeded into a 96-well plate at 5 × 10⁴ cells/well one day prior to the experiment. On the day of the experiment, the culture supernatant was removed, each well was washed once with 300 µL of PBS, and 100 µL of 4% paraformaldehyde was added to each well for immobilization at room temperature for 20 min. The paraformaldehyde was removed, and each well was washed twice with 300 µL of PBS, and 100 µL of 2% BSA in PBS was added to each well for blocking at 37 °C for 2 h. After the blocking was completed, 30 µL of the test sample (hybridoma supernatant or purified antibody) was added to each well and the plate was incubated at 37 °C for 2 h. The liquid was removed, and each well was washed three times with 300 µL of PBST. 100 µL of URP-conjugated secondary antibody in 2% BSA was added to each well, and the plate was incubated at 37 °C for 1 h. In the case that the test sample was a hybridoma or a murine antibody, an HRP-labeled anti-mouse secondary antibody (Jackson, 115-035-003) was used; and in the case that the test sample was a humanized antibody, an HRP-labeled anti-human secondary antibody (Jackson, 109-035-088) was used. The secondary antibody was removed, each well was washed five times with 300 µL of PBST, and 100 µL of TMB substrate (Huzhou InnoReagents Co., Ltd., TMB-S-004) was added to each well for color development for 5-20 min. 50 µL of 2 N HCl was added to each well to stop the reaction, and OD450nm values were read using a microplate reader (manufacturer: MD, model: SpectraMax) to determine the binding of the samples to TF-positive cells.

### Antibody purity assay by SEC-HPLC

The recombinantly expressed and purified antibody was subjected to an antibody purity assay by SEC-HPLC. The assay method was performed as follows:
instrument: Waters Alliance e2695 HPLC;
chromatographic column: Thermo MabPac SEC-1, 5 µm, 7.8 × 300 mm;
mobile phase: 61 mmol/L Na₂HPO₄, 39 mmol/L NaH₂PO₄, 200 mmol/L NaCl, 5% IPA; and
instrument parameters: sample chamber temperature: 8 °C; column temperature: 30 °C; flow rate: 0.5 mL/min; sample injection volume: 20 µg; detection wavelength: 280 nm; and isocratic run: 30 min.

### Antibody hydrophilicity/hydrophobicity assay by HIC

The recombinantly expressed and purified antibody was subjected to an antibody hydrophilicity/hydrophobicity assay by HIC. The assay method was performed as follows:
The hydrophilicity/hydrophobicity assay was performed on an Agilent HPLC instrument using a hydrophobic chromatography column from Tosoh Corporation (TOSOH Tskgel Buty-NPR (2.5), 4.6 × 100) with mobile phase A of 1.5 M (NH₄)₂SO₄ and mobile phase B of 25 mM Na₂HPO₄ (pH = 7.0) + 25% IPA. The instrument parameters were set as follows: sample chamber temperature: 8 °C; column temperature: 30 °C; flow rate: 0.5 mL/min; and detection wavelength: 280 nm. The test sample was diluted to the final concentration of 1 mg/mL with the mobile phase A, and 20 µL of the dilution was injected for gradient elution. The elution gradient was as follows:

| | | | | | | |
|---|---|---|---|---|---|---|
| T/min | 0 | 2 | 39 | 41 | 42 | 45 |
| A% | 95 | 95 | 0 | 0 | 95 | 95 |
| B% | 5 | 5 | 100 | 100 | 5 | 5 |

### Example 1. Preparation of Anti-TF Murine Monoclonal Antibodies

Three female mice aged 5-6 weeks were selected from each of the 3 strains, i.e., Balb/C, Kunming (KM), and CD01. Human TF-his protein and cynomolgus monkey TF-his protein were used for immunization. The immunization regimen is shown in Table 1.

**Table 1. Mouse immunization regimen**

| Immunization process | Immunizing antigen* |
|---|---|
| Primary | 100 ug h.TF1-His++10 ug CpG+CFA |
| Boost 1st | 100 ug h.TF1-His+IFA |
| Boost 2nd | 50 ug h.TF1-His+IFA |
| Final Boost | 2 ug h.TF1-His+2 ug c.TF1-His |

| | |
|---|---|
| *: CFA: complete Freund's adjuvant; IFA: incomplete Freund's adjuvant; and CpG: CpG oligodeoxynucleotide adjuvant. | |

After 2 boosts, serum titers of immunized animals were determined in ELISA plates coated with h.TF-His and c.TF-His antigens, respectively, by the antigenic protein-based ELISA assay, as described in the general methods. Based on the results of the assay, CD1 immunized mice and Balb/c immunized mice were selected for hybridoma fusion taking into account the affinity to human and monkey TFs, and a final boost was performed prior to the hybridoma fusion.

Cell suspensions were prepared from the spleens and lymph nodes of the mice and mixed with SP2/0 mouse myeloma cells at a 1:1 ratio. The cells were resuspended in a cell electrofusion buffer and subjected to an electrofusion reaction using a BTX-ECM2001 cell electrofusion apparatus. After the electrofusion reaction, the cells were resuspended in a complete fusion medium (RPMI160 + 15% FBS + 1 × HAT), added to a 96-well cell culture plate at 20,000 to 25,000 cells/well, and cultured at 37 °C with 5% CO₂.

After 7 days of culture, ELISA rescreening was sequentially performed by using ELISA plates coated with human TF-his and cynomolgus monkey TF-his; and cell ELISA rescreening was performed using TF-positive tumor cells (MDA-MB-231 or NCI-H358). Positive parent clones were selected for subcloning.

75 cells were taken from each parent clone and added to a 96-well plate for subclone culture. Then, the subclone supernatant was taken and sequentially subjected to ELISA and cell ELISA screenings by coating a human TF-his protein, a cynomolgus monkey TF-his protein, and a rat TF-his protein, and NCI-H358 cells, respectively. Based on the screening results, 119 positive clones were selected and subjected to amplification culture in a serum-free medium. Supernatants of the cultured hybridoma monoclones were collected and purified using packing of antibody purification media ProA (GE, Mabselect XL) to obtain murine antibodies.

Purified murine antibodies were sequentially evaluated by ELISA and cell ELISA by coating a human TF-his protein, a cynomolgus monkey TF-his protein, and NCI-H358 cells, respectively. The experimental procedures were as described in the general methods, wherein the murine antibody was diluted in a 3-fold gradient starting at 10 µg/mL to establish 11 concentration points. Based on the assay results, 13 hybridoma monoclones with high affinity were selected for antibody sequence determination and humanization.

### Example 2. Sequence determination and humanization of anti-TF murine monoclonal antibodies

About 1 × 10⁵ hybridoma cell candidates were collected. RNA was extracted by Trizol (Invitrogen, 15596026), and then cDNA was obtained by reverse transcription through PolyA using PrimeScript RT reagent kit (TAKARA, RR047A). The forward primers were designed upstream of the heavy chain and the light chain, and the reverse primers were designed in the CH1 region of the heavy chain and the CL region of the light chain. After the product was amplified by PCR, the fragments were recovered using an agarose gel extraction kit, and sent to Tsingke Biotechnology Co., Ltd. for sequencing to obtain murine antibody sequences.

The murine antigen sequences were humanized by CDR grafting. Firstly, a human germline sequence with the highest homology to the original murine sequence was found by a conventional BLAST method and used as a template; CDRs of the murine antibody were grafted to the human template to construct a chimera; FR amino acids capable of retaining the original conformation of the murine antibody were analyzed according to the structure, and the corresponding amino acids in the chimera were back-mutated to the murine amino acids to keep the original affinity; and the constructed humanized antibody was subjected to calculation and immunogenicity analysis to find a high-immunogenicity fragment, which was used for replacing a low-immunogenicity fragment. The sequences of 17 humanized antibodies were obtained.

### Example 3. Expression and Purification of Humanized Antibodies

The amino acid sequences of the heavy and light variable regions of each of the above humanized antibodies and the reference antibody (Benchmark (BM), Tisotumab from Genmab, having sequences derived from SEQ ID NO: 5 (VH) and SEQ ID NO: 45 (VL) of the patent CN103119065B) were submitted to General Biosystems for gene synthesis, codon-optimized, and then constructed on a PTT5 vector. After the synthesis, the plasmid was transfected into HEK293E cells by PEImax and expressed for about 7 days, and the supernatant was collected by centrifugation. The supernatant was purified using MabSelect SuRe ProActive affinity packing (GE, cat # 17547401). The expressed reference antibody had the same human IgG1 heavy chain constant region (SEQ ID NO: 106) and human Kappa light chain constant region (SEQ ID NO: 105) as the humanized antibody. The purified antibodies were all exchanged into PBS buffers by ultrafiltration, determined for the concentration, and stored at -20 °C.

### Example 4. Evaluation of Affinity of Humanized Antibodies by ELISA

### Antigenic protein-based ELISA assay

The assay was performed as described in the general methods. Briefly, an ELISA plate coated with a human TF-his protein or a cynomolgus monkey TF-his protein was blocked with 2% BSA at 37 °C for 2 h, and each of the antibodies obtained by the gradient dilution (starting at 10 µg/mL, 3-fold graded dilution, 11 concentration points) was added. The plate was incubated at 37 °C for 2 h. Then, an anti-human secondary antibody (Peroxidase-AffiniPure Goat Anti-Human IgG (H+L), Jackson, 109-035-088) was added, the plate was incubated at 37 °C for 1 h, and a TMB substrate (Huzhou InnoReagents Co., Ltd., TMB-S-004) was added for color development. After the color development was stopped, the absorbance values at 450 nm were read on a machine and the curves were fitted using GraphPad software.

### Cell-based ELISA assay

The assay was performed as described in the general methods. Briefly, TF antigen-positive NCI-H358 cells were digested with trypsin, collected, seeded into a 96-well plate at 5 × 10⁴ cells/well and incubated overnight, and then immobilized with 4% paraformaldehyde. After the paraformaldehyde was removed and the cells were washed with PBS, the plate was blocked with 2% BSA at 37 °C for 2 h. Each of the antibodies obtained by the gradient dilution (starting at 10 µg/mL, 3-fold graded dilution, 11 concentration points) was added. The plate was incubated at 37 °C for 2 h. Then, an anti-human secondary antibody (Peroxidase-AffiniPure Goat Anti-Human IgG (H+L), Jackson, 109-035-088) was added, the plate was incubated at 37 °C for 1 h, and a TMB substrate (Huzhou InnoReagents Co., Ltd., TMB-S-004) was added for color development. After the color development was stopped, the absorbance values at 450 nm were read on a machine and the curves were fitted using GraphPad software.

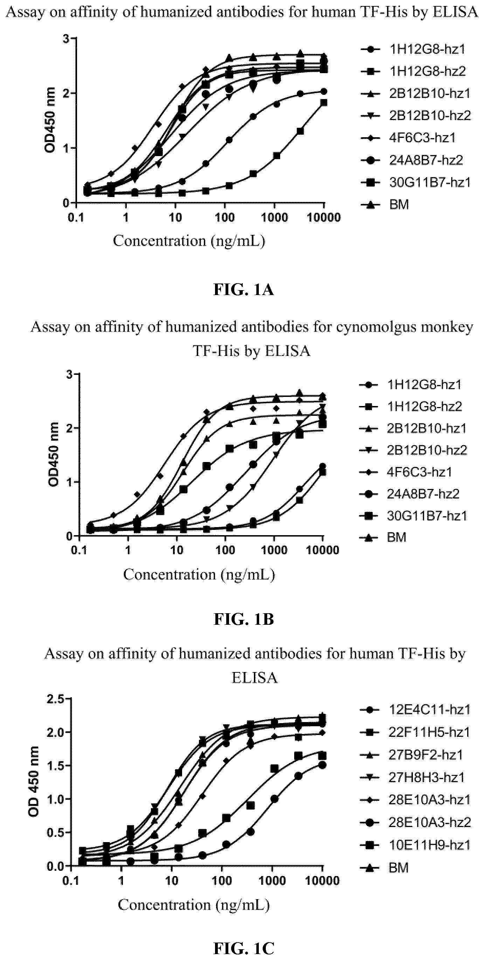

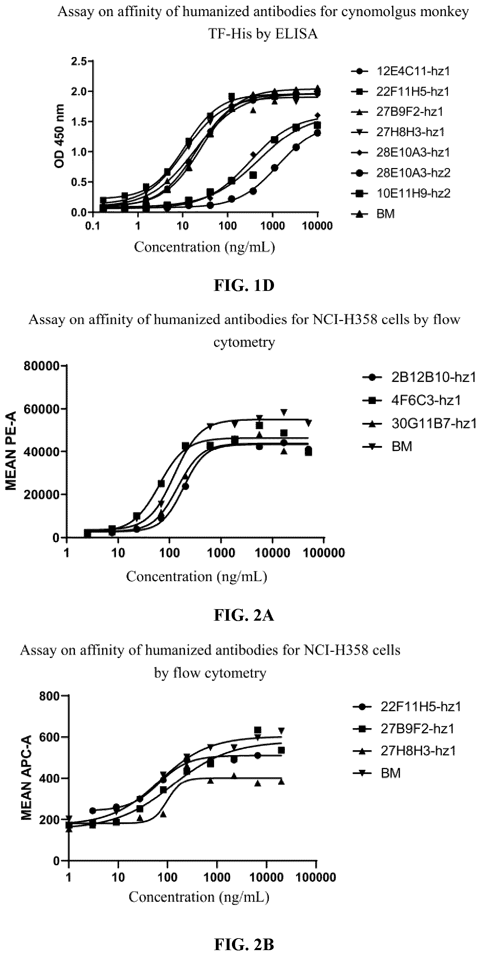

The experimental results are shown in Table 2 and FIG. 1. Humanized antibodies, 2B12B10-hz1, 4F6C3-hz1, 30G11B7-hz1, 22F11H5-hz1, 27B9F2-hz1, and 27H8H3-hz1, which showed good binding activity to human/cynomolgus TF-his and TF-positive cells, were selected for subsequent evaluation.

**Table 2. ELISA assay results for humanized antibodies**

| No. | Antibody No. | h.TF-His | c.TF-His | NCI-H358 |
|---|---|---|---|---|
| | | EC50 (ng/mL) | EC50 (ng/mL) | EC50(ng/mL) |
| 1 | 1H12G8-hz1 | 116.4 | 4105 | 65.61 |
| 2 | 1H12G8-hz2 | 3785 | 10948 | 1537 |
| 3 | 2B12B10-hz1 | 6.724 | 13.63 | 3.877 |
| 4 | 2B12B10-hz2 | 17.06 | 917.1 | 18.27 |
| 5 | 4F6C3-hz1 | 3.571 | 5.679 | 3.007 |
| 6 | 24A8B7-hz1 | 5.478 | 19.54 | 5.862 |
| 7 | 24A8B7-hz2 | 8.051 | 259.1 | 4.417 |
| 8 | 30G11B7-hz1 | 7.912 | 20.79 | 4.727 |
| 9 | 9H4B4-hz1 | 19.52 | 18.64 | Not tested |
| 10 | 10E11H9-hz1 | 1604 | 2593 | Not tested |
| 11 | 12E4C11-hz1 | 17.3 | 20.66 | Weak binding |
| 12 | 22F11H5-hz1 | 8.577 | 11.41 | 11.95 |
| 13 | 27B9F2-hz1 | 14.55 | 18.9 | 19.56 |
| 14 | 27H8H3-hz1 | 8.147 | 11.37 | 34.79 |
| 15 | 28E10A3-hz1 | 40.36 | 313.4 | Weak binding |
| 16 | 28E10A3-hz2 | 804.2 | 1326 | Weak binding |
| 17 | 10E11H9-hz2 | 320.4 | 478.2 | Weak binding |
| | Benchmark | 14.43±7.32 (n=2) | 18.45±7.71 (n=2) | 21.62±2.00 (n=2) |

### Example 5. Quality Identification of Humanized Antibodies

The above humanized antibodies, which have been subjected to recombinant expression and protein A affinity purification, were identified for purity by SEC-HPLC as described in the general methods, and assayed for hydrophilicity/hydrophobicity by HIC. The SEC and HIC assay results are shown in Table 3. The SEC results show that the selected 6 humanized antibodies all have high purity more than 90%; and the HIC results show that the selected 6 humanized antibodies are weakly bound to a hydrophobic chromatographic column, and have short retention time and good hydrophilicity.

**Table 3. Quality Identification of Humanized Antibodies**

| Antibody No. | SEC | | | HIC (min) |
|---|---|---|---|---|
| | HMW% | Main peak% | LMW % | |
| 2B12B10-hz1 | 2.56 | 97.44 | 0 | 12.56 |
| 4F6C3-hz1 | 1.13 | 98.87 | 0 | 11.86 |
| 30G11B7-hz1 | 1.55 | 98.45 | 0 | 12.02 |
| 22F11H5-hz1 | 3.76 | 96.24 | 0 | 10.89 |
| 27B9F2-hz1 | 7.63 | 92.37 | 0 | 10.13 |
| 27H8H3-hz1 | 0.93 | 99.07 | 0 | 10.43 |

### Example 6. Evaluation of Flow Affinity of Humanized Antibodies

A TF low/medium-expressing cell line NCI-H358 (source: Nanjing Cobioer, CBP60136) and a TF high-expressing cell line KYSE520 (source: Nanjing Cobioer, CBP60658) were digested with trypsin, collected by centrifugation, and washed three times with pre-cooled PBS. The cells were resuspended in 1% BSA and seeded into a 96-well sharp-bottom plate at 3 × 10⁵ cells/well. Each of the antibodies obtained by the gradient dilution was added, and the plate was incubated at 4 °C for 1 h. The cells were washed three times with pre-cooled PBS. 1 µL of anti-human fluorescent secondary antibody (APC anti-human IgG Fc Antibody, Biolegend, 410712) was added to each well, and the plate was incubated at 4 °C for 0.5 h. The cells were washed three times with pre-cooled PBS, resuspended, and detected by a flow cytometer (Sony, LE-SA3800GA). The data were imported into GraphPad software for curve fitting.

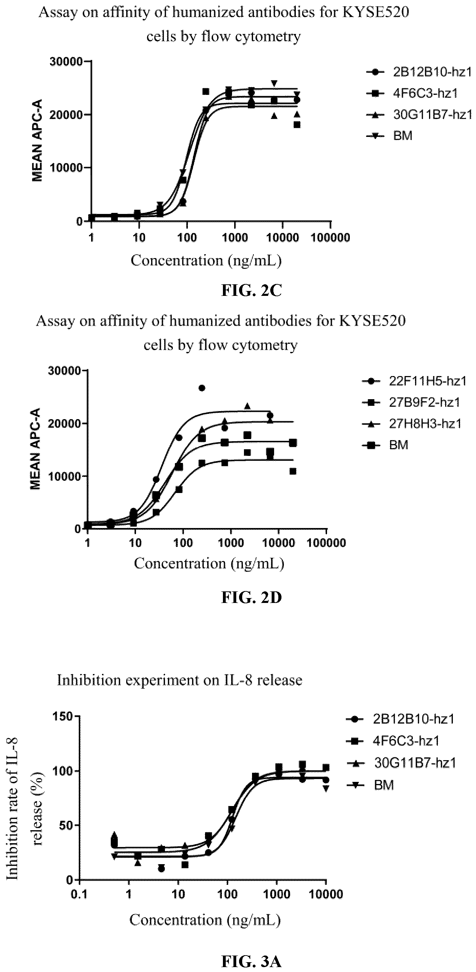

The results are shown in Table 4 and FIG. 2, which show that 6 candidate humanized antibodies all have strong binding to tumor cells.

**Table 4. Flow cytometry results for humanized antibodies**

| No. | Antibody No. | NCI-H358 (EC50, ng/mL) | KYSE520 (EC50, ng/mL) |
|---|---|---|---|
| 1 | 2B12B10-hz1 | 187.4 | 140.3 |
| 2 | 4F6C3-hz1 | 63.48 | 96.69 |
| 3 | 30G11B7-hz1 | 145.1 | 138.3 |
| 4 | 22F11H5-hz1 | 67.09 | 35.7 |
| 5 | 27B9F2-hz1 | 126.5 | 68.67 |
| 6 | 27H8H3-hz1 | 99.06 | 59.43 |
| | BM | 97.81±39.73 (n=2) | 77.48±50.24 (n=2) |

### Example 7. Evaluation of Dynamic Affinity of Humanized Antibodies

The dynamic affinity of the humanized antibodies was determined by ForteBio. The experimental procedures were as follows: a ProA sensor (18-5010, Octet) was taken out and pre-wetted with a PBST diluent (pH 7.4) for 10 min. The antibodies to be immobilized were diluted to 5 µg/mL. The antigens h.TF-His and c.TF-His were subjected to a 2-fold dilution starting at 75 nM to obtain 4 concentration points, and 0 concentration point was set. The program was set up, the sensor plate and sample plate were put in, then the program started. The sensor was regenerated with 20 mM glycine solution (pH 1.7). The data were analyzed using Octet analysis software to derive a result graph. The results are shown in Table 5, which shows that 6 candidate humanized antibodies all have high affinity for human and monkey TFs.

**Table 5. Dynamic affinity assay results for humanized antibodies**

| Antibody No. | h.TF-his | | | c.TF-his | | |
|---|---|---|---|---|---|---|
| | KD (M) | ka (1/Ms) | kdis (1/s) | KD (M) | ka (1/Ms) | kdis (1/s) |
| 2B12B10-hz1 | 1.38E-08 | 2.09E+05 | 2.89E-03 | 3.38E-08 | 3.44E+05 | 1.16E-02 |
| 4F6C3-hz1 | 1.77E-08 | 1.81E+05 | 3.20E-03 | 2.18E-08 | 3.55E+05 | 7.72E-03 |
| 30G11B7-hz1 | 2.58E-08 | 1.97E+05 | 5.06E-03 | 1.46E-07 | 3.64E+05 | 5.30E-02 |
| 22F11H5-hz1 | 9.80E-09 | 3.02E+05 | 2.96E-03 | 1.27E-08 | 3.24E+05 | 4.11E-03 |
| 27B9F2-hz1 | 6.32E-09 | 1.85E+05 | 1.17E-03 | 2.97E-08 | 1.72E+05 | 5.09E-03 |
| 27H8H3-hz1 | 4.25E-08 | 2.73E+05 | 1.16E-02 | 4.25E-08 | 2.95E+05 | 1.25E-02 |
| BM | 1.42E-08 | 1.66E+05 | 2.34E-03 | 2.05E-08 | 1.79E+05 | 3.66E-03 |

### Example 8. Evaluation of Endocytic Activity of Humanized Antibodies

The endocytic activity of the humanized monoclonal antibodies was assayed by flow cytometry (FACS). The experimental procedures were as follows: NCI-H358 cells were digested with trypsin, collected by centrifugation, and incubated at 4 °C for 1 h with the test antibody at a concentration of 10 µg/mL. The cells were washed three times with PBS, resuspended in RPMI1640 + 10% FBS, and divided into 3 aliquots, which were incubated at 37 °C for 0 h, 2 h, and 4 h, respectively. The cells were washed three times with PBS, and 1 µL of anti-human fluorescent secondary antibody (APC anti-human IgG Fc Antibody, Biolegend, 410712) was added. The cells were incubated at 4 °C for 0.5 h, washed three times with PBS, resuspended, and assayed on a machine. Isotype control negative antibody HLE IgG1 was used as a negative control. The endocytosis rate was calculated according to the following formula: endocytosis rate (%) = [1-(average fluorescence value of test sample at the time point - average fluorescence value of negative control sample at the time point) / (average fluorescence value of test sample at 0 h-average fluorescence value of negative control sample at 0 h)] × 100.

The experimental results are shown in Table 6, which shows that the endocytosis rates of 2B12B10-hz1, 4F6C3-hz1, 30G11B7-hz1, 22F11H5-hz1, and 27B9F2-hz1 are substantially comparable to that of the reference antibody, and the endocytosis rate of 27H8H3-hz1 is superior to that of the reference antibody.

**Table 6. Endocytosis assay results for humanized antibodies**

| Endocytosis rate (%) | | | | | | | |
|---|---|---|---|---|---|---|---|
| Time | 2B12B10-hz1 | 4F6C3-hz1 | 30G11B7-hz1 | 22F11H5-hz1 | 27B9F2-hz1 | 27H8H3-hz1 | BM |
| 2h | 34.32 | 17.03 | 19.77 | 34.32 | 19.35 | 49.17 | 20.26 |
| 4h | 49.63 | 47.67 | 46.10 | 56.07 | 54.95 | 73.37 | 56.17 |

### Example 9. Evaluation of Signaling Pathway Blocking Activity of Humanized Antibodies

The binding of FVIIa to the tissue factor on the surface of TF-positive tumor cells MDA-MB-231 will activate the TF downstream signaling pathway, resulting in the release of IL-8. Therefore, the blocking activity of the anti-TF humanized antibodies against the TF signaling pathway can be reflected by detecting the release of IL-8.

The experimental procedures were as follows: FVIIa (40 nM, Haematologic Technologies Inc., HCVIIA-0031) and each of the anti-TF humanized antibodies obtained by gradient dilution (starting at 60 µg/mL, 3-fold gradient dilution, 11 concentration points) were incubated with MDA-MB-231 cells (5 × 10⁴ cells/well, Nanjing Cobioer, CBP60382) for 5 h. The supernatants were collected and detected for the release of IL-8 by using an IL-8 detection kit (MABTECH, 3114-1H-20). IL-8 detection procedures were as follows: the night prior to the experiment, a 96-well plate was coated with the capture antibody MT8H6 in PBS at 200 ng/well and incubated at 4 °C overnight. The coating solution was removed, the plate was washed once with 300 µL of PBST, and 100 µL of 2% BSA in PBS was added to each well of the 96-well plate for blocking at 37 °C for 2 h. 50 µL of cell culture supernatant was directly added and incubated at 37 °C for 2 h. The supernatant was removed, each well was washed three times with 300 µL of PBST, 100 µL (1 µg/mL) of MT8F19-biotin secondary antibody (in 2% BSA) was added to each well, and the plate was incubated at 37 °C for 1 h. The supernatant was removed, each well was washed four times with 300 µL of PBST, 100 µL of peroxidase-conjugated streptavidin (1:10,000 dilution) was added to each well, and the plate was incubated at 37 °C for 1 h. The supernatant was removed, each well was washed five times with 300 µL of PBST, and 100 µL of TMB substrate was added to each well for color development for 5-20 min. 50 µL of 2 N HCl was added to each well to stop the reaction, and OD450mm values were read using a microplate reader. The results were imported into GraphPad for plotting, and the inhibition rate and IC50 were calculated.

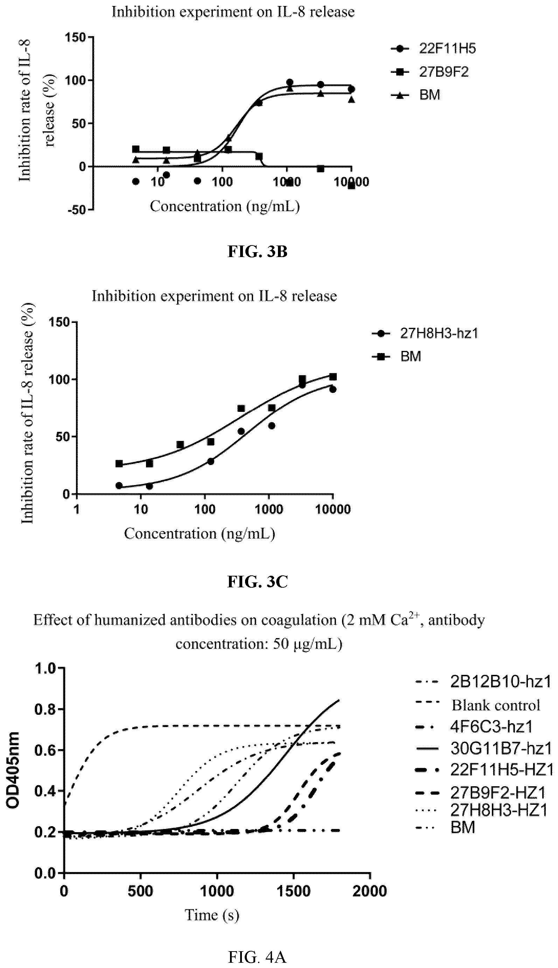

The experimental results are shown in Table 7 and FIG. 3, which show that except for 27B9F2-hz1, 5 humanized antibodies all have strong inhibitory effects on the TF signaling pathway.

**Table 7. IL-8 release assay results for blocking activity of humanized antibodies**

| Antibody No. | IC50 (ng/mL) | Maximum inhibition rate (%) |
|---|---|---|
| 2B12B10-hz1 | 128.1 | 94.06 |
| 4F6C3-hz1 | 120.9 | 100 |
| 30G11B7-hz1 | 129.4 | 100 |
| 22F11H5-hz1 | 179.7 | 94.3 |
| 27B9F2-hz1 | Weak | Weak |
| 27H8H3-hz1 | 463.3 | 100 |
| BM | 225.37±118.10 (n=3) | 100 |

### Example 10. Evaluation of Effect of Humanized Antibodies on Coagulation

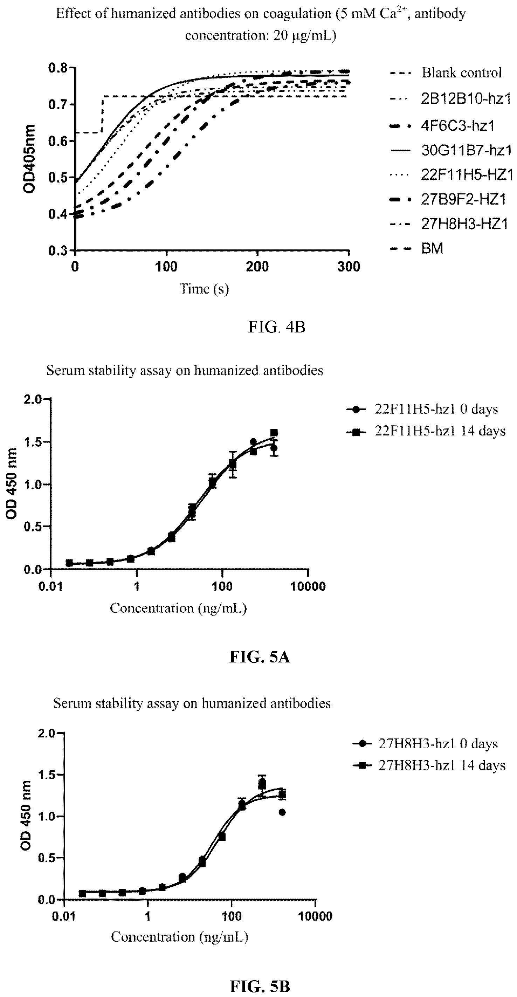

Each of the anti-TF humanized antibodies was diluted with a HEPES solution containing CaCl₂ and incubated with 50,000 TF-positive MDA-MB-231 cells at 37 °C for 30 min. Then, 50 µL of human plasma was added, the mixture was rapidly mixed uniformly, and absorbance values at 405 nm were continuously read immediately for the calculation of the anticoagulation effect on coagulation initiated by TF on the cell surface. The results were imported into GraphPad for plotting. A total of two experiments were performed. In the first experiment, the CaCl₂ concentration was 2 mM, and the final antibody concentration was 50 µg/mL; in the second experiment, the CaCl₂ concentration was 5 mM and the final antibody concentration was 20 µg/mL. The experimental results are shown in FIG. 4 (FIG. 4A: 2 mM Ca²⁺, antibody concentration: 50 µg/mL; FIG. 4B: 5 mM Ca²⁺, antibody concentration: 20 µg/mL) and in Table 8 below. Combining the results of the two experiments, 27H8H3-hz1 and 2B12B10-hz1 have the least effect on human coagulation among the test antibodies and are superior to the reference antibody.

**Table 8. Effect of humanized antibodies on coagulation time V50**

| Antibody No. | First experiment 2 mM Ca²⁺, 50 µg/mL antibody V50 (s) | Second experiment 5 mM Ca²⁺, 20 µg/mL antibody V50 (s) |
|---|---|---|
| 2B12B10-hz1 | 882.5 | 24.25 |
| *4F6C3-hz1 | - | 94.69 |
| 30G11B7-hz1 | 1446 | 29.16 |
| 22F11H5-hz1 | 1643 | 49.36 |
| 27B9F2-hz1 | 1542 | 115.2 |
| 27H8H3-hz1 | 750.5 | 26.20 |
| BM | 1141 | 74.88 |
| Blank control | 19.96 | ~29.96 |

| | | |
|---|---|---|
| *: in the presence of 2 mM Ca²⁺ and 50 µg/mL antibody 4F6C3-hz1, essentially no coagulation occurred within 2000 s. | | |

### Example 11. Stability Evaluation of Humanized Antibodies

### Accelerated stability evaluation of humanized antibodies

Each of the anti-TF humanized antibody samples was exchanged by ultrafiltration into PBS (pH = 7.4) at a concentration of 5 mg/mL, sterilized by filtration, left to stand at 37 °C for 0 days, 3 days, 7 days, and 14 days, and subjected to 4 and 8 repeated freeze-thawing cycles. Subsequently, the samples were determined for SEC-HPLC purity and CE-SDS purity as described in the general methods.

The experimental results show that the humanized antibodies 22F11H5-hz1 and 27H8H3-hz1 tested under accelerated and repeated freeze-thawing conditions have good stability. After accelerated stability and repeated freeze-thaw tests, the SEC purity of the two humanized antibodies remained above 95% or more; the CE-SDS non-reduction purity remained above 87%, and the CE-SDS reduction purity remained above 95%.

### Determination of Tm values of humanized antibodies

Tm values of the humanized antibodies were determined using DSF to reflect the thermal stability of the antibodies. The experimental procedures were as follows: the test antibody samples were diluted to 1 mg/mL with PBS. SYPRO Orange dye (Thermo # 56651) was subjected to a 40-fold dilution with ddH₂O. The reaction system was 12.5 µL of sample + 2.5 µL of 40 × dye + 5 µL of ddH₂O. The plate was sealed with a film and subjected to transient centrifugation. Q-PCR detection was performed, with Q-PCR parameters set as follows: Target (ROX), procedure (25 °C, 3 min; 1% rate, 95 °C; 95 °C, 2 min).

The results show that 5 candidate humanized antibodies 2B12B10-hz1, 4F6C3-hz1, 30G11B7-hz1, 22F11H5-hz1, and 27H8H3-hz1 all have high Tm values (all higher than 70 °C), suggesting that these candidate humanized antibodies have better thermal stability.

### Example 12. Serum Stability Evaluation of Humanized Antibodies

Anti-TF humanized antibodies were diluted to 20 µg/mL with human serum, left to stand at 37 °C for 14 days, and then detected for the change in affinity by antigenic protein-based ELISA. The experimental procedures of the ELISA method were described in the general methods. The experimental results are shown in FIGs. 5A and 5B, which show that 22F11H5-hz1 and 27H8H3-hz1 show no significant change in affinity after 14 days of storage in human serum.

### Example 13. Assay on PK of Humanized Antibodies in Rats

The test samples were administered to male rats (Chengdu Dossy) by injection at the tail vein at a dose of 10 mg/kg, with 3 rats in each group. Serum samples were collected 30 min, 1 h, 2 h, 6 h, 24 h, 48 h, 96 h, 168 h, and 336 h after the administration. The corresponding antibodies were diluted (starting at 1 µg/mL, 2-fold gradient dilution) with 2% BSA containing negative rat serum to obtain 11 concentration points, which were used as standard curve samples. The plasma concentration was determined by antigenic protein-based ELISA, as described in the general methods. The experimental results show that the humanized antibodies 22F11H5-hz1 and 27H8H3-hz1 have a half-life of about 120 h or more in rats.

Based on the above antibody characterization, 4 candidate humanized antibodies, 2B12B10-hz1, 30G11B7-hz1, 22F11H5-hz1, and 27H8H3-hz1, were identified for subsequent ADC construction and performance characterization. The sequences of 4 selected antibodies are shown in the sequence listing.

### Example II. Preparation and Characterization of Antibody-Drug Conjugates (ADCs)

### Example 1. Preparation of Antibody-Drug Conjugates (ADCs)

### Example 1.1. Synthesis of intermediates used in the synthesis of "drug-linker compounds"

### Example 1.1.1: synthesis of (S)-7-ethyl-7-hydroxy-14-(3-hydroxypropyl)-10,13-dihydro-11H-[1,3]dioxolo[4,5-g]pyrano[3',4':6,7]indolizino[1,2-b]quinoline-8,11(7H)-dione (A1)

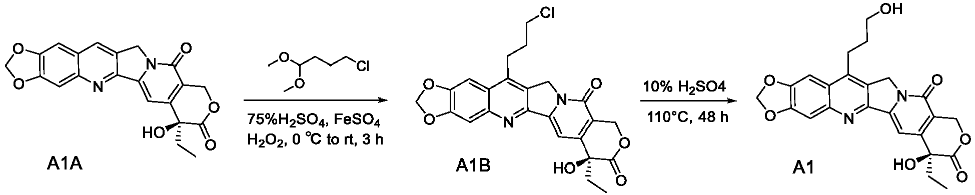

### Step 1: synthesis of (S)-7-ethyl-7-hydroxy-14-(3-chloropropyl)-10,13-dihydro-11H-[1,3]dioxolo[4,5-g]pyrano[3',4':6,7]indolizino[1,2-b]quinoline-8,11(7H)-dione (A1B)

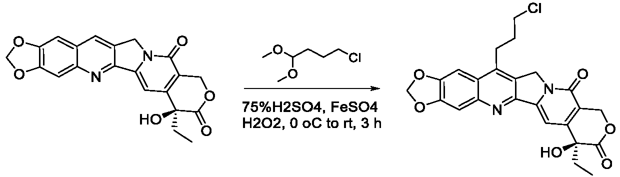

To a 75% sulfuric acid solution (5 mL) of the compound (*S*)-7-ethyl-7-hydroxy-10,13-dihydro-11*H*-[1,3]dioxolo[4,5-*g*]pyrano[3',4':6,7]indolizino[1,2-*b*]quinoline-8,11(7*H*)-dione (A1A, Cas No.: 151636-76-9, 500 mg) were added ferrous sulfate heptahydrate (570 mg of ferrous sulfate heptahydrate dissolved in 1 mL of water) and 4,4-dimethoxychlorobutane (3.89 g) in an ice bath, and the mixture was stirred for three minutes, followed by the dropwise addition of hydrogen peroxide (29%, 2.5 mL). The reaction solution was stirred at 0 °C for 5 min, heated to room temperature, and stirred for 3 h. The reaction solution was diluted with water (50 mL) and extracted with ethyl acetate (80 mL × 2). The organic phase was washed with saturated brine, dried over anhydrous sodium sulfate, and concentrated to give a crude product. The crude product was further purified using a C18 column (acetonitrile/0.05% aqueous formic acid solution: 5%-60%) to obtain the target compound A1B (yellow solid, 400 mg, yield: 67%).
LCMS (ESI) [M+H]⁺: 468.9;
¹H NMR (400 MHz, DMSO-d6) δ 7.65 (s, 1H), 7.51 (s, 1H), 7.24 (s, 1H), 6.50 (s, 1H), 6.30 (s, 2H), 5.42 (s, 2H), 5.26 (s, 2H), 3.81 (d, *J =* 5.9 Hz, 2H), 3.22 (s, 2H), 1.98 (d, *J =* 6.7 Hz, 4H), 0.88 (t, *J* = 7.2 Hz, 3H).

### Step 2: synthesis of (S)-7-ethyl-7-hydroxy-14-(3-hydroxypropyl)-10,13-dihydro-11H-[1,3]dioxolo[4,5-g]pyrano[3',4':6,7]indolizino[1,2-b]quinoline-8,11(7H)-dione (A1)

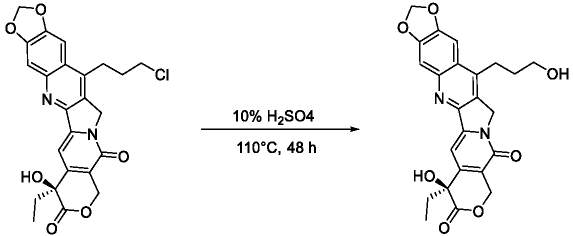

The compound (*S*)-7-ethyl-7-hydroxy-14-(3-chloropropyl)-10,13-dihydro-11*H-*[1,3]dioxolo[4,5-*g*]pyrano[3',4':6,7]indolizino[1,2-*b*]quinoline-8,11(7*H*)-dione (100 mg, 0.213 mmol) was dissolved in a 10% sulfuric acid (5 mL) solution and reacted at 110 °C for 48 h. To the reaction solution was added a saturated sodium bicarbonate solution (30 mL), and the resulting mixture was extracted with dichloromethane (10 mL × 5), dried over anhydrous sodium sulfate, filtered under vacuum, and concentrated under reduced pressure to give a crude product. The crude product was purified by high performance liquid chromatography (acetonitrile/water containing 0.05% formic acid) to give (*S*)-7-ethyl-7-hydroxy-14-(3-hydroxypropyl)-10,13-dihydro-11*H-*[1,3]dioxolo[4,5-*g*]pyrano[3',4':6,7]indolizino[1,2-*b*]quinoline-8,11(7*H*)-dione (A1, 1.78 mg).
LCMS (ESI) [M+H]⁺: 451.0;
¹H NMR (400 MHz, DMSO-*d6*) δ 7.63 (s, 1H), 7.50 (s, 1H), 7.24 (s, 1H), 6.48 (s, 1H), 6.28 (s, 2H), 5.47 - 5.37 (m, 2H), 5.32 - 5.19 (m, 2H), 3.51 - 3.46 (m, 2H), 3.17 - 3.13 (m, 2H), 1.92 - 1.76 (m, 4H), 0.90 - 0.84 (m, 3H).

### Example 1.1.2: synthesis of (S)-2-amino-N-((3-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propoxy)methyl)acetamide (B1)

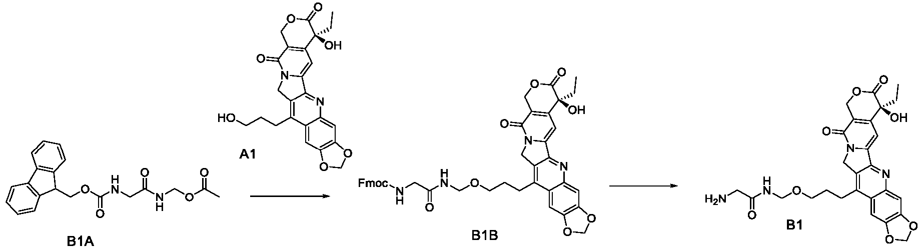

Step 1: the compound (B1A) (Cas No.: 1599440-06-8, 368 mg), the compound (A1) (440 mg), and pyridinium p-toluenesulfonate (PPTS) (25 mg) were refluxed in dichloromethane (20 mL) for 20 h, then washed sequentially with an aqueous sodium bicarbonate solution and an aqueous hydrochloric acid solution. The mixture was concentrated under reduced pressure to remove the organic solvent to give a crude product. The crude product was purified by column chromatography (dichloromethane:methanol = 10/1) to give the target compound B1B (240 mg).

LCMS (ESI) [M+H]⁺: 759.5.

Step 2: B1B (240 mg) was dissolved in DMF (5 mL), piperidine (1 mL) was added, and the mixture was stirred for 20 min. The reaction solution was concentrated under reduced pressure to remove low boiling components, and the residue was used directly in the next step. A small amount of the crude product was purified by reverse-phase chromatography (acetonitrile/0.05% FA in water: 5% to 50%) to give the target compound B1.
ESI-MS (m/z): 537.4 [M+H]⁺;
¹H NMR (400 MHz, DMSO-d6) δ 9.13 (t, 1H), 8.04 (br, 2H), 7.58 (s, 1H), 7.51 (s, 1H), 7.25 (s, 1H), 6.29 (s, 2H), 5.43 (S, 2H), 5.21 (s, 2H), 4.65 (d, 2H), 3.63 (m, 2H), 3.53 (m, 2H), 3.11 (m, 2H), 1.87 (m, 4H), 0.88 (t, 3H).

### Example 1.1.3: N⁶,N⁶-dimethyl-N²-((6-(2-(methylsulfonyl)pyrimidin-5-yl)hex-5-ynoyl)-L-valyl)-L-lysine (C1)

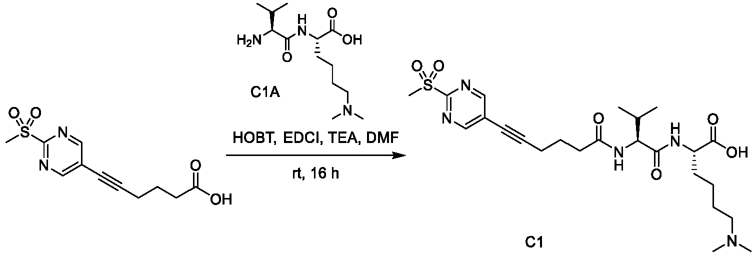

6-(2-(methylsulfonyl)pyrimidin-5-yl)hex-5-ynoic acid (268 mg, Cas No. 2356229-58-6), the compound C1A (328 mg), and triethylamine (322 mg) were dissolved in *N*,*N*-dimethylformamide (5 mL). 1-hydroxybenzotriazole (HOBT, 162 mg) and 1-(3-dimethylaminopropyl)-3-ethylcarbodiimide hydrochloride (EDCI, 229 mg) were then added, and the mixture was stirred at room temperature for 16 h. The reaction solution was directly purified by C18 reverse-phase column chromatography (acetonitrile and 0.05% aqueous formic acid solution system) to give the target compound *N*⁶,*N*⁶-dimethyl-*N²*-((6-(2-(methylsulfonyl)pyrimidin-5-yl)hex-5-ynoyl)-*L*-valyl)-Z-lysine (C1, white solid, 327 mg).

LCMS (ESI) [M+H]⁺: 524.4.

¹H NMR (400 MHz,) δ 9.13 (s, 2H), 7.95 (t, *J =* 8.8 Hz, 2H), 4.21 (dd, *J =* 8.8, 6.9 Hz, 1H), 4.08 - 4.03 (m, 1H), 3.41 (s, 3H), 2.55 (t, *J* = 7.0 Hz, 2H), 2.42 - 2.32 (m, 4H), 2.27 (s, 6H), 1.98 (dd, *J* = 13.6, 6.8 Hz, 1H), 1.86 - 1.77 (m, 2H), 1.74 - 1.55 (m, 2H), 1.47 - 1.37 (m, 2H), 1.31 - 1.23 (m, 2H), 0.85 (dd, *J =* 12.8, 6.8 Hz, 6H).

### Example 1.1.4: N⁶,N⁶-diethyl-N²-((6-(2-(methylsulfonyl)pyirimidin-5-yl)hex-5-ynoyl)-L-valyl)-L-lysine (C2)

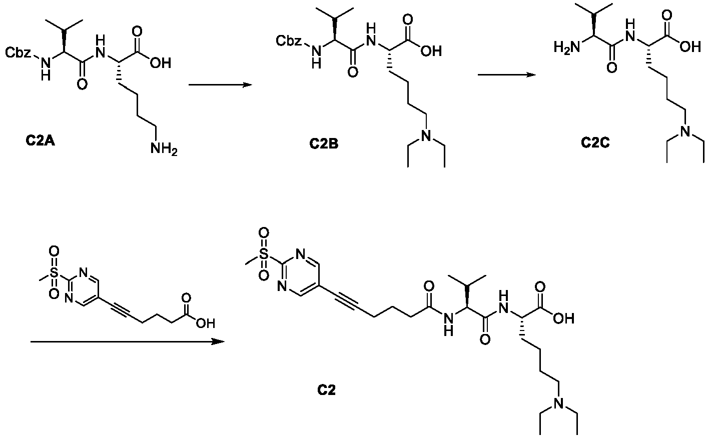

Step 1:
compound C2A (5.0 g, 12.05 mmol, Cas: 1872-06-8) was dissolved in dichloromethane (100 mL), and acetaldehyde (3.2 g, 72.3 mmol) was added to the solution. The mixture was stirred at room temperature for 10 min. Sodium triacetoxyborohydride (12.8 g, 60.25 mmol) was added to the reaction solution, and the resulting mixture was stirred at room temperature for 1 h. After the reaction was completed as detected by LCMS, to the reaction solution was added a saturated aqueous ammonium chloride solution, and the resulting mixture was stirred for one hour. The reaction solution was dried by rotary evaporation and filtered, and the filtrate was purified by C18 reverse phase column chromatography (acetonitrile:0.05% aqueous formic acid solution: 5% to 55%) to give the target compound C2B (4.57 g, yield: 82.0%) as a white solid.
LCMS (ESI) [M+H]⁺ = 436.4;
¹H NMR (400 MHz, DMSO-d6) δ 7.70 (d, *J =* 7.0 Hz, 1H), 7.41 (d, *J* 9.0 Hz, 1H), 7.38 - 7.26 (m, 5H), 5.08 - 4.99 (m, 2H), 4.00 (dd, *J =* 12.6, 6.5 Hz, 1H), 3.86 (dd, *J =* 8.6, 6.8 Hz, 1H), 2.74 (dd, *J =* 14.0, 6.9 Hz, 4H), 2.64 - 2.54 (m, 2H), 2.05 - 1.94 (m, 1H), 1.72 - 1.52 (m, 2H), 1.52 - 1.38 (m, 2H), 1.38 - 1.18 (m, 2H), 1.04 (t, *J =* 7.1 Hz, 6H), 0.87 - 0.81 (m, 6H).

Step 2:
compound C2B (1.6 g, 3.68 mmol) was dissolved in methanol (80 mL) at room temperature, and Pd/C (0.16 g) was added to the solution. The mixture was stirred at room temperature for 12 h under hydrogen atmosphere. After the reaction was completed as detected by LCMS, the reaction solution was filtered, and the filtrate was concentrated under reduced pressure to give the target compound C2C (900 mg, yield: 82%) as a creamy-white solid.

¹H NMR (400 MHz, DMSO-d6) δ 8.04 (br s, 1H), 4.02 - 3.99 (m, 1H), 3.10 (d, *J =* 4.5 Hz, 1H), 2.65 (q, *J = 7.1* Hz, 4H), 2.55 - 2.51 (m, 2H), 2.06 - 1.93 (m, 1H), 1.73 - 1.54 (m, 2H), 1.47 - 1.38 (m, 2H), 1.30 - 1.21 (m, 2H), 1.01 (t, *J =* 7.1 Hz, 6H), 0.89 (d, *J =* 6.9 Hz, 3H), 0.79 (d, *J =* 6.8 Hz, 3H).

Step 3:
compound 6-(2-(methylsulfonyl)pyrimidin-5-yl)hex-5-ynoic acid (268 mg, 1 mmol) was dissolved in DMF (8 mL), and HATU (380 mg, 1 mmol) and triethylamine (322 mg, 2.5 mmol) were added sequentially. The mixture was stirred at room temperature for 20 min, and compound C2C (301 mg, 1 mmol) was added. The resulting mixture was stirred at room temperature for 30 min. After the reaction was completed as detected by LCMS, the reaction solution was directly purified by C18 reverse-phase column chromatography (acetonitrile and 0.05% aqueous formic acid solution system) to give the target compound C2 (280 mg, yield: 51%) as a white solid.
LCMS (ESI) [M+H]⁺ = 552.3;
¹H NMR (400 MHz, DMSO-d6) δ 9.13 (s, 2H), 7.95 (d, *J =* 8.9 Hz, 1H), 7.86 (d, *J =* 7.2 Hz, 1H), 4.18 (dd, *J* = 8.8, 6.8 Hz, 1H), 4.02 (dd, *J =* 12.8, 7.2 Hz, 1H), 3.41 (s, 3H), 2.74 - 2.69 (m, 4H), 2.62 - 2.52 (m, 4H), 2.44 - 2.29 (m, 2H), 2.04 - 1.94 (m, 1H), 1.86 - 1.77 (m, 2H), 1.72 - 1.54 (m, 2H), 1.51 - 1.39 (m, 2H), 1.33 - 1.23 (m, 2H), 1.02 (t, *J =* 7.2 Hz, 6H), 0.87 - 0.82 (m, 6H).

### Example 1.1.5: N⁶,N⁶-dipropyl-N²-((6-(2-(methylsulfonyl)pyrimidin-5-yl)hex-5-ynoyl)-L-valyl)-L-lysine (C3)

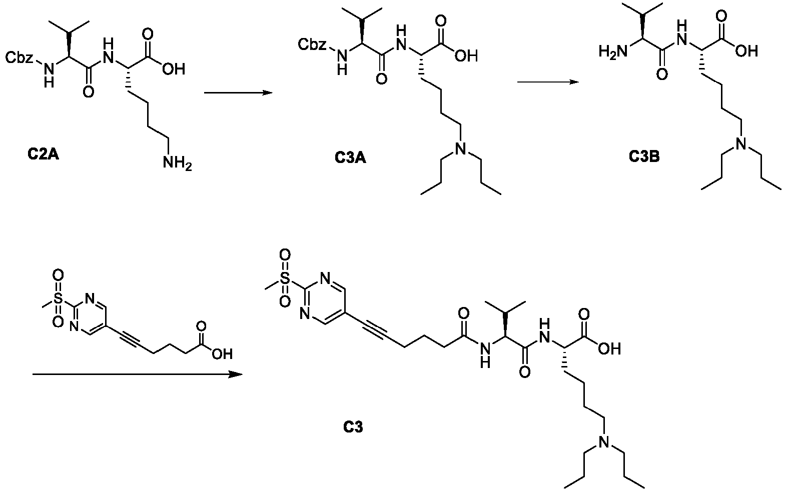

Step 1:
compound C2A (5.0 g, 12 mmol) was dissolved in dichloromethane (100 mL), and *n-*propionaldehyde (4.2 g, 72.3 mmol) was added to the solution. The mixture was stirred at room temperature for 10 min. Sodium triacetoxyborohydride (12.8 g, 60.25 mmol) was added to the reaction solution, and the resulting mixture was stirred at room temperature for 1 h. After the reaction was completed as detected by LCMS, to the reaction solution was added a saturated aqueous ammonium chloride solution, and the resulting mixture was stirred for one hour. The reaction solution was dried by rotary evaporation and filtered, and the filtrate was purified by C18 reverse phase column chromatography (acetonitrile:0.05% aqueous formic acid solution: 5% to 55%) to give the target compound C3A (4.57 g, yield: 82.0%) as a white solid.
LCMS (ESI) [M+H]⁺ = 464.0;
¹H NMR (400 MHz, DMSO-d6) δ 7.81 (d, *J =* 7.3 Hz, 1H), 7.38 - 7.28 (m, 5H), 5.04 (d, *J =* 1.7 Hz, 2H), 4.12 - 4.02 (m, 1H), 3.94 - 3.82 (m, 1H), 2.65 - 2.52 (m, 6H), 2.06 - 1.94 (m, 1H), 1.76 - 1.64 (m, 1H), 1.64 - 1.53 (m, 1H), 1.52 - 1.40 (m, 6H), 1.34 - 1.18 (m, 2H), 0.92 - 0.80 (m, 12H).

Step 2:
compound C3A (2.0 g, 4.32 mmol) was dissolved in methanol (80 mL) at room temperature, and Pd/C (0.16 g) was added to the solution. The mixture was stirred at room temperature for 12 h under hydrogen atmosphere. After the reaction was completed as detected by LCMS, the reaction solution was filtered, and the filtrate was concentrated under reduced pressure to give the target compound C3B (1.2 g, yield: 85.5%) as a white solid.

Step 3: compound 6-(2-(methylsulfonyl)pyrimidin-5-yl)hex-5-ynoic acid (100 mg, 0.373 mmol) was dissolved in *N*,*N*-dimethylformamide (1 mL), and HATU (142 mg, 0.373 mmol) and *N,N-*diisopropylethylamine (120 mg, 0.93 mmol) were added. The system was stirred for 30 min, and compound C3B (122 mg, 0.371 mmol) was added. The mixture was stirred at room temperature for 1 h. After the reaction was completed as detected by LCMS, the reaction solution was directly purified by C18 reverse-phase column chromatography (acetonitrile and 0.05% aqueous formic acid solution system) to give the target compound *N*⁶,*N*⁶-dipropyl-*N*²-((6-(2-(methylsulfonyl)pyrimidin-5-yl)hex-5-ynoyl)-*L*-valyl)-*L*-lysine (C3, 50 mg, yield: 28%) as a light yellow solid.
LCMS (ESI) [M+H]⁺ = 580.0;
¹H NMR (400 MHz, DMSO-d6) δ 8.24 (s, 2H), 7.98 - 7.93 (m, 2H), 4.24 - 4.16 (m, 1H), 4.10 (d, *J =* 5.2 Hz, 1H), 3.41 (s, 3H), 2.79 - 2.64 (m, 6H), 2.55 (t, *J =* 7.1 Hz, 2H), 2.45 - 2.26 (m, 2H), 2.06 - 1.91 (m, 1H), 1.89 - 1.78 (m, 2H), 1.76 - 1.66 (m, 1H), 1.64 - 1.57 (m, 1H), 1.57 - 1.42 (m, 6H), 1.37 - 1.24 (m, 2H), 0.93 - 0.78 (m, 12H).

### Example 1.2 Synthesis of Drug-Linker Compounds

### Example 1.2.1: N-((11S,14S)-11-(4-(dipropylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methylsulfonyl)pyrimidin-5-yl)hex-5-ynamide (DL-01)

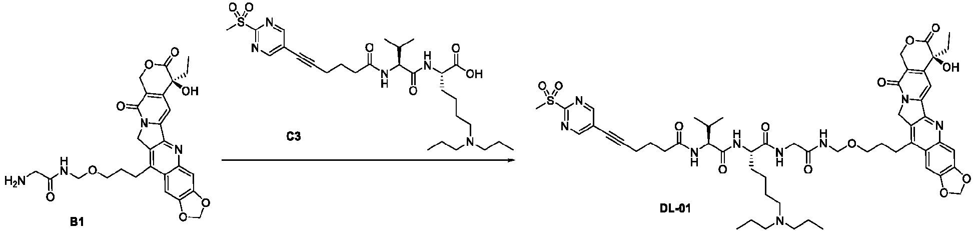

Compound C3 (43 mg, 0.074 mmol) and compound B1 (40 mg, 0.075 mmol) were dissolved in *N*,*N*-dimethylformamide (1 mL), and HBTU (28 mg, 0.075 mmol) and *N,N-*diisopropylethylamine (24 mg, 0.187 mmol) were added sequentially. The mixture was stirred at room temperature for 1 h. After the reaction was completed as detected by LCMS, the reaction solution was directly purified by preparative chromatography (0.01% aqueous trifluoroacetic acid solution, acetonitrile) to give the target compound (DL-01, 8.5 mg, yield: 10%) as a yellow solid.
LCMS (ESI) [M+H]⁺ = 1098.6;
¹H NMR (400 MHz, DMSO-d6) δ 9.10 (s, 2H), 9.03 (s, 1H), 8.64 (t, *J =* 6.4 Hz, 1H), 8.19 (t, *J =* 5.9 Hz, 1H), 8.08 (d, *J =* 7.4 Hz, 1H), 7.92 (d, *J =* 8.5 Hz, 1H), 7.59 (s, 1H), 7.51 (s, 1H), 7.24 (s, 1H), 6.49 (s, 1H), 6.29 (s, 2H), 5.42 (s, 2H), 5.24 (s, 2H), 4.66 - 4.52 (m, 2H), 4.31 - 4.21 (m, 1H), 4.19 - 4.10 (m, 1H), 3.74 (d, *J =* 5.5 Hz, 2H), 3.49 - 3.48 (m, 2H), 3.40 (s, 3H), 3.15 - 3.06 (m, 2H), 3.02 - 2.95 (m, 6H), 2.59 - 2.52 (m, 3H), 2.41 - 2.29 (m, 2H), 2.05 - 1.90 (m, 2H), 1.91 - 1.77 (m, 6H), 1.63 - 1.57 (m, 6H), 1.31 - 1.29 (m, 2H), 0.92 - 0.80 (m, 15H).

### Example 1.2.2: N((11S, 14S)-11-(4-(diethylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methylsulfonyl)pyrimidin-5-yl)hex-5-ynamide (DL-02)

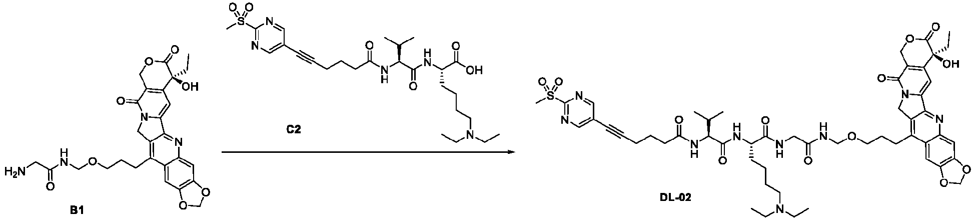

Compound C2 (40 mg, 0.075 mmol) and compound B1 (41 mg, 0.075 mmol) were dissolved in DMF (1 mL), and HOBt (15.2 mg, 0.113 mmol) and 1-(3-dimethylaminopropyl)-3-ethylcarbodiimide hydrochloride (21.5 mg, 0.113 mmol) were added, followed by triethylamine (23 mg, 0.225mmol). After the addition, the mixture was stirred at room temperature for 16 h. After the reaction was completed as detected by LCMS, the reaction solution was concentrated to give a crude product. The crude product was purified by preparative chromatography (0.01% aqueous TFA solution, acetonitrile) to give the target compound (DL-02, 16 mg, yield: 20%) as a yellow solid.
LCMS (ESI) [M+H]⁺ = 1070.6;
¹H NMR (400 MHz, DMSO-d6) δ 9.13 - 9.08 (m, 2H), 9.01 (s, 1H), 8.64 (t, *J =* 6.5 Hz, 1H), 8.20 (t, *J =* 5.7 Hz, 1H), 8.09 (d, *J =* 7.4 Hz, 1H), 7.92 (d, *J =* 8.4 Hz, 1H), 7.59 (s, 1H), 7.51 (s, 1H), 7.24 (s, 1H), 6.50 (s, 1H), 6.29 (s, 2H), 5.43 (s, 2H), 5.24 (s, 2H), 4.65 - 4.53 (m, 2H), 4.26 (d, *J =* 6.5 Hz, 1H), 4.20 - 4.10 (m, 1H), 3.74 (d, *J =* 5.5 Hz, 2H), 3.50 (t, *J =* 5.8 Hz, 2H), 3.41 (s, 3H), 3.14 - 3.07 (m, 6H), 2.98 (s, 2H), 2.55 (d, *J =* 7.3 Hz, 2H), 2.41 - 2.29 (m, 2H), 2.03 - 1.89 (m, 2H), 1.89 - 1.77 (m, 7H), 1.61 - 1.56 (m, 2H), 1.32 (s, 2H), 1.16 (t, *J* = 7.2 Hz, 6H), 0.91 - 0.78 (m, 9H).

### Example 1.2.3: N-((11S,14AS)-11-(4-(dimethylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methylsulfonyl)pyrimidin-5-yl)hex-5-ynamide (DL-03)

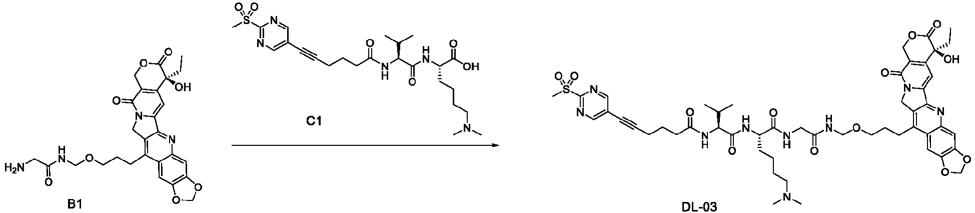

Compound B1 (100 mg, 0.186 mmol) and the compound *N*⁶,*N*⁶-dimethyl-*N*²-((6-(2-(methylsulfonyl)pyrimidin-5-yl)hex-5-ynoyl)-*L*-valyl)-*L*-lysine (C1, 100 mg, 0.191 mmol) were dissolved in DMF (2 mL). 1-hydroxybenzotriazole (38 mg, 0.280 mmol) and 1-(3-dimethylaminopropyl)-3-ethylcarbodiimide hydrochloride (53 mg, 0.280 mmol) were added, followed by triethylamine (56 mg, 0.559 mmol). After the addition, the reaction solution was stirred at room temperature for 16 h. After the reaction was completed as detected by LCMS, the reaction solution was directly purified by preparative chromatography (0.01% aqueous TFA solution, acetonitrile) to give the target compound (DL-03, 20 mg, yield: 10%) as a yellow solid.
LCMS (ESI) [M+H]⁺ = 1042.5;
¹H NMR (400 MHz, DMSO-d6) δ 9.28 (s, 1H, TFA), 9.10 (s, 2H), 8.64 (br s, 1H), 8.19 (br s, 1H), 8.09 (d, *J =* 6.4 Hz, 1H), 7.92 (d, *J =* 7.8 Hz, 1H), 7.59 (s, 1H), 7.51 (s, 1H), 7.24 (s, 1H), 6.50 (s, 1H), 6.29 (s, 2H), 5.43 (s, 2H), 5.24 (s, 2H), 4.67 - 4.52 (m, 2H), 4.30 - 4.10 (m, 2H), 3.74 (br s, 2H), 3.50 (br s, 2H), 3.41 (s, 3H), 3.17 - 3.07 (m, 2H), 3.04 - 2.91 (m, 2H), 2.75 (s, 6H), 2.46 - 2.24 (m, 3H), 2.04 - 1.66 (m, 9H), 1.63 - 1.46 (m, 3H), 1.32 - 1.29 (m, 2H), 0.87 - 0.82 (m, 9H).

### Example 1.3. Preparation of Anti-TF Antibody-Drug Conjugates

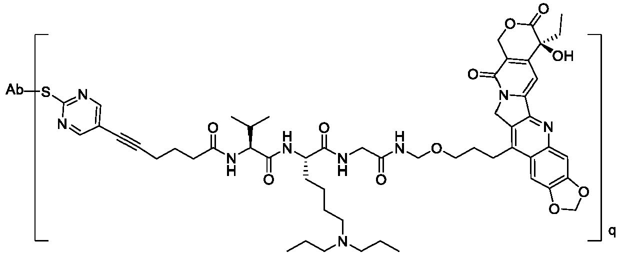

Ab is an anti-TF antibody.

### 1.3.1 Preparation of TF-ADC (DAR8) sample

30 mg of the anti-TF antibody prepared according to Example I was diluted with a diluent (20 mM PB, pH 7.5), a sodium edetate solution at the final concentration of 1 mM was added, and the mixture was mixed uniformly. TCEP solution that was 6.0 times the equivalent of the antibody was added, and the resulting mixture was mixed uniformly and left to stand at room temperature for 60 min. DL-01 dissolved in dimethyl sulfoxide that was 15 times the equivalent of the antibody was added into the above solution system, and the resulting mixture was mixed uniformly and left to stand at room temperature for 1 h to obtain a conjugate sample. After the reaction was completed, the sample was exchanged into 20 mM histidine buffer solution with pH of 6.0 using a 30 KDa ultrafiltration tube to remove low molecular substances, and finally the sample was concentrated to obtain the solution TF-ADC (DAR8) of the ADC composition containing the anti-TF antibody. The DAR value was determined to be 8.0 by mass spectrometry described in Example 1.4.

### 1.3.2 Preparation of TF-ADC (DAR3) sample

30 mg of the anti-TF antibody prepared according to Example I was diluted with a diluent (20 mM PB, pH 7.5), a sodium edetate solution at the final concentration of 1 mM was added, and the mixture was mixed uniformly. TCEP solution that was 6.0 times the equivalent of the antibody was added, and the resulting mixture was mixed uniformly and left to stand at room temperature for 60 min. DL-01 dissolved in dimethyl sulfoxide that was 4.0 times the equivalent of the antibody was added into the above solution system, and the resulting mixture was mixed uniformly and left to stand at room temperature for 1 h to obtain a conjugate sample. After the reaction was completed, the sample was exchanged into 20 mM histidine buffer solution with pH of 6.0 using a 30 KDa ultrafiltration tube to remove low molecular substances, and finally the sample was concentrated to obtain the solution TF-ADC (DAR3) of the ADC composition containing the anti-TF antibody. The DAR value was determined to be 3.0 by mass spectrometry.

### Example 1.4. Determination of DAR Values of Conjugate Samples by Mass Spectrometry

### 1.4.1 Analysis of LC-MS molecular weight and DAR value of TF-ADC (DAR3)

Chromatographic conditions:
chromatographic column: PLRP-S, 2.1 × 50 mm, 5 µm;
mobile phase A: 0.1% FA/H₂O, mobile phase B: 0.1% FA/ACN;
column temperature: 30 °C;
sample chamber temperature: 8 °C;
flow rate: 0.6 mL/min; and
sample injection volume: 2 µL.

| Time (min) | 1 | 5 | 5.1 | 7 | 10 |
|---|---|---|---|---|---|
| Mobile phase A | 90 | 40 | 10 | 90 | 90 |
| Mobile phase B | 10 | 60 | 90 | 10 | 10 |

Sample treatment: 50 µg of the sample was taken, 2 µL of 1 M DTT was added, and ultrapure water was added to bring the volume to 50 µL, so that the solution was diluted to a concentration of about 1.0 mg/mL. The solution was mixed uniformly and reduced at room temperature for 30 min.
LC/MS model: Agilent 1290-6545XT Q-TOF

Mass spectrum conditions: gas temp: 320 °C; drying gas: nitrogen; nebulizer: 35 psi; sheath gas temp: 350 °C; sheath gas flow: 11 L/min; and m/z: 500-3000.

The results are shown in Table 9 below.

**Table 9. Theoretical molecular weight and measured molecular weight**

| Peptide chain | | mAb | DAR1 | DAR2 | DAR3 |
|---|---|---|---|---|---|
| LC | Theoretical value | 23466.8 | 24484.9 | 25503.1 | 26521.3 |
| | Measured value | 23467 | 24485 | Not detected | Not detected |
| HC | Theoretical value | 50283.0 | 51301.1 | 52319.3 | 53337.4 |
| | Measured value | 50284 | 51302 | 52320 | 53338 |

In Table 9, mAb represents an unconjugated monoclonal antibody; LC represents an antibody light chain; HC represents an antibody heavy chain; DAR1 represents a conjugate comprising a light chain or a heavy chain conjugated to 1 toxin molecule; DAR2 represents a conjugate comprising a light chain or a heavy chain conjugated to 2 toxin molecules; DAR3 represents a conjugate comprising a light chain or a heavy chain conjugated to 3 toxin molecules; wherein the theoretical molecular weight of the monoclonal antibody is calculated by G0F glycoform. Hereinafter mAb, LC, HC, DAR1, DAR2, DAR3 are as described above.

The detection results show that in the TF-ADC (DAR3), the antibody light chain is conjugated to 0-1 toxin molecule (the proportions of LC and DAR1 are 53.8% and 46.2%, respectively), and the antibody heavy chain is conjugated to 0-3 toxin molecules (the proportions of mAb, DAR1, DAR2, and DAR3 are 30.6%, 42.4%, 20.2%, and 6.8%, respectively). Therefore, the conjugation ratio (average DAR value) of the TF-ADC (DAR3) sample is calculated to be 3.0.

### 1.4.2 Analysis of LC-MS molecular weight and DAR value of TF-ADC (DAR8)

Chromatographic conditions:
chromatographic column: Xbridge Protein BEH SEC (2.5 µm, 4.6 × 150 mm);
mobile phase A: 0.1% FA/H₂O, mobile phase B: 0.1% FA/ACN;
column temperature: 30 °C;
sample chamber temperature: 8 °C;
flow rate: 0.3 mL/min; and
sample injection volume: 1 µL.

| Time (min) | 0 | 5 | 7 | 7.1 | 10 |
|---|---|---|---|---|---|
| Mobile phase A | 90 | 20 | 20 | 90 | 90 |
| Mobile phase B | 10 | 80 | 80 | 10 | 10 |

Sample treatment: 50 µg of the sample was taken, 2 µL of 1 M DTT was added, and ultrapure water was added to bring the volume to 50 µL, so that the solution was diluted to a concentration of about 1.0 mg/mL. The solution was mixed uniformly and reduced at room temperature for 30 min.
LC/MS model: UPLC (AB SCIEX), high resolution mass spectrometer (AB SCIEX)

Mass spectrum conditions: Gas1: 45; Gas2: 45; CUR: 30; TEM: 450; ISVF: 5000; DP: 120; CE: 12; and mass number range: 600-4000.

The results are shown in Table 10 below.

**Table 10. Theoretical molecular weight and measured molecular weight**

| Peptide chain | | mAb | DAR1 | DAR2 | DAR3 |
|---|---|---|---|---|---|
| LC | Theoretical value | 23466.8 | 24484.9 | 25503.1 | 26521.3 |
| | Measured value | Not detected | 24485.1 | Not detected | Not detected |
| HC | Theoretical value | 50283.0 | 51301.1 | 52319.3 | 53337.4 |
| | Measured value | Not detected | Not detected | Not detected | 53338.1 |

The detection results show that in the TF-ADC (DAR8), the antibody light chain is conjugated to 0-1 toxin molecule (the proportions of LC and DAR1 are 0% and 100.0%, respectively), and the antibody heavy chain is conjugated to 0-3 toxin molecules (the proportions of mAb, DAR1, DAR2, and DAR3 are 0%, 0%, 0%, and 100.0%, respectively). Therefore, the conjugation ratio (average DAR value) of the TF-ADC (DAR8) sample is calculated to be 8.0.

### Example 2. Evaluation of Anti-TF Antibody ADCs

### Example 2.1 Affinity assay on Anti-TF ADCs

ADCs of each of 4 candidate antibodies of the present invention (2B12B10-hz1, 30G11B7-hz1, 22F11H5-hz1, and 27H8H3hz1) and the reference antibody (BM) with toxin-Linker (B81, i.e., DL-01 molecule) were prepared according to the method used in the previous example for preparing the ADC sample TF-ADC (DAR8).

Before and after the conjugation of the anti-TF humanized antibodies separately with toxin-Linker (B81, i.e., DL-01 molecule), the change in the affinity of the antibodies was measured by antigenic protein-based ELISA according to a method similar to that described in Example I.

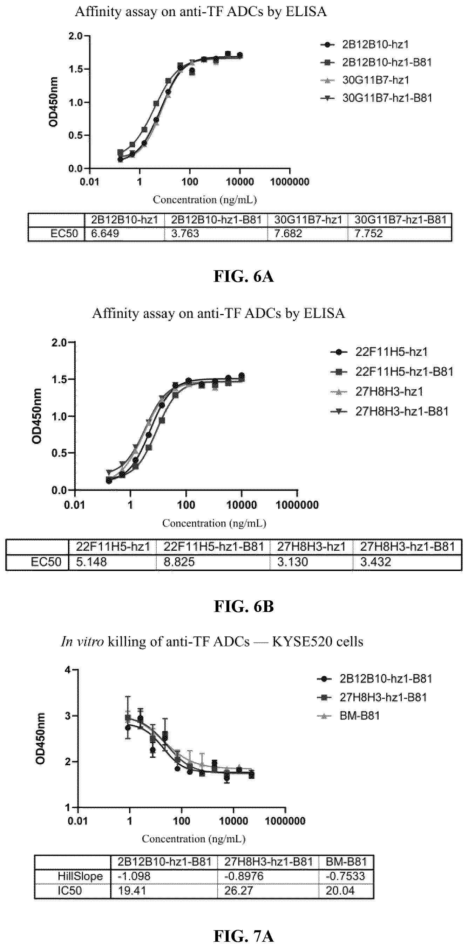

As shown in Table 11 and FIGs. 6A and 6B, the affinity of 4 humanized antibodies was not significantly changed after the conjugation with B81.

**Table 11. Affinity assay on humanized antibody ADCs before and after conjugation**

| Sample No. | EC50 (ng/mL) |
|---|---|
| 2B12B10-hz1 | 6.65 |
| 2B12B10-hz1-B81 | 3.76 |
| 30G11B7-hz1 | 7.68 |
| 30G11B7-hz1-B81 | 7.75 |
| 22F11H5-hz1 | 5.15 |
| 22F11H5-hz1-B81 | 8.83 |
| 27H8H3-hz1 | 3.13 |
| 27H8H3-hz1-B81 | 3.43 |

### Example 2.2 In vitro killing activity assay on ADCs

TF-positive tumor cells KYSE520 were digested with trypsin, collected by centrifugation, resuspended in RPMI1640 + 2% FBS, and seeded in a plate at 2000 cells/well. The test ADC was subjected to a gradient dilution (starting at 50 µg/mL, 3-fold dilution) with RPMI1640 + 2% FBS to obtain 11 concentration points. The ADCs obtained by the dilution were added to the plate, and the plate was incubated at 37 °C for 120 h. After the incubation was completed, CCK8 reagent was added at 20 µL/well for a reaction for 2-4 h. The absorbance values at 450 nm were read using a microplate reader, and imported into Graphpad Prism for curve fitting.

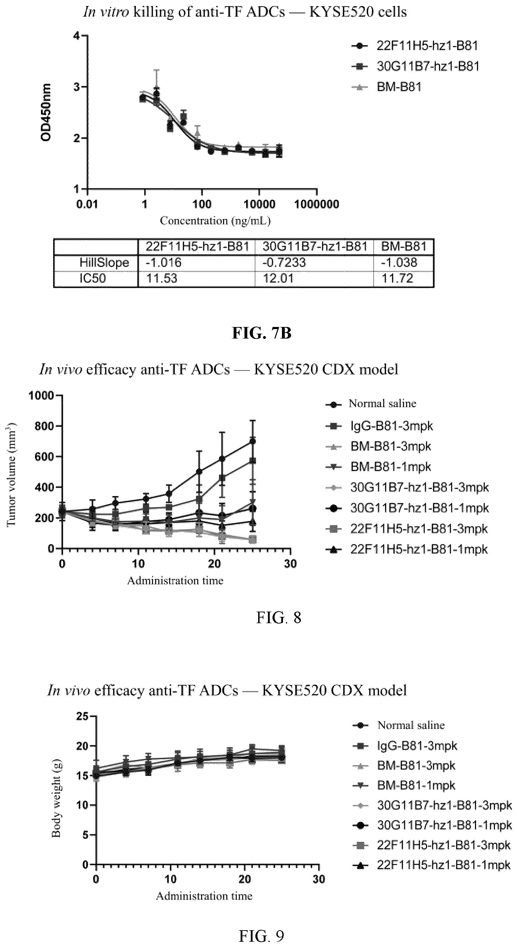

The experimental results are shown in FIGs. 7A and 7B, which show that 2B12B10-hz1, 30G11B7-hz1, 22F11H5-hz1, and 27H8H3-hz1 are all able to effectively kill KYSE520 cells after the conjugation with B81, with the killing IC₅₀ values substantially comparable to that of the reference antibody ADC.

### Example 2.3 In vivo efficacy of ADCs

To verify the *in vivo* efficacy of anti-TF humanized ADC drugs, and to evaluate the anti-tumor effect of the test drugs in a female Balb/c Nude mouse model with subcutaneous KYSE520 xenograft, female Balb/c Nude mice aged 5-6 weeks were purchased. KYSE520 cells in the logarithmic growth phase were digested with trypsin and resuspended in PBS, and 5 × 10⁶ cells were subcutaneously inoculated into each mouse. When the tumor grew to the size of 250 mm³, each of the test ADCs was intravenously administered once a week at 1 mg/kg or 3 mg/kg. The specific regimen is shown in Tables 13 and 14 below. The main observation indexes of the experiment were as follows: 1) TGI (%), calculated as: TGI (%) = (1 - T/C) × 100% (T and C are relative tumor volumes at a specific time point for treatment and control groups, respectively); 2) photographs showing the tumor volume size, and tumor weight at the end of the experiment; and 3) effect of ADC drugs on the body weight of the mice. A total of 2 experiments were performed.

The administration regimen and results of the first experiment are shown in Table 12 and FIG. 8, which show that 22F11H5-hz1 and 30G11B7 both have good anti-tumor effects in mice after the conjugation with B81, with TGI at a dose of 3 mg/kg comparable to that of BM-B81, and TGI at a dose of 1 mg/kg superior to that of BM-B81. The change in body weight of the mice is shown in FIG. 9, which shows that 2 ADCs do not affect the body weight of the mice throughout the administration.

**Table 12. In vivo efficacy of anti-TF ADCs**

| Grouping | Number of mice | Sample | Dose (mg/kg) | Route of administration | Frequency of administration | TGI (%) |
|---|---|---|---|---|---|---|
| 1 | 5 | Normal saline | / | i.v. | QW*3 | / |
| 2 | 5 | IgG-B81 | 3 | i.v. | QW*3 | 18.3 |
| 3 | 5 | BM-B81 | 3 | i.v. | QW*3 | 91.5 |
| 4 | 5 | BM-B81 | 1 | i.v. | QW*3 | 56.9 |
| 5 | 5 | 30G11B7-hz1-B81 | 3 | i.v. | QW*3 | 91.8 |
| 6 | 5 | 30G11B7-hz1-B81 | 1 | i.v. | QW*3 | 62.6 |
| 7 | 5 | 22F11H5-hz1-B81 | 3 | i.v. | QW*3 | 91.4 |
| 8 | 5 | 22F11H5-hz1-B81 | 1 | i.v. | QW*3 | 74.7 |

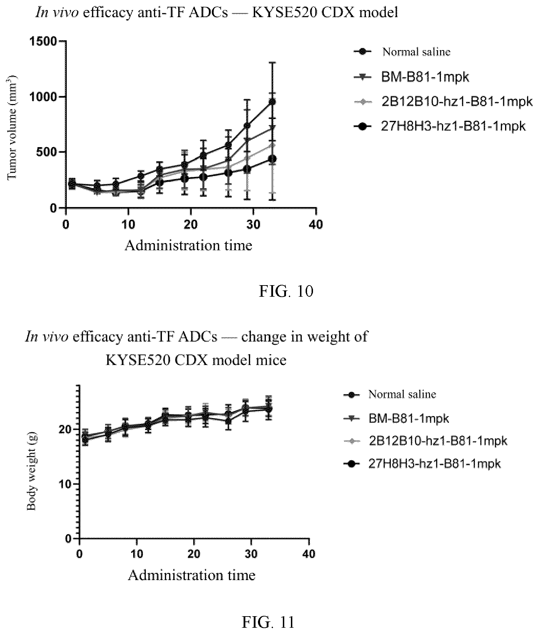

The experimental regimen and results of the second experiment are shown in Table 13 and FIG. 10, which show that 2B12B10-hz1 and 27H8H3-hz1 both have good anti-tumor effects in mice after the conjugation with B81, and are superior to BM-B81 at a dose of 1 mg/kg. The change in body weight of the mice is shown in FIG. 11, which shows that 2 ADCs do not affect the body weight of the mice.

**Table 13. In vivo efficacy of anti-TF ADCs**

| Grouping | Number of mice | Sample | Dose (mg/kg) | Route of administration | Frequency of administration | TGI (%) |
|---|---|---|---|---|---|---|
| 1 | 5 | Normal saline | / | *i*.*v*. | QW*3 | / |
| 2 | 5 | BM-B81 | 1 | *i.v*. | QW*3 | 18.5 |
| 3 | 5 | 2B12B10-B81 | 1 | *i.v*. | QW*3 | 41.4 |
| 4 | 5 | 27H8H3-hz1-B81 | 1 | *i.v*. | QW*3 | 52.8 |

### Sequence Listing:

| SEQ ID NO: | Description | Sequence |
|---|---|---|
| 1 | Heavy chain variable region (VH) sequence of 2B12B10-hz1 | EVQLVESGGGLVQPGGSLRLSCAASGFAFSDS GMHWVRQAPGKGLEWVAYISRDSNTIYYADS VKGRFTISRDNAKNSLYLQMNSLRAEDTAVY YCGRRYYGDYFDYWGQGTLVTVSS |
| 2 | Light chain variable region (VL) sequence of 2B12B10-hz1 | DIVLTQSPATLSLSPGERATLSCRASQRVTNYL HWYQQKPGQSPRLLIKYASQSISGIPARFSGSG SGTDFTLTISSLEPEDFAVYFCQQSYNWPLTFG QGTKVEIK |
| 3 | Heavy chain variable region (VH) sequence of 30G11B7-hz1 | EVQLVESGGGLVKPGGSLRLSCAASGFTFSDY GMFWVRQAPGKGLEWVAYISRGSTTIYYADS VKGRFTISRDNAKNSLYLQMNSLRAEDTAVYF CTRRYYGDYFDYWGQGTLVTVSS |
| 4 | Light chain variable region (VL) sequence of 30G11B7-hz1 | EIVLTQSPDFQSVTPKEKVTITCRASQSISNYL HWYQQKPDQSPKLLIKYASQSISGVPSRFSGS GSGTDFTLTINSLEAEDAATYFCQQSYNWPLT FGQGTKVEIK |
| 5 | Heavy chain variable region (VH) sequence of 22F11H5-hz1 | QVQLVQSGAEVKQPGASVKVSCKASGYTFTT YWMHWVRQAPGQGLEWMGMIDPSDSYTTY AQKFQGRVTLTVDTSTSTAYMELSSLRSEDTA VYYCARGSGPGLFAYWGQGTLVTVSS |
| 6 | Light chain variable region (VL) sequence of 22F11H5-hz1 | DIQETQSPSSLSASVGDRVTITCRASQDIRNYL NWYQQKPGKAPKLLIYHTSRLQSGVPSRFSGS GSGTDYTLTISSLQPEDFATYYCQQGNMPPYT FGQGTKVEIK |
| 7 | Heavy chain variable region (VH) sequence of 27H8H3-hz1 | QVQLVQSGAEVKKPGASVKVSCKASGYTFTT YWMHWVRQAPGQGLEWMGMIDPSDSYTSY AQKFQGRVTLTVDTSTSTAYMELSSLRSEDTA VYYCTRGSGPGLFAYWGQGTLVTVSS |
| 8 | Light chain variable region (VL) sequence of 27H8H3-hz1 | DIQETQSPSSLSASVGDRVTITCRASQGISNYL NWYQQKPGKAPKLLIYHTSRLHSGVPSRFSGS VSGTDFTLTISSLQPEDFATYFCQQGNTLPYTF GQGTKVEIK |
| 9 | Heavy chain CDR1 of 2B12B10-hz1_Chothia | GFAFSDS |
| 10 | Heavy chain CDR2 of 2B12B10-hz1_Chothia | SRDSNT |
| 11 | Heavy chain CDR3 of 2B12B10-hz1_Chothia | RYYGDYFDY |
| 12 | Heavy chain CDR1 of 2B12B10-hz1_Abm | GFAFSDSGMH |
| 13 | Heavy chain CDR2 of 2B12B10-hz1_Abm | YISRDSNTIY |
| 14 | Heavy chain CDR3 of 2B12B10-hz1_Abm | RYYGDYFDY |
| 15 | Heavy chain CDR1 of 2B12B10-hz1_Kabat | DSGMH |
| 16 | Heavy chain CDR2 of 2B12B10-hz1_Kabat | YISRDSNTIYYADSVKG |
| 17 | Heavy chain CDR3 of 2B12B10-hz1_Kabat | RYYGDYFDY |
| 18 | Heavy chain CDR1 of 2B12B10-hz1_IMGT | GFAFSDSG |
| 19 | Heavy chain CDR2 of 2B12B10-hz1_IMGT | ISRDSNTI |
| 20 | Heavy chain CDR3 of 2B12B10-hz1_IMGT | GRRYYGDYFDY |
| 21 | Light chain CDR1 of 2B12B10-hz1_Chothia | RASQRVTNYLH |
| 22 | Light chain CDR2 of 2B12B10-hz1_Chothia | YASQSIS |
| 23 | Light chain CDR3 of 2B12B10-hz1_Chothia | QQSYNWPLT |
| 24 | Light chain CDR1 of 2B12B10-hz1_Abm | RASQRVTNYLH |
| 25 | Light chain CDR2 of 2B12B10-hz1_Abm | YASQSIS |
| 26 | Light chain CDR3 of 2B12B10-hz1_Abm | QQSYNWPLT |
| 27 | Light chain CDR1 of 2B12B10-hz1_Kabat | RASQRVTNYLH |
| 28 | Light chain CDR2 of 2B12B10-hz1_Kabat | YASQSIS |
| 29 | Light chain CDR3 of 2B12B10-hz1_Kabat | QQSYNWPLT |
| 30 | Light chain CDR1 of 2B12B10-hz1_IMGT | QRVTNY |
| 31 | Light chain CDR2 of 2B12B10-hz1_IMGT | YAS |
| 32 | Light chain CDR3 of 2B12B10-hz1_IMGT | QQSYNWPLT |
| 33 | Heavy chain CDR1 of 30G11B7-hz1_Chothia | GFTFSDY |
| 34 | Heavy chain CDR2 of 30G11B7-hz1_Chothia | SRGSTT |
| 35 | Heavy chain CDR3 of 30G11B7-hz1_Chothia | RYYGDYFDY |
| 36 | Heavy chain CDR1 of 30G11B7-hz1_Abm | GFTFSDYGMF |
| 37 | Heavy chain CDR2 of 30G11B7-hz1_Abm | YISRGSTTIY |
| 38 | Heavy chain CDR3 of 30G11B7-hz1_Abm | RYYGDYFDY |
| 39 | Heavy chain CDR1 of 30G11B7-hz1_Kabat | DYGMF |
| 40 | Heavy chain CDR2 of 30G11B7-hz1_Kabat | YISRGSTTIYYADSVKG |
| 41 | Heavy chain CDR3 of 30G11B7-hz1_Kabat | RYYGDYFDY |
| 42 | Heavy chain CDR1 of 30G11B7-hz1_IMGT | GFTFSDYG |
| 43 | Heavy chain CDR2 of 30G11B7-hz1_IMGT | ISRGSTTI |
| 44 | Heavy chain CDR3 of 30G11B7-hz1_IMGT | TRRYYGDYFDY |
| 45 | Light chain CDR1 of 30G11B7-hz1_Chothia | RASQSISNYLH |
| 46 | Light chain CDR2 of 30G11B7-hz1_Chothia | YASQSIS |
| 47 | Light chain CDR3 of 30G11B7-hz1_Chothia | QQSYNWPLT |
| 48 | Light chain CDR1 of 30G11B7-hz1_Abm | RASQSISNYLH |
| 49 | Light chain CDR2 of 30G11B7-hz1_Abm | YASQSIS |
| 50 | Light chain CDR3 of 30G11B7-hz1_Abm | QQSYNWPLT |
| 51 | Light chain CDR1 of 30G11B7-hz1_Kabat | RASQSISNYLH |
| 52 | Light chain CDR2 of 30G11B7-hz1_Kabat | YASQSIS |
| 53 | Light chain CDR3 of 30G11B7-hz1_Kabat | QQSYNWPLT |
| 54 | Light chain CDR1 of 30G11B7-hz1_IMGT | QSISNY |
| 55 | Light chain CDR2 of 30G11B7-hz1_IMGT | YAS |
| 56 | Light chain CDR3 of 30G11B7-hz1_IMGT | QQSYNWPLT |
| 57 | Heavy chain CDR1 of 22F11H5-hz1_Chothia | GYTFTTY |
| 58 | Heavy chain CDR2 of 22F11H5-hz1_Chothia | DPSDSY |
| 59 | Heavy chain CDR3 of 22F11H5-hz1_Chothia | GSGPGLFAY |
| 60 | Heavy chain CDR1 of 22F11H5-hz1_Abm | GYTFTTYWMH |
| 61 | Heavy chain CDR2 of 22F11H5-hz1_Abm | MIDPSDSYTT |
| 62 | Heavy chain CDR3 of 22F11H5-hz1_Abm | GSGPGLFAY |
| 63 | Heavy chain CDR1 of 22F11H5-hz1_Kabat | TYWMH |
| 64 | Heavy chain CDR2 of 22F11H5-hz1_Kabat | MIDPSDSYTTYAQKFQG |
| 65 | Heavy chain CDR3 of 22F11H5-hz1_Kabat | GSGPGLFAY |
| 66 | Heavy chain CDR1 of 22F11H5-hz1_IMGT | GYTFTTYW |
| 67 | Heavy chain CDR2 of 22F11H5-hz1_IMGT | IDPSDSYT |
| 68 | Heavy chain CDR3 of 22F11H5-hz1_IMGT | ARGSGPGLFAY |
| 69 | Light chain CDR1 of 22F11H5-hz1_Chothia | RASQDIRNYLN |
| 70 | Light chain CDR2 of 22F11H5-hz1_Chothia | HTSRLQS |
| 71 | Light chain CDR3 of 22F11H5-hz1_Chothia | QQGNMPPYT |
| 72 | Light chain CDR1 of 22F11H5-hz1_Abm | RASQDIRNYLN |
| 73 | Light chain CDR2 of 22F11H5-hz1_Abm | HTSRLQS |
| 74 | Light chain CDR3 of 22F11H5-hz1_Abm | QQGNMPPYT |
| 75 | Light chain CDR1 of 22F11H5-hz1_Kabat | RASQDIRNYLN |
| 76 | Light chain CDR2 of 22F11H5-hz1_Kabat | HTSRLQS |
| 77 | Light chain CDR3 of 22F11H5-hz1_Kabat | QQGNMPPYT |
| 78 | Light chain CDR1 of 22F11H5-hz1_IMGT | QDIRNY |
| 79 | Light chain CDR2 of 22F11H5-hz1_IMGT | HTS |
| 80 | Light chain CDR3 of 22F11H5-hz1_IMGT | QQGNMPPYT |
| 81 | Heavy chain CDR1 of 27H8H3-hz1_Chothia | GYTFTTY |
| 82 | Heavy chain CDR2 of 27H8H3-hz1_Chothia | DPSDSY |
| 83 | Heavy chain CDR3 of 27H8H3-hz1_Chothia | GSGPGLFAY |
| 84 | Heavy chain CDR1 of 27H8H3-hz1_Abm | GYTFTTYWMH |
| 85 | Heavy chain CDR2 of 27H8H3-hz1_Abm | MIDPSDSYTS |
| 86 | Heavy chain CDR3 of 27H8H3-hz1_Abm | GSGPGLFAY |
| 87 | Heavy chain CDR1 of 27H8H3-hz1_Kabat | TYWMH |
| 88 | Heavy chain CDR2 of 27H8H3-hz1_Kabat | MIDPSDSYTSYAQKFQG |
| 89 | Heavy chain CDR3 of 27H8H3-hz1_Kabat | GSGPGLFAY |
| 90 | Heavy chain CDR1 of 27H8H3-hz1_IMGT | GYTFTTYW |
| 91 | Heavy chain CDR2 of 27H8H3-hz1_IMGT | IDPSDSYT |
| 92 | Heavy chain CDR3 of 27H8H3-hz1_IMGT | TRGSGPGLFAY |
| 93 | Light chain CDR1 of 27H8H3-hz1_Chothia | RASQGISNYLN |
| 94 | Light chain CDR2 of 27H8H3-hz1_Chothia | HTSRLHS |
| 95 | Light chain CDR3 of 27H8H3-hz1_Chothia | QQGNTLPYT |
| 96 | Light chain CDR1 of 27H8H3-hz1_Abm | RASQGISNYLN |
| 97 | Light chain CDR2 of 27H8H3-hz1_Abm | HTSRLHS |
| 98 | Light chain CDR3 of 27H8H3-hz1_Abm | QQGNTLPYT |
| 99 | Light chain CDR1 of 27H8H3-hz1_Kabat | RASQGISNYLN |
| 100 | Light chain CDR2 of 27H8H3-hz1_Kabat | HTSRLHS |
| 101 | Light chain CDR3 of 27H8H3-hz1_Kabat | QQGNTLPYT |
| 102 | Light chain CDR1 of 27H8H3-hz1_IMGT | QGISNY |
| 103 | Light chain CDR2 of 27H8H3-hz1_IMGT | HTS |
| 104 | Light chain CDR3 of 27H8H3-hz1_IMGT | QQGNTLPYT |
| 105 | IgG1 Kappa CL | |
| 106 | IgG1 CH | |
| 107 | Heavy chain variable region (VH) of benchmark | EVQLLESGGGLVQPGGSLRLSCAASGFTFSNY AMSWVRQAPGKGLEWVSSISGSGDYTYYTDS VKGRFTISRDNSKNTLYLQMNSLRAEDTAVY YCARSPWGYYLDSWGQGTLVTVSS |
| 108 | Light chain variable region of benchmark | EIVLTQSPGTLSLSPGERATLSCRASQSVSSSYL AWYQQKPGQAPRLLIYGASSRATGIPDRFSGS GSGTDFTLTISRLEPEDFAVYYCQQYGSSLTFG GGTKVEIK |

## Claims

1. An antibody-drug conjugate (ADC), having the following formula (I), or a pharmaceutical acceptable salt or solvate thereof:
Ab-[L-D]_{q} (I)
wherein,
Ab represents an anti-TF antibody,
L represents a linker,
D represents a cytotoxic or cytostatic drug, e.g., a topoisomerase I inhibitor, and
q = 1-20, e.g., q = 1-10, 1-8, 3-8, 4-8, or 6-8,
wherein the Ab comprises:
- three CDRs of a heavy chain variable region (VH) sequence set forth in SEQ ID NO: 1 and three CDRs of a light chain variable region (VL) sequence set forth in SEQ ID NO: 2,
- three CDRs of a heavy chain variable region (VH) sequence set forth in SEQ ID NO: 3 and three CDRs of a light chain variable region (VL) sequence set forth in SEQ ID NO: 4,
- three CDRs of a heavy chain variable region (VH) sequence set forth in SEQ ID NO: 5 and three CDRs of a light chain variable region (VL) sequence set forth in SEQ ID NO: 6, or
- three CDRs of a heavy chain variable region (VH) sequence set forth in SEQ ID NO: 7 and three CDRs of a light chain variable region (VL) sequence set forth in SEQ ID NO: 8,
wherein the CDRs are defined according to Chothia, AbM, Kabat, IMGT, or any combination thereof.

2. The antibody-drug conjugate or the pharmaceutical acceptable salt or solvate thereof according to claim 1, wherein the Ab comprises 3 heavy chain complementarity determining regions (HCDR) and 3 light chain complementarity determining regions (LCDR), wherein
(i) according to the IMGT scheme, the HCDR1 comprises or consists of an amino acid sequence set forth in SEQ ID NO: 18, the HCDR2 comprises or consists of an amino acid sequence set forth in SEQ ID NO: 19, the HCDR3 comprises or consists of an amino acid sequence set forth in SEQ ID NO: 20, the LCDR1 comprises or consists of an amino acid sequence set forth in SEQ ID NO: 30, the LCDR2 comprises or consists of an amino acid sequence set forth in SEQ ID NO: 31, and the LCDR3 comprises or consists of an amino acid sequence set forth in SEQ ID NO: 32; or
(ii) according to the IMGT scheme, the HCDR1 comprises or consists of an amino acid sequence set forth in SEQ ID NO: 42, the HCDR2 comprises or consists of an amino acid sequence set forth in SEQ ID NO: 43, the HCDR3 comprises or consists of an amino acid sequence set forth in SEQ ID NO: 44, the LCDR1 comprises or consists of an amino acid sequence set forth in SEQ ID NO: 54, the LCDR2 comprises or consists of an amino acid sequence set forth in SEQ ID NO: 55, and the LCDR3 comprises or consists of an amino acid sequence set forth in SEQ ID NO: 56; or
(iii) according to the IMGT scheme, the HCDR1 comprises or consists of an amino acid sequence set forth in SEQ ID NO: 66, the HCDR2 comprises or consists of an amino acid sequence set forth in SEQ ID NO: 67, the HCDR3 comprises or consists of an amino acid sequence set forth in SEQ ID NO: 68, the LCDR1 comprises or consists of an amino acid sequence set forth in SEQ ID NO: 78, the LCDR2 comprises or consists of an amino acid sequence set forth in SEQ ID NO: 79, and the LCDR3 comprises or consists of an amino acid sequence set forth in SEQ ID NO: 80; or
(iv) according to the IMGT scheme, the HCDR1 comprises or consists of an amino acid sequence set forth in SEQ ID NO: 90, the HCDR2 comprises or consists of an amino acid sequence set forth in SEQ ID NO: 91, the HCDR3 comprises or consists of an amino acid sequence set forth in SEQ ID NO: 92, the LCDR1 comprises or consists of an amino acid sequence set forth in SEQ ID NO: 102, the LCDR2 comprises or consists of an amino acid sequence set forth in SEQ ID NO: 103, and the LCDR3 comprises or consists of an amino acid sequence set forth in SEQ ID NO: 104.

3. The antibody-drug conjugate or the pharmaceutical acceptable salt or solvate thereof according to any one of claims 1-2, wherein the Ab comprises:
- a heavy chain variable region comprising SEQ ID NO: 1 or an amino acid sequence having at least 80%, 85%, 90%, 95%, 96%, 97%, 98% or 99% sequence identity thereto; and a light chain variable region comprising SEQ ID NO: 2 or an amino acid sequence having at least 80%, 85%, 90%, 95%, 96%, 97%, 98% or 99% sequence identity thereto, or
- a heavy chain variable region comprising SEQ ID NO: 3 or an amino acid sequence having at least 80%, 85%, 90%, 95%, 96%, 97%, 98% or 99% sequence identity thereto; and a light chain variable region comprising SEQ ID NO: 4 or an amino acid sequence having at least 80%, 85%, 90%, 95%, 96%, 97%, 98% or 99% sequence identity thereto, or
- a heavy chain variable region comprising SEQ ID NO: 5 or an amino acid sequence having at least 80%, 85%, 90%, 95%, 96%, 97%, 98% or 99% sequence identity thereto; and a light chain variable region comprising SEQ ID NO: 6 or an amino acid sequence having at least 80%, 85%, 90%, 95%, 96%, 97%, 98% or 99% sequence identity thereto, or
- a heavy chain variable region comprising SEQ ID NO: 7 or an amino acid sequence having at least 80%, 85%, 90%, 95%, 96%, 97%, 98% or 99% sequence identity thereto; and a light chain variable region comprising SEQ ID NO: 8 or an amino acid sequence having at least 80%, 85%, 90%, 95%, 96%, 97%, 98% or 99% sequence identity thereto,
preferably, the Ab is an IgG1 antibody.

4. The antibody-drug conjugate or the pharmaceutical acceptable salt or solvate thereof according to any one of claims 1-3, wherein D represents a camptothecin drug, preferably FLU 8 or a derivative thereof, e.g., a camptothecin drug comprising the following structure: 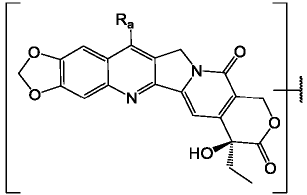 wherein Rₐ is selected from:
hydrogen;
C₃-C₈ cycloalkyl;
phenyl; and
C₁-C₈ alkyl optionally substituted with a substituent selected from: halogen, hydroxy, C₁-C₄ alkoxy optionally substituted with NH₂, NH(C₁-C₄ alkyl), and N(C₁-C₄ alkyl)₂, C₃-C₈ cycloalkyl, heterocycloalkyl, phenyl, and NR¹R²,
wherein R¹ and R² are each independently selected from:
hydrogen;
C₁-C₈ alkyl optionally substituted with a substituent selected from: hydroxy, amino, amino substituted with one or two C₁-C₄ alkyl, amino substituted with one or two C₁-C₄ hydroxyalkyl, and amino substituted with (C₁-C₄ hydroxyalkyl) and (C₁-C₄ alkyl);
C₁-C₄ alkyl substituted with 1 or 2 C₃-C₁₀ cycloalkyl, C₃-C₁₀ heterocycloalkyl, phenyl or heteroaryl;
C₃-C₁₀ cycloalkyl;
C₃-C₁₀ heterocycloalkyl;
C₂-C₆ heteroalkyl;
heteroaryl;
optionally halogenated phenyl; and
C₁-C₈ alkyl-C(=O)- optionally substituted with hydroxy or amino;
or, R¹ and R², in combination with the nitrogen atom to which they are connected, form a 5-, 6-, or 7-membered heterocyclic ring having 0-3 substituents selected from halogen, C₁-C₄ alkyl, OH, C₁-C₄ alkoxy, NH₂, NH(C₁-C₄ alkyl), and N(C₁-C₄ alkyl)₂,
wherein the cycloalkyl, heterocycloalkyl, phenyl, and heteroaryl, at each occurrence, are each independently and optionally substituted with 0-3 substituents selected from: OH, C₁-C₄ alkyl, C₁-C₄ alkoxy, NH₂, NH(C₁-C₄ alkyl), and N(C₁-C₄ alkyl)₂,
preferably, Rₐ is -C₁-C₄ alkyl-OH, -C₁-C₄ alkyl-O-C₁-C₄ alkyl-NH₂, and -C₁-C₄ alkyl-NH₂,
wherein preferably, the drug D unit is linked to the linker L unit via a hydroxy or amino group present thereon.

5. The antibody-drug conjugate or the pharmaceutical acceptable salt or solvate thereof according to any one of claims 1-3, wherein the L-D unit in formula (I) comprises the following structure: 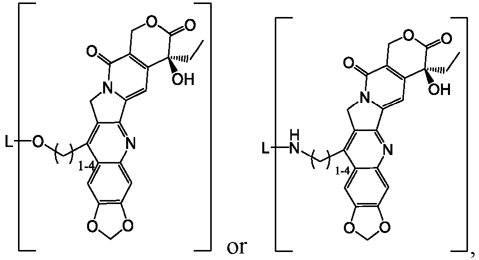 preferably, the L-D unit in formula (I) comprises the following structure:

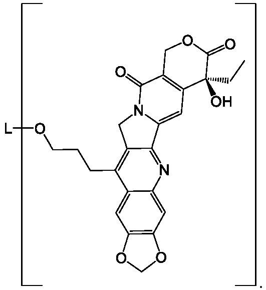

6. The antibody-drug conjugate or the pharmaceutical acceptable salt or solvate thereof according to any one of claims 1-5, wherein L is a peptide-containing linker and comprises the following structure of formula (II):
-Z-Y-M-, (II)
wherein,
Z is a linking group linked to Ab,
Y is a peptide of 2-5 amino acids, preferably dipeptide, tripeptide or tetrapeptide, and
M is absent or is a spacer group for linking to the drug D.

7. The antibody-drug conjugate or the pharmaceutical acceptable salt or solvate thereof according to claim 6, wherein Y is a peptide having the following amino acid sequence from the N-terminus to the C-terminus;
Xaa₁-Xaa₂-Xaa₃-Xaa₄-Xaa₅,
wherein Xaa₁ is absent or is an amino acid selected from valine, glycine, alanine, and glutamic acid;
Xaa₂ is an amino acid selected from phenylalanine, leucine, and valine, preferably valine;
Xaa₃ is unsubstituted or substituted lysine;
Xaa₄ is an amino acid selected from leucine, glycine, and alanine;
Xaa₅ is absent or is an amino acid selected from glycine and alanine;
wherein the N-terminus of the amino acid sequence is linked to Z, and the C-terminus is linked to M (if M is present) or directly linked to the drug D,
preferably, Xaa₃ is lysine in which an ε-amino group is monosubstituted or disubstituted with C₁-C₃ alkyl,
and more preferably, Y is a peptide selected from: Phe-Lys-Gly, Leu-lys-Gly, Gly-Val-Lys-Gly, Val-Lys-Gly-Gly, Val-Lys-Gly, Val-Lys-Ala and Val-Lys-Leu, wherein the Lys residue is unsubstituted lysine or lysine monosubstituted or disubstituted with C₁-C₃ alkyl.

8. The antibody-drug conjugate or the pharmaceutical acceptable salt or solvate thereof according to any one of claims 6-7, wherein Y is 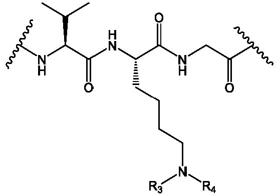
wherein R₃ and R₄ are each independently selected from methyl, ethyl and propyl,
preferably, R₃ and R₄ are identical, and more preferably, R₃ and R₄ are each independently propyl,
wherein the left wavy line indicates the position wherein Y is linked to Z; and the right wavy line indicates the position where Y is linked to M.

9. The antibody-drug conjugate or the pharmaceutical acceptable salt or solvate thereof according to claims 6-8, wherein Z has the following structure:
-Z₁-Z₂-Z₃-Z₄-,
wherein Z₁ is a sulfur atom in Ab;
Z₂ is a 5- to 10-membered heterocyclyl, preferably containing 1 or 2 heteroatoms selected from N, S, and O;
Z₃ is selected from a bond, -C(=O)-, -C₁-C₁₀ alkylene-C(=O)-, -C₃-C₁₀ alkynylene-C(=O)-, -C₃-C₁₀ alkenylene-C(=O)-, -C₁-C₁₀ heteroalkylene-C(=O)-, -C₃-C₈ cycloalkylene-C(=O)-, -O-C₁-C₈ alkylene-C(=O)-, -arylene-C(=O)-, -C₁-C₁₀ alkylene-arylene-C(=O)-, -arylene-C₁-C₁₀ alkylene-C(=O)-, -C₁-C₁₀ alkylene-Cs-Cs cycloalkylene-C(=O)-, -C₃-C₈ cycloalkylene-C₁-C₁₀-alkylene-C(=O)-, -C₃-C₈-heteroalkylene-C(=O)-, C₁-C₁₀ alkylene-C₃-C₈-heteroalkylene-C(=O)-, and C₃-C₈-heteroalkylene-C₁-C₁₀ alkylene-C(=O)-; and
Z₄ is a bond or a PEG unit represented by the following formula: 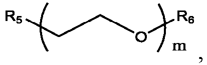
wherein R₅ is selected from C₁₋₄ alkylene, -NH-, and -NH-C₁₋₄ alkylene-heteroaryl-, wherein the heteroaryl is a 5- or 6-membered nitrogen-containing heteroaryl, preferably triazolyl; and R₆ is -C(=O)-, C₁₋₄ alkylene, C₁₋₄ alkylene-C(=O)-, -NH-C(=O)-(CH₂OCH₂)-C(=O)-, and C₁₋₄ alkylene-NH-C(=O)-(CH₂OCH₂)-C(=O)-, wherein m is an integer of 2-12, for example, m = 2, 4, 6 or 8,
preferably, Z has the following structure: 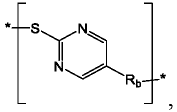
wherein R_{b} is alkynylene-C(=O)- or alkenylene-C-(=O)-,
preferably, R_{b} is: 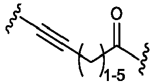
wherein the left wavy line indicates the position where Z is linked to the antibody (Ab); and the right wavy line indicates the position wherein Z is linked to Y;
and more preferably, Z has the following structure:

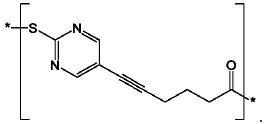

10. The antibody-drug conjugate or the pharmaceutical acceptable salt or solvate thereof according to claims 6-9, wherein M is absent or amino-C₁-C₃ alkylene, e.g., -NH-CH₂- or aminophenyl-C₁-C₃ alkylene-O-C(=O)-, preferably, Mis: 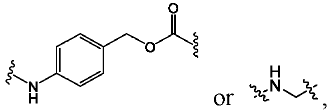 wherein the left wavy line indicates the linkage to Y and the right wavy line indicates the linkage to the drug D unit.

11. The antibody-drug conjugate or the pharmaceutical acceptable salt or solvate thereof according to claims 6-10, wherein L is a linker comprising the following structure: 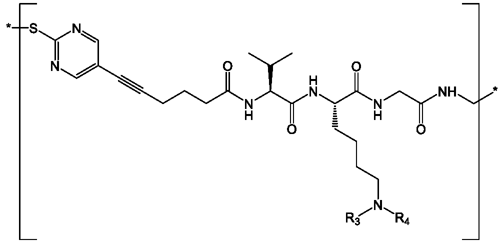 wherein R₃ and R₄ are each independently selected from methyl, ethyl, and propyl.

12. The antibody-drug conjugate or the pharmaceutical acceptable salt or solvate thereof according to claim 11, wherein R₃ and R₄ are both methyl; or R₃ and R₄ are both ethyl; or R₃ and R₄ are both propyl.

13. The antibody-drug conjugate or the pharmaceutical acceptable salt or solvate thereof according to claim 1, wherein the L-D unit of formula I is linked to the antibody by forming a thioether bond with a sulfhydryl group of a cysteine of the light and/or heavy chain of Ab.

14. The antibody-drug conjugate or the pharmaceutical acceptable salt or solvate thereof according to claim 1, wherein the ADC is selected from: 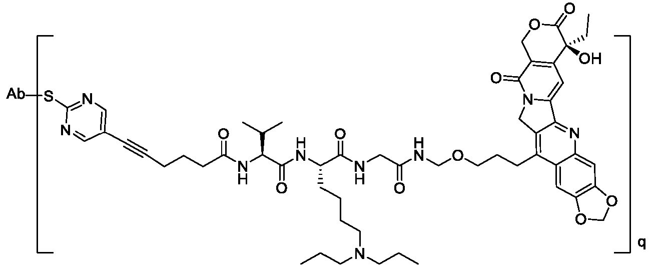 and 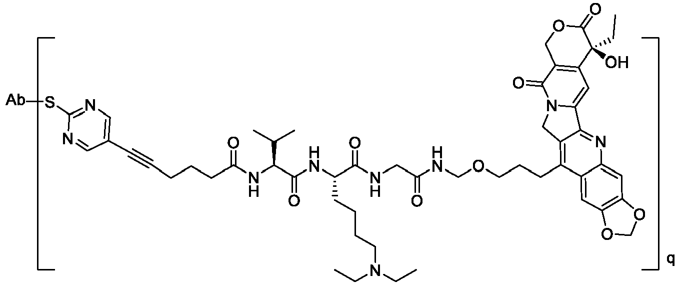 or 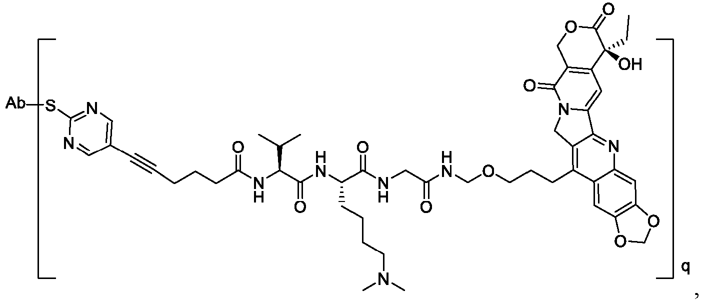
wherein q is an average DAR value of 1-8, preferably, q is about 2, about 3, about 4, about 5, about 6, about 7 or about 8,
preferably, the Ab is a full-length IgG1 antibody and comprises three CDRs of a heavy chain variable region (VH) sequence set forth in SEQ ID NO: 7 and three CDRs of a light chain variable region (VL) sequence set forth in SEQ ID NO: 8; or more preferably, comprises a heavy chain variable region set forth in SEQ ID NO: 7 and a light chain variable region set forth in SEQ ID NO: 8.

15. A pharmaceutical composition, comprising the antibody-drug conjugate or the pharmaceutical acceptable salt or solvate thereof according to any one of the preceding claims 1-14, and optionally a pharmaceutical auxiliary material.

16. A tissue factor (TF)-binding antibody or an antigen-binding fragment thereof, comprising:
(i) HCDR1, 2 and 3 sequences of a heavy chain variable region set forth in SEQ ID NO: 1, and LCDR1, 2 and 3 sequences of a light chain variable region set forth in SEQ ID NO: 2; or
(ii) HCDR1, 2 and 3 sequences of a heavy chain variable region set forth in SEQ ID NO: 3, and LCDR1, 2 and 3 sequences of a light chain variable region set forth in SEQ ID NO: 4; or
(iii) HCDR1, 2 and 3 sequences of a heavy chain variable region set forth in SEQ ID NO: 5, and LCDR1, 2 and 3 sequences of a light chain variable region set forth in SEQ ID NO: 6; or
(iv) HCDR1, 2 and 3 sequences of a heavy chain variable region set forth in SEQ ID NO: 7, and LCDR1, 2 and 3 sequences of a light chain variable region set forth in SEQ ID NO: 8,
wherein preferably, the CDRs are defined according to Chothia, AbM, Kabat or IMGT, or a combination thereof.

17. The antibody or the antigen-binding fragment thereof according to claim 16, wherein the antibody or the antigen-binding fragment thereof comprises a heavy chain variable region and a light chain variable region, wherein
(i) the heavy chain variable region comprises an amino acid sequence set forth in SEQ ID NO: 1 or an amino acid sequence having at least 80%, 85%, 90%, 95%, 96%, 97%, 98% or 99% identity thereto, and the light chain variable region comprises an amino acid sequence set forth in SEQ ID NO: 2 or an amino acid sequence having at least 80%, 85%, 90%, 95%, 96%, 97%, 98% or 99% identity thereto, or
(ii) the heavy chain variable region comprises an amino acid sequence set forth in SEQ ID NO: 3 or an amino acid sequence having at least 80%, 85%, 90%, 95%, 96%, 97%, 98% or 99% identity thereto, and the light chain variable region comprises an amino acid sequence set forth in SEQ ID NO: 4 or an amino acid sequence having at least 80%, 85%, 90%, 95%, 96%, 97%, 98% or 99% identity thereto, or
(iii) the heavy chain variable region comprises an amino acid sequence set forth in SEQ ID NO: 5 or an amino acid sequence having at least 80%, 85%, 90%, 95%, 96%, 97%, 98% or 99% identity thereto, and the light chain variable region comprises an amino acid sequence set forth in SEQ ID NO: 6 or an amino acid sequence having at least 80%, 85%, 90%, 95%, 96%, 97%, 98% or 99% identity thereto, or
(iv) the heavy chain variable region comprises an amino acid sequence set forth in SEQ ID NO: 7 or an amino acid sequence having at least 80%, 85%, 90%, 95%, 96%, 97%, 98% or 99% identity thereto, and the light chain variable region comprises an amino acid sequence set forth in SEQ ID NO: 8 or an amino acid sequence having at least 80%, 85%, 90%, 95%, 96%, 97%, 98% or 99% identity thereto;
wherein preferably,
the antibody or the antigen-binding fragment thereof comprises a heavy chain variable region and a light chain variable region selected from:
(i) a heavy chain variable region comprising an amino acid sequence set forth in SEQ ID NO: 1 and a light chain variable region comprising an amino acid sequence set forth in SEQ ID NO: 2, or
(ii) a heavy chain variable region comprising an amino acid sequence set forth in SEQ ID NO: 3 and a light chain variable region comprising an amino acid sequence set forth in SEQ ID NO: 4, or
(iii) a heavy chain variable region comprising an amino acid sequence set forth in SEQ ID NO: 5 and a light chain variable region comprising an amino acid sequence set forth in SEQ ID NO: 6, or
(iv) a heavy chain variable region comprising an amino acid sequence set forth in SEQ ID NO: 7 and a light chain variable region comprising an amino acid sequence set forth in SEQ ID NO: 8.

18. The antibody or the antigen-binding fragment thereof according to any one of claims 16-17, wherein
the antibody comprises an Fc region, preferably a human Fc region; or
the antibody is an IgG1, IgG2, IgG3 or IgG4 antibody, and preferably has a human IgG1 heavy chain constant region; or
the antigen-binding fragment is an antibody fragment selected from: Fab, Fab', Fab'-SH, Fv, single-chain antibody, scFv, scFab, disulfide-linked scFv, disulfide-linked scFab, (Fab')₂ fragment, or a linear antibody.

19. The antibody or the antigen-binding fragment thereof according to any one of claims 16-18, wherein the antibody is a murine antibody, a chimeric antibody or a humanized antibody, preferably a humanized antibody.

20. An isolated nucleic acid, encoding the anti-TF antibody or the antigen-binding fragment thereof according to any one of claims 16-19.

21. A vector, comprising the nucleic acid according to claim 20, wherein preferably, the vector is an expression vector.

22. A host cell, comprising the nucleic acid according to claim 20 or the vector according to claim 21, wherein preferably, the host cell is a mammalian cell.

23. An immunoconjugate or immunofusion or a multispecific antibody, comprising the antibody or the antigen-binding fragment thereof according to any one of claims 16-19.

24. A pharmaceutical composition, comprising the antibody or the antigen-binding fragment thereof according to any one of the preceding claims 16-19, or the immunoconjugate or immunofusion or the multispecific antibody according to claim 23, and optionally a pharmaceutical auxiliary material.

25. Use of the antibody or the antigen-binding fragment thereof according to any one of claims 16-19, or the immunoconjugate or immunofusion or the multispecific antibody according to claim 23 for: *in vivo* or *in vitro,*
(1) targeting and binding to TF-positive tumor cells;
(2) blocking a TF/VIIa-mediated downstream signaling pathway in the TF-positive tumor cells; or
(3) inhibiting and/or killing the TF-positive tumor cells,
or for preparing a medicament for use in any one of the preceding uses.

26. A method for preventing or treating a TF-positive tumor in a subject, comprising administering to the subject an effective amount of the antibody-drug conjugate or the pharmaceutical acceptable salt or solvate thereof according to any one of the preceding claims 1-14, or the anti-TF antibody or the antigen-binding fragment thereof according to any one of claims 16-19, or the immunoconjugate or immunofusion or the multispecific antibody according to claim 23, or the pharmaceutical composition according to claim 15 or 24, wherein preferably, the tumor is cervical cancer, pancreatic cancer, lung cancer, prostate cancer, bladder cancer, ovarian cancer, breast cancer, colorectal cancer, esophageal cancer, head and neck cancer, and gastric cancer, including primary or advanced or metastatic cancer.

27. A method for detecting TF in a sample, comprising:
(a) contacting a sample with the antibody or the antigen-binding fragment thereof according to any one of the preceding claims 16-19; and
(b) detecting the formation of a complex by the antibody or the antigen-binding fragment thereof and TF; optionally, wherein the antibody is detectably labeled.
